(19) Europäisches Patentamt
European Patent Office
Office européen des brevets

(11) EP 1 529 842 A2

(12) # EUROPEAN PATENT APPLICATION

(43) Date of publication:
**11.05.2005 Bulletin 2005/19**

(21) Application number: **04024742.1**

(22) Date of filing: **15.03.2000**

(51) Int Cl.[7]: **C12N 15/12**, C07K 14/47, A61K 38/17, G01N 33/53, C12Q 1/68, C12N 15/62, C07K 16/18

(84) Designated Contracting States:
**AT BE CH CY DE DK ES FI FR GB GR IE IT LI LU MC NL PT SE**

(30) Priority: **20.07.1999 US 144758 P**

(62) Document number(s) of the earlier application(s) in accordance with Art. 76 EPC:
**00919420.0 / 1 198 568**

(71) Applicant: **Genetech, Inc.**
**South San Francisco, CA 94080-4990 (US)**

(72) Inventors:
- **Ashkenazi, Avi J.**
  **San Mateo, CA 94420 (US)**
- **Baker, Kevin P.**
  **Darnestown, MD 20878 (US)**
- **Fong, Sherman**
  **Alameda, CA 94502 (US)**
- **Goddard, Audrey**
  **San Francisco, CA 94131 (US)**
- **Godowski, Paul J.**
  **Burlingame, CA 94010 (US)**
- **Gurney, Austin L.**
  **San Francisco, CA 94114-3627 (US)**
- **Hillan, Kenneth J.**
  **San Francisco, CA 94114 (US)**
- **Mark, Melanie R.**
  **Burlingame, CA 94010 (US)**
- **Marsters, Scot A.**
  **San Carlos, CA 94070 (US)**
- **Pitti, Robert M.**
  **El Cerrito, CA 94530 (US)**
- **Tumas, Daniel**
  **Orinda, CA 94563 (US)**
- **Watanabe, Colin K.**
  **Moraga, CA 94556 (US)**
- **Wood, William I.**
  **Cupertino,CA 95014 (US)**

(74) Representative:
**Denison, Christopher Marcus et al**
**Mewburn Ellis LLP**
**York House**
**23 Kingsway**
**London WC2B 6HP (GB)**

Remarks:
This application was filed on 18 - 10 - 2004 as a divisional application to the application mentioned under INID code 62.

(54) **Compositions and methods for the treatment of immune related diseases**

(57) The present invention relates to a composition containing novel proteins and methods for the diagnosis and treatment of immune related diseases.

EP 1 529 842 A2

## Description

### Field of the Invention

The present invention relates to compositions and methods for the diagnosis and treatment of immune related diseases.

### Background of the Invention

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

Many immune related diseases are known and have been extensively studied. Such diseases include immune-mediated inflammatory diseases, non-immune-mediated inflammatory diseases, infectious diseases, immunodeficiency diseases, neoplasia, etc.

T lymphocytes (T cells) are an important component of a mammalian immune response. T cells recognise antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen-MHC complex on an antigen presenting cell. Helper T cells also secrete a variety of cytokines, i.e. lymphokines, which play a central role in the activation of B cells, cytotoxic T cells and a variety of other cells which participate in the immune response.

A central event in both humoral and cell mediated immune responses is the activation and clonal expansion of helper T cells. Helper T cell activation is initiate by the interaction of the T cell receptor (TCR) - CD3 complex with an antigen-MHC on the surface of an antigen presenting cell. This interaction mediates a cascade of biochemical events that induce the resting helper T cell to enter a cell cycle (the Go to G1 transition) and results in the expression of a high affinity receptor for IL-2 and sometimes IL-4. The activated T cell progresses through the cycle proliferating and differentiating into memory cells or effector cells.

In addition to the signals mediated through the TCR, activation of T cells involves additional costimulation induced by cytokines released by the antigen presenting cell or through interactions with membrane bound molecules on the antigen presenting cell and the T cell. The cytokines IL-1 and IL-6 have been shown to provide a costimulatory signal. Also, the interaction between the B7 molecule expressed on the surface of an antigen presenting cell and CD28 and CTLA-4 molecules expressed on the T cell surface effect T cell activation. Activated T cells express an increased number of cellular adhesion molecules, such as ICAM-1, integrins, VLA-4, LFA-1, CD56, etc.

T-cell proliferation in a mixed lymphocyte culture or mixed lymphocyte reaction (MLR) is an established indication of the ability of a compound to stimulate the immune system. In many immune responses, inflammatory cells infiltrate the site of injury or infection. The migrating cells may be neutrophilic, eosinophilic, monocytic or lymphocytic as can be determined by histologic examination of the affected tissues. Current Protocols in Immunology, ed. John E. Coligan, 1994, John Wiley & Sons, Inc.

Immune related diseases can be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

### Summary of the Invention

#### A. Embodiments

The present invention concerns compositions and methods for the diagnosis and treatment of immune related disease in mammals, including humans. The present invention is based on the identification of proteins (including agonist and antagonist antibodies) which either stimulate or inhibit the immune response in mammals. Immune related diseases can be treated by suppressing or enhancing the immune response. Molecules that enhance the immune response stimulate or potentiate the immune response to an antigen. Molecules which stimulate the immune response can be used therapeutically where enhancement of the immune response would be beneficial. Such stimulatory molecules can also be inhibited where suppression of the immune response would be of value. Neutralizing antibodies are examples of molecules that inhibit molecules having immune stimulatory activity and which would be beneficial in the treatment of immune related and inflammatory diseases. Molecules which inhibit the immune response can also be utilized (proteins directly or via the use of antibody agonists) to inhibit the immune response and thus ameliorate immune related disease.

Accordingly, the proteins of the invention encoded by the genes of the invention are useful for the diagnosis and/or treatment (including prevention) of immune related diseases. Antibodies which bind to stimulatory proteins are useful to suppress the immune system and the immune response. Antibodies which bind to inhibitory proteins are useful to stimulate the immune system and the immune response. The proteins and antibodies of the invention are also useful to prepare medicines and medicaments for the treatment of immune related and inflammatory diseases.

In one embodiment, the present invention concerns an isolated antibody which binds a PRO179, PRO175, PRO182, PRO366, PRO240, PRO256, PRO306, PRO364, PRO356, PRO826, PRO1068, PRO1343 or PRO1375 polypeptide. In one aspect, the antibody mimics the activity of a PRO179, PRO175, PRO182, PRO366, PRO240, PRO256, PRO306, PRO364, PRO356, PRO826, PRO1068, PRO1343 or PRO1375 polypeptide (an agonist antibody) or conversely the antibody inhibits or neutralizes the activity of a PRO179, PRO175, PRO182, PRO366, PRO240, PRO256, PRO306, PRO364, PRO356, PRO826, PRO1068, PRO1343 or PRO1375 polypeptide (an antagonist antibody). In another aspect, the antibody is a monoclonal antibody, which preferably has nonhuman complementarity determining region (CDR) residues and human framework region (FR) residues. The antibody may be labeled and may be immobilized on a solid support. In a further aspect, the antibody is an antibody fragment, a single-chain antibody, or an anti-idiotypic antibody.

In another embodiment, the invention concerns a composition containing a PRO179, PRO175, PRO182, PRO366, PRO240, PRO256, PRO306, PRO364, PRO356, PRO826, PRO1068, PRO1343 or PRO1375 polypeptide or an agonist or antagonist antibody which binds the polypeptide in admixture with a carrier or excipient. In one aspect, the composition contains a therapeutically effective amount of the peptide or antibody. In another aspect, when the composition contains an immune stimulating molecule, the composition is useful for: (a) increasing infiltration of inflammatory cells into a tissue of a mammal in need thereof, (b) stimulating or enhancing an immune response in a mammal in need thereof, or (c) increasing the proliferation of T-lymphocytes in a mammal in need thereof in response to an antigen. In a further aspect, when the composition contains an immune inhibiting molecule, the composition is useful for: (a) decreasing infiltration of inflammatory cells into a tissue of a mammal in need thereof, (b) inhibiting or reducing an immune response in a mammal in need thereof, or (c) decreasing the proliferation of T-lymphocytes in a mammal in need thereof in response to an antigen. In another aspect, the composition contains a further active ingredient, which may, for example, be a further antibody or a cytotoxic or chemotherapeutic agent. Preferably, the composition is sterile.

In another embodiment, the invention concerns the use of the polypeptides and antibodies of the invention to prepare a composition or medicament which has the uses described above.

In a further embodiment, the invention concerns nucleic acid encoding an anti-PRO179, PRO175, PRO182, PRO366, PRO240, PRO256, PRO306, PRO364, PRO356, PRO826, PRO1068, PRO1343 or PRO1375 antibody, and vectors and recombinant host cells comprising such nucleic acid. In a still further embodiment, the invention concerns a method for producing such an antibody by culturing a host cell transformed with nucleic acid encoding the antibody under conditions such that the antibody is expressed, and recovering the antibody from the cell culture.

The invention further concerns antagonists and agonists of a PRO179, PRO175, PRO182, PRO366, PRO240, PRO256, PRO306, PRO364, PRO356, PRO826, PRO1068, PRO1343 or PRO1375 polypeptide that inhibit one or more of the functions or activities of the PRO179, PRO175, PRO182, PRO366, PRO240, PRO256, PRO306, PRO364, PRO356, PRO826, PRO1068, PRO1343 or PRO1375 polypeptide.

In a further embodiment, the invention concerns isolated nucleic acid molecules that hybridize to the complement of the nucleic acid molecules encoding the PRO179, PRO175, PRO182, PRO366, PRO240, PRO256, PRO306, PRO364, PRO356, PRO826, PRO1068, PRO1343 or PRO1375 polypeptides. The nucleic acid preferably is DNA, and hybridization preferably occurs under stringent conditions. Such nucleic acid molecules can act as antisense molecules of the amplified genes identified herein, which, in turn, can find use in the modulation of the respective amplified genes, or as antisense primers in amplification reactions. Furthermore, such sequences can be used as part of ribozyme and/or triple helix sequence which, in turn, may be used in regulation of the amplified genes.

In another embodiment, the invention concerns a method for determining the presence of a PRO 179, PRO175, PRO182, PRO366, PRO240, PRO256, PRO306, PRO364, PRO356, PRO826, PRO1068, PRO1343 or PRO1375 polypeptide comprising exposing a cell suspected of containing the polypeptide to an anti-PRO179, PRO175, PRO182, PRO366, PRO240, PRO256, PRO306, PRO364, PRO356, PRO826, PRO1068, PRO1343 or PRO1375 antibody and determining binding of the antibody to the cell.

In yet another embodiment, the present invention concerns a method of diagnosing an immune related disease in a mammal, comprising detecting the level of expression of a gene encoding a PRO179, PRO175, PRO182, PRO366, PRO240, PRO256, PRO306, PRO364, PRO356, PRO826, PRO1068, PRO1343 or PRO1375 polypeptide (a) in a test sample of tissue cells obtained from the mammal, and (b) in a control sample of known normal tissue cells of the same cell type, wherein a higher expression level in the test sample indicates the presence of immune related disease in the mammal from which the test tissue cells were obtained.

In another embodiment, the present invention concerns a method of diagnosing an immune disease in a mammal, comprising (a) contacting an anti-PRO179, PRO175, PRO182, PRO366, PRO240, PRO256, PRO306, PRO364, PRO356, PRO826, PRO1068, PRO1343 or PRO1375 antibody with a test sample of tissue cells obtained from the mammal , and (b) detecting the formation of a complex between the antibody and the PRO179, PRO175, PRO182, PRO366, PRO240, PRO256, PRO306, PRO364, PRO356, PRO826, PRO1068, PRO1343 or PRO1375 polypeptide in the test sample. The detection may be qualitative or quantitative, and may be performed in comparison with monitoring the complex formation in a control sample of known normal tissue cells of the same cell type. A larger quantity of complexes formed in the test sample indicates the presence of tumor in the mammal from which the test tissue cells were obtained. The antibody preferably carries a detectable label. Complex formation can be monitored, for example, by light microscopy, flow cytometry, fluorimetry, or other techniques known in the art. The test sample is usually obtained from an individual suspected of having a deficiency or abnormality of the immune system.

In another embodiment, the present invention concerns a diagnostic kit, containing an anti- PRO179, PRO175, PRO182, PRO366, PRO240, PRO256, PRO306, PRO364, PRO356, PRO826, PRO1068, PRO1343 or PRO1375 antibody and a carrier (e.g. a buffer) in suitable packaging. The kit preferably contains instructions for using the antibody to detect the PRO179, PRO175, PRO182, PRO366, PRO240, PRO256, PRO306, PRO364, PRO356, PRO826, PRO1068, PRO1343 or PRO1375 polypeptide.

In a further embodiment, the invention concerns an article of manufacture, comprising:

a container;
a label on the container; and
a composition comprising an active agent contained within the container; wherein the composition is effective for stimulating or inhibiting an immune response in a mammal, the label on the container indicates that the composition can be used to treat an immune related disease, and the active agent in the composition is an agent stimulating or inhibiting the expression and/or activity of the PRO179, PRO175, PRO182, PRO366, PRO240, PRO256, PRO306, PRO364, PRO356, PRO826, PRO1068, PRO1343 or PRO1375 polypeptide. In a preferred aspect, the active agent is a PRO179, PRO175, PRO182, PRO366, PRO240, PRO256, PRO306, PRO364, PRO356, PRO826, PRO1068, PRO1343 or PRO1375 polypeptide or an anti-PRO179, PRO175, PRO182, PRO366, PRO240, PRO256, PRO306, PRO364, PRO356, PRO826, PRO1068, PRO1343 or PRO1375 antibody.

A further embodiment is a method for identifying a compound capable of inhibiting the expression and/or activity of a PRO179, PRO175, PRO182, PRO366, PRO240, PRO256, PRO306, PRO364, PRO356, PRO826, PRO1068, PRO1343 or PRO1375 polypeptide by contacting a candidate compound with a PRO179, PRO175, PRO182, PRO366, PRO240, PRO256, PRO306, PRO364, PRO356, PRO826, PRO1068, PRO1343 or PRO1375 polypeptide under conditions and for a time sufficient to allow these two components to interact. In a specific aspect, either the candidate compound or the PRO179, PRO175, PRO182, PRO366, PRO240, PRO256, PRO306, PRO364, PRO356, PRO826, PRO1068, PRO1343 or PRO 1375 polypeptide is immobilized on a solid support. In another aspect, the non-immobilized component carries a detectable label.

In another embodiment, the invention is directed to a method of stimulating the proliferation of T lymphocytes, wherein the method comprises contacting T lymphocytes with a PRO256, PRO306, PRO364, PRO826, PRO1068, PRO1343 or PRO1375 polypeptide in an amount sufficient to stimulate the proliferation of the T lymphocytes.

Yet another embodiment is directed to a method of stimulating an immune response in a mammal, wherein the method comprises administering to the mammal a PRO175, PRO179, PRO240, PRO366 or PRO1375 polypeptide in an amount sufficient to stimulate an immune response.

B. Additional Embodiments

In other embodiments of the present invention, the invention provides vectors comprising DNA encoding any of the herein described polypeptides. Host cell comprising any such vector are also provided. By way of example, the host cells may be CHO cells, *E. coli*, or yeast. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

In other embodiments, the invention provides an isolated nucleic acid molecule comprising a nucleotide sequence that encodes a PRO polypeptide.

In one aspect, the isolated nucleic acid molecule comprises a nucleotide sequence having at least about 80% nucleic acid sequence identity, alternatively at least about 81 % nucleic acid sequence identity, alternatively at least about 82 % nucleic acid sequence identity, alternatively at least about 83% nucleic acid sequence identity, alternatively at least about 84 % nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92 % nucleic acid sequence identity, alternatively at least about 93 % nucleic acid sequence identity, alternatively at least about 94 % nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81 % nucleic acid sequence identity, alternatively at least about 82 % nucleic acid sequence identity, alternatively at least about 83 % nucleic acid sequence identity, alternatively at least about 84 % nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94 % nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95 % nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs deposited with the ATCC as disclosed herein, or (b) the complement of the DNA molecule of (a).

Another aspect the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides isolated PRO polypeptide encoded by any of the isolated nucleic acid sequences hereinabove identified.

In a certain aspect, the invention concerns an isolated PRO polypeptide, comprising an amino acid sequence having at least about 80 % amino acid sequence identity, alternatively at least about 81 % amino acid sequence identity, alternatively at least about 82 % amino acid sequence identity, alternatively at least about 83% amino acid sequence identity, alternatively at least about 84% amino acid sequence identity, alternatively at least about 85% amino acid sequence identity, alternatively at least about 86 % amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92 % amino acid sequence identity, alternatively at least about 93% amino acid sequence identity, alternatively at least about 94% amino acid sequence identity, alternatively at least about 95 % amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs deposited with the ATCC as disclosed herein.

In a further aspect, the invention concerns an isolated PRO polypeptide comprising an amino acid sequence scoring at least about 80% positives, alternatively at least about 81 % positives, alternatively at least about 82% positives, alternatively at least about 83% positives, alternatively at least about 84% positives, alternatively at least about 85% positives, alternatively at least about 86 % positives, alternatively at least about 87% positives, alternatively at least about 88% positives, alternatively at least about 89% positives, alternatively at least about 90% positives, alternatively at least about 91 % positives, alternatively at least about 92 % positives, alternatively at least about 93% positives, alternatively at least about 94% positives, alternatively at least about 95% positives, alternatively at least about 96% positives, alternatively at least about 97% positives, alternatively at least about 98% positives and alternatively at least about 99% positives when compared with the amino acid sequence of a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a specific aspect, the invention provides an isolated PRO polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists and antagonists of a native PRO polypeptide as defined herein. In a particular embodiment, the agonist or antagonist is an anti-PRO antibody or a small molecule.

In a further embodiment, the invention concerns a method of identifying agonists or antagonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist or antagonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist or antagonist thereof as hereinbefore described, or an anti-PRO antibody, for the preparation of a medicament useful in the treatment of a condition which is responsive to the PRO polypeptide, an agonist or antagonist thereof or an anti-PRO antibody.

Brief Description of the Drawings

Figure 1 shows the nucleotide sequence of a cDNA containing a nucleotide sequence encoding native sequence PRO179 (UNQ153), wherein the nucleotide sequence (SEQ ID NO:1) is a clone designated herein as "DNA16451-1388". Also presented in bold font and underlined are the positions of the respective start and stop codons.

Figure 2 shows the amino acid sequence (SEQ ID NO:2) of a native sequence PRO179 polypeptide as derived from the coding sequence of Figure 1. Also shown are the approximate locations of various other important polypeptide domains if known.

Figure 3 shows the nucleotide sequence of a cDNA containing a nucleotide sequence encoding native sequence PRO175 (UNQ149), wherein the nucleotide sequence (SEQ ID NO:3) is a clone designated herein as "DNA19355-1150". Also presented in bold font and underlined are the positions of the respective start and stop codons.

Figure 4 shows the amino acid sequence (SEQ ID NO:4) of a native sequence PRO175 polypeptide as derived from the coding sequence of Figure 3. Also shown are the approximate locations of various other important polypeptide domains if known.

Figure 5 shows the nucleotide sequence of a cDNA containing a nucleotide sequence encoding native sequence PRO182 (UNQ156), wherein the nucleotide sequence (SEQ ID NO:5) is a clone designated herein as "DNA27865-1091". Also presented in bold font and underlined are the positions of the respective start and stop codons.

Figure 6 shows the amino acid sequence (SEQ ID NO:6) of a native sequence PRO182 polypeptide as derived from the coding sequence of Figure 5. Also shown are the approximate locations of various other important polypeptide domains if known.

Figure 7 shows the nucleotide sequence of a cDNA containing a nucleotide sequence encoding native sequence PRO366 (UNQ321), wherein the nucleotide sequence (SEQ ID NO:7) is a clone designated herein as "DNA33085-1110". Also presented in bold font and underlined are the positions of the respective start and stop codons.

Figure 8 shows the amino acid sequence (SEQ ID NO:8) of a native sequence PRO366 polypeptide as derived from the coding sequence of Figure 7. Also shown are the approximate locations of various other important polypeptide domains if known.

Figure 9 shows the nucleotide sequence of a cDNA containing a nucleotide sequence encoding native sequence PRO240 (UNQ214), wherein the nucleotide sequence (SEQ ID NO:9) is a clone designated herein as "DNA34387-1138". Also presented in bold font and underlined are the positions of the respective start and stop codons.

Figure 10 shows the amino acid sequence (SEQ ID NO:10) of a native sequence PRO240 polypeptide as derived from the coding sequence of Figure 9. Also shown are the approximate locations of various other important polypeptide domains if known.

Figure 11 shows the nucleotide sequence of a cDNA containing a nucleotide sequence encoding native sequence PRO256 (UNQ223), wherein the nucleotide sequence (SEQ ID NO:11) is a clone designated herein as "DNA35880-1160". Also presented in bold font and underlined are the positions of the respective start and stop codons.

Figure 12 shows the amino acid sequence (SEQ ID NO:12) of a native sequence PRO256 polypeptide as derived from the coding sequence of Figure 11. Also shown are the approximate locations of various other important polypeptide domains if known.

Figure 13 shows the nucleotide sequence of a cDNA containing a nucleotide sequence encoding native sequence PRO306 (UNQ269), wherein the nucleotide sequence (SEQ ID NO:13) is a clone designated herein as "DNA39984-1221". Also presented in bold font and underlined are the positions of the respective start and stop codons.

Figure 14 shows the amino acid sequence (SEQ ID NO:14) of a native sequence PRO306 polypeptide as derived from the coding sequence of Figure 13. Also shown are the approximate locations of various other important polypeptide domains if known.

Figure 15 shows the nucleotide sequence of a cDNA containing a nucleotide sequence encoding native sequence PRO364 (UNQ319), wherein the nucleotide sequence (SEQ ID NO:15) is a clone designated herein as "DNA47365-1206". Also presented in bold font and underlined are the positions of the respective start and stop codons.

Figure 16 shows the amino acid sequence (SEQ ID NO:16) of a native sequence PRO364 polypeptide as derived from the coding sequence of Figure 15. Also shown are the approximate locations of various other important polypeptide domains if known.

Figure 17 shows the nucleotide sequence of a cDNA containing a nucleotide sequence encoding native sequence PRO356 (UNQ313), wherein the nucleotide sequence (SEQ ID NO:17) is a clone designated herein as "DNA47470-1130". Also presented in bold font and underlined are the positions of the respective start and stop codons.

Figure 18 shows the amino acid sequence (SEQ ID NO:18) of a native sequence PRO356 polypeptide as derived from the coding sequence of Figure 17. Also shown are the approximate locations of various other important polypeptide domains if known.

Figure 19 shows the nucleotide sequence of a cDNA containing a nucleotide sequence encoding native sequence PRO826 (UNQ467), wherein the nucleotide sequence (SEQ ID NO:19) is a clone designated herein as "DNA57694-1341". Also presented in bold font and underlined are the positions of the respective start and stop codons.

Figure 20 shows the amino acid sequence (SEQ ID NO:20) of a native sequence PRO826 polypeptide as derived from the coding sequence of Figure 19. Also shown are the approximate locations of various other important polypeptide domains if known.

Figure 21 shows the nucleotide sequence of a cDNA containing a nucleotide sequence encoding native sequence PRO1068 (UNQ525), wherein the nucleotide sequence (SEQ ID NO:21) is a clone designated herein as "DNA59214-1449". Also presented in bold font and underlined are the positions of the respective start and stop codons.

Figure 22 shows the amino acid sequence (SEQ ID NO:22) of a native sequence PRO1068 polypeptide as derived from the coding sequence of Figure 21. Also shown are the approximate locations of various other important polypeptide domains if known.

Figure 23 shows the nucleotide sequence of a cDNA containing a nucleotide sequence encoding native sequence PRO1343 (UNQ698), wherein the nucleotide sequence (SEQ ID NO:23) is a clone designated herein as "DNA66675-1587". Also presented in bold font and underlined are the positions of the respective start and stop codons.

Figure 24 shows the amino acid sequence (SEQ ID NO:24) of a native sequence PRO1343 polypeptide as derived from the coding sequence of Figure 23. Also shown are the approximate locations of various other important polypeptide domains if known.

Figure 25 shows the nucleotide sequence of a cDNA containing a nucleotide sequence encoding native sequence PRO1375 (UNQ712), wherein the nucleotide sequence (SEQ ID NO:25) is a clone designated herein as "DNA67004-1614". Also presented in bold font and underlined are the positions of the respective start and stop codons.

Figure 26 shows the amino acid sequence (SEQ ID NO:26) of a native sequence PRO1375 polypeptide as derived from the coding sequence of Figure 25. Also shown are the approximate locations of various other important polypeptide domains if known.

## Detailed Description of the Preferred Embodiments

### I. Definitions

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are immune-mediated inflammatory diseases, non-immune-mediated inflammatory diseases, infectious diseases, immunodeficiency diseases, neoplasia, etc.

The term "T cell mediated" disease means a disease in which T cells directly or indirectly mediate or otherwise contribute to a morbidity in a mammal. The T cell mediated disease may be associated with cell mediated effects, lymphokine mediated effects, etc., and even effects associated with B cells if the B cells are stimulated, for example, by the lymphokines secreted by T cells.

Examples of immune-related and inflammatory diseases, some of which are imune or T cell mediated, which can be treated according to the invention include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjogren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft-versus-host-disease. Infectious diseases including viral diseases such as AIDS (HIV infection), hepatitis A, B, C, D, and E, herpes, etc., bacterial infections, fungal infections, protozoal infections and parasitic infections.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. In treatment of an immune related disease, a therapeutic agent may directly decrease or increase the magnitude of response of a component of the immune response, or render the disease more susceptible to treatment by other therapeutic agents, e.g. antibiotics, antifungals, anti-inflammatory agents, chemotherapeutics, etc.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

The "pathology" of an immune related disease includes all phenomena that compromise the well-being of the patient. This includes, without limitation, abnormal or uncontrollable cell growth, antibody production, auto-antibody production, complement production and activation, interference with the normal functioning of neighboring cells, release of cytokines or other secretory products at abnormal levels, suppression or aggravation of any inflammatory or immunological response, infiltration of inflammatory cells (neutrophilic, eosinophilic, monocytic, lymphocytic) into tissue spaces, etc.

"Mammal" for purposes of treatment refers to any animal classified as a mammal, including humans, domestic and farm animals, and zoo, sports, or pet animals, such as dogs, horses, cats, cows, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

The term "cytotoxic agent" as used herein refers to a substance that inhibits or prevents the function of cells and/or causes destruction of cells. The term is intended to include radioactive isotopes (e.g. 1131, I125, Y90 and Re186), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, e.g. paclitaxel (Taxol, Bristol-Myers Squibb Oncology, Princeton, NJ), and doxetaxel (Taxotere, Rhone-Poulenc Rorer, Antony, Rnace), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or *in vivo.* Thus, the growth inhibitory agent is one which significantly reduces the percentage of cells overexpressing such genes in S phase. Examples of growth inhibitory agents include agents that block cell cycle progression (at a place other than S phase), such as agents that induce G 1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1a, IL-2, IL-3, IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each PRO polypeptide of the invention individually. The term "PRO polypeptide" also includes variants of the PRO/number polypeptides disclosed herein.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide *(e.g.,* an extracellular domain sequence), naturally-occurring variant forms (*e.g.*, alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are comtemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide. as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81% amino acid sequence identity, alternatively at least about 82% amino acid sequence identity, alternatively at least about 83 % amino acid sequence identity, alternatively at least about 84% amino acid sequence identity, alternatively at least about 85 % amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95 % amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in the specific PRO polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues.

Unless specifically stated otherwise, all % amino acid sequence identity values used herein are obtained as described in the immediately preceding paragraph using the ALIGN-2 computer program. However, % amino acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % amino acid sequence identity value is determined by dividing (a) the number of matching identical amino acid residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

Percent amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

"PRO variant polynucleotide" or "PRO variant nucleic acid sequence" means a nucleic acid molecule which encodes an active PRO polypeptide as defined below and which has at least about 80% nucleic acid sequence identity with a nucleotide acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94 % nucleic acid sequence identity, alternatively at least about 95 % nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to PRO-encoding nucleic acid sequences identified herein is defined as the percentage of nucleotides in a candidate sequence that are identical with the nucleotides in the PRO nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

100 times the fraction W/Z

where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides.

Unless specifically stated otherwise, all % nucleic acid sequence identity values used herein are obtained as described in the immediately preceding paragraph using the ALIGN-2 computer program. However, % nucleic acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (i.e., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

Percent nucleic acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

100 times the fraction W/Z

where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In other embodiments, PRO variant polynucleotides are nucleic acid molecules that encode an active PRO polypeptide and which are capable of hybridizing, preferably under stringent hybridization and wash conditions, to nucleotide sequences encoding a full-length PRO polypeptide as disclosed herein. PRO variant polypeptides may be those that are encoded by a PRO variant polynucleotide.

The term "positives", in the context of sequence comparison performed as described above, includes residues in the sequences compared that are not identical but have similar properties (e.g. as a result of conservative substitutions, see Table 6 below). For purposes herein, the % value of positives is determined by dividing (a) the number of amino acid residues scoring a positive value between the PRO polypeptide amino acid sequence of interest having a sequence derived from the native PRO polypeptide sequence and the comparison amino acid sequence of interest (i.e., the amino acid sequence against which the PRO polypeptide sequence is being compared) as determined in the BLOSUM62 matrix of WU-BLAST-2 by (b) the total number of amino acid residues of the PRO polypeptide of interest.

Unless specifically stated otherwise, the % value of positives is calculated as described in the immediately preceding paragraph. However, in the context of the amino acid sequence identity comparisons performed as described for ALIGN-2 and NCBI-BLAST-2 above, includes amino acid residues in the sequences compared that are not only identical, but also those that have similar properties. Amino acid residues that score a positive value to an amino acid residue of interest are those that are either identical to the amino acid residue of interest or are a preferred substitution (as defined in Table 6 below) of the amino acid residue of interest.

For amino acid sequence comparisons using ALIGN-2 or NCBI-BLAST2, the % value of positives of a given amino acid sequence A to, with, or against a given amino acid sequence B (which can alternatively be phrased as a given amino acid sequence A that has or comprises a certain % positives to, with, or against a given amino acid sequence B) is calculated as follows:

100 times the fraction X/Y

where X is the number of amino acid residues scoring a positive value as defined above by the sequence alignment program ALIGN-2 or NCBI-BLAST2 in that program's alignment of A and B, and where Y is the total number of amino acid residues in B. It will be appreciated that where the length of amino acid sequence A is not equal to the length of amino acid sequence B, the % positives of A to B will not equal the % positives of B to A.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO polypeptide natural environment will not be present. Ordinarily, however, isolated polypeptide will be prepared by at least one purification step.

An "isolated" PRO polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist, antagonist, and neutralizing antibodies), anti-PRO antibody compositions with polyepitopic specificity, single chain anti-PRO antibodies, and fragments of anti-PRO antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1 % bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1 % sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of a PRO polypeptide which retain a biological activity of native or naturally-occurring PRO, wherein "biological" activity refers to a biological function (either inhibitory or stimulatory) caused by a native or naturally-occurring PRO.

The term "antagonist" is used in the broadest sense, and includes any molecule that partially or fully blocks, inhibits, or neutralizes a biological activity of a native PRO polypeptide disclosed herein. In a similar manner, the term "agonist" is used in the broadest sense and includes any molecule that mimics a biological activity of a native PRO polypeptide disclosed herein. Suitable agonist or antagonist molecules specifically include agonist or antagonist antibodies or antibody fragments, fragments or amino acid sequence variants of native PRO polypeptides, peptides, antisense oligonucleotides, small organic molecules, etc. Methods for identifying agonists or antagonists of a PRO polypeptide may comprise contacting a PRO polypeptide with a candidate agonist or antagonist molecule and measuring a detectable change in one or more biological activities normally associated with the PRO polypeptide.

"Antibody fragments" comprise a portion of an intact antibody, preferably the antigen binding or variable region of the intact antibody. Examples of antibody fragments include Fab, Fab', $F(ab')_2$, and Fv fragments; diabodies; linear antibodies (Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an $F(ab')_2$ fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the $V_H$-$V_L$ dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgGI, IgG2, IgG3, IgG4, IgA, and IgA2.

Antibodies and fragments thereof in this invention also include "affinity matured" antibodies in which an antibody is altered to change the amino acid sequence of one or more of the CDR regions and/or the framework regions to alter the affinity of the antibody or fragment thereof for the antigen to which it binds. Affinity maturation may result in an increase or in a decrease in the affinity of the matured antibody for the antigen relative to the starting antibody. Typically, the starting antibody will be a humanized, human, chimeric or murine antibody and the affinity matured antibody will have a higher affinity than the starting antibody. During the maturation process, one or more of the amino acid residues in the CDRs or in the framework regions are changed to a different residue using any standard method. Suitable methods include point mutations using well known cassette mutagenesis methods (Wells et al, 1985, Gene, 34:315) or oligonucleotide mediated mutagenesis methods (Zoller et al., 1987, Nucleic Acids Res., 10:6487-6504). Affinity maturation may also be performed using known selection methods in which many mutations are produced and mutants having the desired affinity are selected from a pool or library of mutants based on improved affinity for the antigen or ligand. Known phage display techniques can be conveniently used in this approach. See, for example, U. S. 5,750,373; U.S. 5,223,409, etc.

Human antibodies are also with in the scope of the antibodies of the invention. Human antibodies can be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985); Boerner et al., J. Immunol., 147(1):86-95 (1991); U. S. 5,750, 373]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5.545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

"Single-chain Fv" or "sFv" antibody fragments comprise the $V_H$ and $V_L$ domains of antibody, wherein these domains are present in a single polypeptide chain. Preferably, the Fv polypeptide further comprises a polypeptide linker between the $V_H$ and $V_L$ domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain ($V_H$) connected to a light-chain variable domain ($V_L$) in the same polypeptide chain ($V_H$-$V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad.

Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

## Table 1

```
/*
 *
 * C-C increased from 12 to 15
 * Z is average of EQ
 * B is average of ND
 * match with stop is _M; stop-stop = 0; J (joker) match = 0
 */
#define  _M       -8        /* value of a match with a stop */

int        _day[26][26] = {
/*     A  B  C  D  E  F  G  H  I  J  K  L  M  N  O  P  Q  R  S  T  U  V  W  X  Y  Z */
/* A */    { 2, 0,-2, 0, 0,-4, 1,-1,-1, 0,-1,-2,-1, 0,_M, 1, 0,-2, 1, 1, 0, 0,-6, 0,-3, 0},
/* B */    { 0, 3,-4, 3, 2,-5, 0, 1,-2, 0, 0,-3,-2, 2,_M,-1, 1, 0, 0, 0, 0,-2,-5, 0,-3, 1},
/* C */    {-2,-4,15,-5,-5,-4,-3,-3,-2, 0,-5,-6,-5,-4,_M,-3,-5,-4, 0,-2, 0,-2,-8, 0, 0,-5},
/* D */    { 0, 3,-5, 4, 3,-6, 1, 1,-2, 0, 0,-4,-3, 2,_M,-1, 2,-1, 0, 0, 0,-2,-7, 0,-4, 2},
/* E */    { 0, 2,-5, 3, 4,-5, 0, 1,-2, 0, 0,-3,-2, 1,_M,-1, 2,-1, 0, 0, 0,-2,-7, 0,-4, 3},
/* F */    {-4,-5,-4,-6,-5, 9,-5,-2, 1, 0,-5, 2, 0,-4,_M,-5,-5,-4,-3,-3, 0,-1, 0, 0, 7,-5},
/* G */    { 1, 0,-3, 1, 0,-5, 5,-2,-3, 0,-2,-4,-3, 0,_M,-1,-1,-3, 1, 0, 0,-1,-7, 0,-5, 0},
/* H */    {-1, 1,-3, 1, 1,-2,-2, 6,-2, 0, 0,-2,-2, 2,_M, 0, 3, 2,-1,-1, 0,-2,-3, 0, 0, 2},
/* I */    {-1,-2,-2,-2,-2, 1,-3,-2, 5, 0,-2, 2, 2,-2,_M,-2,-2,-2,-1, 0, 0, 4,-5, 0,-1,-2},
/* J */    { 0, 0, 0, 0, 0, 0, 0, 0, 0, 0, 0, 0, 0, 0,_M, 0, 0, 0, 0, 0, 0, 0, 0, 0, 0, 0},
/* K */    {-1, 0,-5, 0, 0,-5,-2, 0,-2, 0, 5,-3, 0, 1,_M,-1, 1, 3, 0, 0, 0,-2,-3, 0,-4, 0},
/* L */    {-2,-3,-6,-4,-3, 2,-4,-2, 2, 0,-3, 6, 4,-3,_M,-3,-2,-3,-3,-1, 0, 2,-2, 0,-1,-2},
/* M */    {-1,-2,-5,-3,-2, 0,-3,-2, 2, 0, 0, 4, 6,-2,_M,-2,-1, 0,-2,-1, 0, 2,-4, 0,-2,-1},
/* N */    { 0, 2,-4, 2, 1,-4, 0, 2,-2, 0, 1,-3,-2, 2,_M,-1, 1, 0, 1, 0, 0,-2,-4, 0,-2, 1},
/* O */          { _M , _M , _M , _M , _M , _M , _M , _M , _M , _M , _M , _M , _M , _M ,
0,_M,_M,_M,_M,_M,_M,_M,_M,_M,_M,_M},
/* P */    { 1,-1,-3,-1,-1,-5,-1, 0,-2, 0,-1,-3,-2,-1,_M, 6, 0, 0, 1, 0, 0,-1,-6, 0,-5, 0},
/* Q */    { 0, 1,-5, 2, 2,-5,-1, 3,-2, 0, 1,-2,-1, 1,_M, 0, 4, 1,-1,-1, 0,-2,-5, 0,-4, 3},
/* R */    {-2, 0,-4,-1,-1,-4,-3, 2,-2, 0, 3,-3, 0, 0,_M, 0, 1, 6, 0,-1, 0,-2, 2, 0,-4, 0},
/* S */    { 1, 0, 0, 0, 0,-3, 1,-1,-1, 0, 0,-3,-2, 1,_M, 1,-1, 0, 2, 1, 0,-1,-2, 0,-3, 0},
/* T */    { 1, 0,-2, 0, 0,-3, 0,-1, 0, 0, 0,-1,-1, 0,_M, 0,-1,-1, 1, 3, 0, 0,-5, 0,-3, 0},
/* U */    { 0, 0, 0, 0, 0, 0, 0, 0, 0, 0, 0, 0, 0, 0,_M, 0, 0, 0, 0, 0, 0, 0, 0, 0, 0, 0},
/* V */    { 0,-2,-2,-2,-2,-1,-1,-2, 4, 0,-2, 2, 2,-2,_M,-1,-2,-2,-1, 0, 0, 4,-6, 0,-2,-2},
/* W */    {-6,-5,-8,-7,-7, 0,-7,-3,-5, 0,-3,-2,-4,-4,_M,-6,-5, 2,-2,-5, 0,-6,17, 0, 0,-6},
/* X */    { 0, 0, 0, 0, 0, 0, 0, 0, 0, 0, 0, 0, 0, 0,_M, 0, 0, 0, 0, 0, 0, 0, 0, 0, 0, 0},
/* Y */    {-3,-3, 0,-4,-4, 7,-5, 0,-1, 0,-4,-1,-2,-2,_M,-5,-4,-4,-3,-3, 0,-2, 0, 0,10,-4},
/* Z */    { 0, 1,-5, 2, 3,-5, 0, 2,-2, 0, 0,-2,-1, 1,_M, 0, 3, 0, 0, 0, 0,-2,-6, 0,-4, 4}
};
```

## Table 1 (cont')

```
/*
 */
#include <stdio.h>
#include <ctype.h>

#define MAXJMP      16      /* max jumps in a diag */
#define MAXGAP      24      /* don't continue to penalize gaps larger than this */
#define JMPS        1024    /* max jmps in an path */
#define MX          4       /* save if there's at least MX-1 bases since last jmp */

#define DMAT        3       /* value of matching bases */
#define DMIS        0       /* penalty for mismatched bases */
#define DINS0       8       /* penalty for a gap */
#define DINS1       1       /* penalty per base */
#define PINS0       8       /* penalty for a gap */
#define PINS1       4       /* penalty per residue */

struct jmp {
        short           n[MAXJMP];      /* size of jmp (neg for dely) */
        unsigned short  x[MAXJMP];      /* base no. of jmp in seq x */
};                                      /* limits seq to 2^16 -1 */

struct diag {
        int             score;          /* score at last jmp */
        long            offset;         /* offset of prev block */
        short           ijmp;           /* current jmp index */
        struct jmp      jp;             /* list of jmps */
};

struct path {
        int     spc;            /* number of leading spaces */
        short   n[JMPS];/* size of jmp (gap) */
        int     x[JMPS];/* loc of jmp (last elem before gap) */
};

char            *ofile;                 /* output file name */
char            *namex[2];              /* seq names: getseqs() */
char            *prog;                  /* prog name for err msgs */
char            *seqx[2];               /* seqs: getseqs() */
int             dmax;                   /* best diag: nw() */
int             dmax0;                  /* final diag */
int             dna;                    /* set if dna: main() */
int             endgaps;                /* set if penalizing end gaps */
int             gapx, gapy;             /* total gaps in seqs */
int             len0, len1;             /* seq lens */
int             ngapx, ngapy;           /* total size of gaps */
int             smax;                   /* max score: nw() */
int             *xbm;                   /* bitmap for matching */
long            offset;                 /* current offset in jmp file */
struct  diag    *dx;                    /* holds diagonals */
struct  path    pp[2];                  /* holds path for seqs */

char            *calloc(), *malloc(), *index(), *strcpy();
char            *getseq(), *g_calloc();
```

## Table 1 (cont')

```
/* Needleman-Wunsch alignment program
 *
 * usage: progs file1 file2
 *   where file1 and file2 are two dna or two protein sequences.
 *   The sequences can be in upper- or lower-case an may contain ambiguity
 *   Any lines beginning with ';', '>' or '<' are ignored
 *   Max file length is 65535 (limited by unsigned short x in the jmp struct)
 *   A sequence with 1/3 or more of its elements ACGTU is assumed to be DNA
 *   Output is in the file "align.out"
 *
 * The program may create a tmp file in /tmp to hold info about traceback.
 * Original version developed under BSD 4.3 on a vax 8650
 */
#include "nw.h"
#include "day.h"

static    _dbval[26] = {
          1,14,2,13,0,0,4,11,0,0,12,0,3,15,0,0,0,5,6,8,8,7,9,0,10,0
};

static    _pbval[26] = {
          1, 2|(1<<('D'-'A'))|(1<<('N'-'A')), 4, 8, 16, 32, 64,
          128, 256, 0xFFFFFFF, 1<<10, 1<<11, 1<<12, 1<<13, 1<<14,
          1<<15, 1<<16, 1<<17, 1<<18, 1<<19, 1<<20, 1<<21, 1<<22,
          1<<23, 1<<24, 1<<25|(1<<('E'-'A'))|(1<<('Q'-'A'))
};

main(ac, av)                                                        main
          int       ac;
          char      *av[];
{
          prog = av[0];
          if (ac != 3) {
                    fprintf(stderr,"usage: %s file1 file2\n", prog);
                    fprintf(stderr,"where file1 and file2 are two dna or two protein sequences.\n");
                    fprintf(stderr,"The sequences can be in upper- or lower-case\n");
                    fprintf(stderr,"Any lines beginning with ';' or '<' are ignored\n");
                    fprintf(stderr,"Output is in the file \"align.out\"\n");
                    exit(1);
          }
          namex[0] = av[1];
          namex[1] = av[2];
          seqx[0] = getseq(namex[0], &len0);
          seqx[1] = getseq(namex[1], &len1);
          xbm = (dna)? _dbval : _pbval;

          endgaps = 0;                      /* 1 to penalize endgaps */
          ofile = "align.out";              /* output file */

          nw();               /* fill in the matrix, get the possible jmps */
          readjmps();         /* get the actual jmps */
          print();            /* print stats, alignment */

          cleanup(0);         /* unlink any tmp files */
}
```

## Table 1 (cont')

```
/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()                                                                                    nw
{
        char            *px, *py;               /* seqs and ptrs */
        int             *ndely, *dely;  /* keep track of dely */
        int             ndelx, delx;    /* keep track of delx */
        int             *tmp;           /* for swapping row0, row1 */
        int             mis;            /* score for each type */
        int             ins0, ins1;     /* insertion penalties */
        register        id;             /* diagonal index */
        register        ij;             /* jmp index */
        register        *col0, *col1;   /* score for curr, last row */
        register        xx, yy;         /* index into seqs */

        dx = (struct diag *)g_calloc("to get diags", len0+len1+1, sizeof(struct diag));

        ndely = (int *)g_calloc("to get ndely", len1+1, sizeof(int));
        dely = (int *)g_calloc("to get dely", len1+1, sizeof(int));
        col0 = (int *)g_calloc("to get col0", len1+1, sizeof(int));
        col1 = (int *)g_calloc("to get col1", len1+1, sizeof(int));
        ins0 = (dna)? DINS0 : PINS0;
        ins1 = (dna)? DINS1 : PINS1;

        smax = -10000;
        if (endgaps) {
                for (col0[0] = dely[0] = -ins0, yy = 1; yy <= len1; yy++) {
                        col0[yy] = dely[yy] = col0[yy-1] - ins1;
                        ndely[yy] = yy;
                }
                col0[0] = 0;    /* Waterman Bull Math Biol 84 */
        }
        else
                for (yy = 1; yy <= len1; yy++)
                        dely[yy] = -ins0;

        /* fill in match matrix
         */
        for (px = seqx[0], xx = 1; xx <= len0; px++, xx++) {
                /* initialize first entry in col
                 */
                if (endgaps) {
                        if (xx == 1)
                                col1[0] = delx = -(ins0+ins1);
                        else
                                col1[0] = delx = col0[0] - ins1;
                        ndelx = xx;
                }
                else {
                        col1[0] = 0;
                        delx = -ins0;
                        ndelx = 0;
                }
```

## Table 1 (cont')

...nw

```
for (py = seqx[1], yy = 1; yy <= len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];

        /* update penalty for del in x seq;
         * favor new del over ongong del
         * ignore MAXGAP if weighting endgaps
         */
        if (endgaps || ndely[yy] < MAXGAP) {
                if (col0[yy] - ins0 >= dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) >= dely[yy]) {
                        dely[yy] = col0[yy] - (ins0+ins1);
                        ndely[yy] = 1;
                } else
                        ndely[yy]++;
        }

        /* update penalty for del in y seq;
         * favor new del over ongong del
         */
        if (endgaps || ndelx < MAXGAP) {
                if (col1[yy-1] - ins0 >= delx) {
                        delx = col1[yy-1] - (ins0+ins1);
                        ndelx = 1;
                } else {
                        delx -= ins1;
                        ndelx++;
                }
        } else {
                if (col1[yy-1] - (ins0+ins1) >= delx) {
                        delx = col1[yy-1] - (ins0+ins1);
                        ndelx = 1;
                } else
                        ndelx++;
        }

        /* pick the maximum score; we're favoring
         * mis over any del and delx over dely
         */
```

**Table 1 (cont')**

**...nw**

```
				id = xx - yy + len1 - 1;
				if (mis >= delx && mis >= dely[yy])
					col1[yy] = mis;
				else if (delx >= dely[yy]) {
					col1[yy] = delx;
					ij = dx[id].ijmp;
					if (dx[id].jp.n[0] && (!dna || (ndelx >= MAXJMP
					&& xx > dx[id].jp.x[ij]+MX) || mis > dx[id].score+DINS0)) {
						dx[id].ijmp++;
						if (++ij >= MAXJMP) {
							writejmps(id);
							ij = dx[id].ijmp = 0;
							dx[id].offset = offset;
							offset += sizeof(struct jmp) + sizeof(offset);
						}
					}
					dx[id].jp.n[ij] = ndelx;
					dx[id].jp.x[ij] = xx;
					dx[id].score = delx;
				}
				else {
					col1[yy] = dely[yy];
					ij = dx[id].ijmp;
	if (dx[id].jp.n[0] && (!dna || (ndely[yy] >= MAXJMP
					&& xx > dx[id].jp.x[ij]+MX) || mis > dx[id].score+DINS0)) {
						dx[id].ijmp++;
						if (++ij >= MAXJMP) {
							writejmps(id);
							ij = dx[id].ijmp = 0;
							dx[id].offset = offset;
							offset += sizeof(struct jmp) + sizeof(offset);
						}
					}
					dx[id].jp.n[ij] = -ndely[yy];
					dx[id].jp.x[ij] = xx;
					dx[id].score = dely[yy];
				}
				if (xx == len0 && yy < len1) {
					/* last col
					 */
					if (endgaps)
						col1[yy] -= ins0+ins1*(len1-yy);
					if (col1[yy] > smax) {
						smax = col1[yy];
						dmax = id;
					}
				}
			}
			if (endgaps && xx < len0)
				col1[yy-1] -= ins0+ins1*(len0-xx);
			if (col1[yy-1] > smax) {
				smax = col1[yy-1];
				dmax = id;
			}
			tmp = col0; col0 = col1; col1 = tmp;
	}
	(void) free((char *)ndely);
	(void) free((char *)dely);
	(void) free((char *)col0);
	(void) free((char *)col1);			}
```

## Table 1 (cont')

```
/*
 *
 * print() -- only routine visible outside this module
 *
 * static:
 * getmat() -- trace back best path, count matches: print()
 * pr_align() -- print alignment of described in array p[]: print()
 * dumpblock() -- dump a block of lines with numbers, stars: pr_align()
 * nums() -- put out a number line: dumpblock()
 * putline() -- put out a line (name, [num], seq, [num]): dumpblock()
 * stars() - -put a line of stars: dumpblock()
 * stripname() -- strip any path and prefix from a seqname
 */

#include "nw.h"

#define SPC       3
#define P_LINE    256     /* maximum output line */
#define P_SPC     3       /* space between name or num and seq */

extern   _day[26][26];
int      olen;            /* set output line length */
FILE     *fx;             /* output file */

print()                                                              print
{
        int     lx, ly, firstgap, lastgap;      /* overlap */

        if ((fx = fopen(ofile, "w")) == 0) {
                fprintf(stderr,"%s: can't write %s\n", prog, ofile);
                cleanup(1);
        }
        fprintf(fx, "<first sequence: %s (length = %d)\n", namex[0], len0);
        fprintf(fx, "<second sequence: %s (length = %d)\n", namex[1], len1);
        olen = 60;
        lx = len0;
        ly = len1;
        firstgap = lastgap = 0;
        if (dmax < len1 - 1) {          /* leading gap in x */
                pp[0].spc = firstgap = len1 - dmax - 1;
                ly -= pp[0].spc;
        }
        else if (dmax > len1 - 1) {  /* leading gap in y */
                pp[1].spc = firstgap = dmax - (len1 - 1);
                lx -= pp[1].spc;
        }
        if (dmax0 < len0 - 1) {         /* trailing gap in x */
                lastgap = len0 - dmax0 -1;
                lx -= lastgap;
        }
        else if (dmax0 > len0 - 1) { /* trailing gap in y */
                lastgap = dmax0 - (len0 - 1);
                ly -= lastgap;
        }
        getmat(lx, ly, firstgap, lastgap);
        pr_align();
}
```

## Table 1 (cont')

getmat

```
/*
 * trace back the best path, count matches
 */
static
getmat(lx, ly, firstgap, lastgap)
        int     lx, ly;                 /* "core" (minus endgaps) */
        int     firstgap, lastgap;      /* leading trailing overlap */
{
        int             nm, i0, i1, siz0, siz1;
        char            outx[32];
        double          pct;
        register        n0, n1;
        register char   *p0, *p1;

        /* get total matches, score
         */
        i0 = i1 = siz0 = siz1 = 0;
        p0 = seqx[0] + pp[1].spc;
        p1 = seqx[1] + pp[0].spc;
        n0 = pp[1].spc + 1;
        n1 = pp[0].spc + 1;

        nm = 0;
        while ( *p0 && *p1 ) {
                if (siz0) {
                        p1++;
                        n1++;
                        siz0--;
                }
                else if (siz1) {
                        p0++;
                        n0++;
                        siz1--;
                }
                else {
                        if (xbm[*p0-'A']&xbm[*p1-'A'])
                                nm++;
                        if (n0++ == pp[0].x[i0])
                                siz0 = pp[0].n[i0++];
                        if (n1++ == pp[1].x[i1])
                                siz1 = pp[1].n[i1++];
                        p0++;
                        p1++;
                }
        }

        /* pct homology:
         * if penalizing endgaps, base is the shorter seq
         * else, knock off overhangs and take shorter core
         */
        if (endgaps)
                lx = (len0 < len1)? len0 : len1;
        else
                lx = (lx < ly)? lx : ly;
        pct = 100.*(double)nm/(double)lx;
        fprintf(fx, "\n");
        fprintf(fx, "<%d match%s in an overlap of %d: %.2f percent similarity\n",
                nm, (nm == 1)? "" : "es", lx, pct);
```

## Table 1 (cont')

...getmat

```
        fprintf(fx, "<gaps in first sequence: %d", gapx);
        if (gapx) {
                (void) sprintf(outx, " (%d %s%s)",
                        ngapx, (dna)? "base":"residue", (ngapx == 1)? "":"s");
                fprintf(fx,"%s", outx);

        fprintf(fx, ", gaps in second sequence: %d", gapy);
        if (gapy) {
                (void) sprintf(outx, " (%d %s%s)",
                        ngapy, (dna)? "base":"residue", (ngapy == 1)? "":"s");
                fprintf(fx,"%s", outx);
        }
        if (dna)
                fprintf(fx,
                "\n<score: %d (match = %d, mismatch = %d, gap penalty = %d + %d per base)\n",
                smax, DMAT, DMIS, DINS0, DINS1);
        else
                fprintf(fx,
                "\n<score: %d (Dayhoff PAM 250 matrix, gap penalty = %d + %d per residue)\n",
                smax, PINS0, PINS1);
        if (endgaps)
                fprintf(fx,
                "<endgaps penalized. left endgap: %d %s%s, right endgap: %d %s%s\n",
                firstgap, (dna)? "base" : "residue", (firstgap == 1)? "" : "s",
                lastgap, (dna)? "base" : "residue", (lastgap == 1)? "" : "s");
        else
                fprintf(fx, "<endgaps not penalized\n");
}

static          nm;             /* matches in core -- for checking */
static          lmax;           /* lengths of stripped file names */
static          ij[2];          /* jmp index for a path */
static          nc[2];          /* number at start of current line */
static          ni[2];          /* current elem number -- for gapping */
static          siz[2];
static char     *ps[2];         /* ptr to current element */
static char     *po[2];         /* ptr to next output char slot */
static char     out[2][P_LINE]; /* output line */
static char     star[P_LINE];   /* set by stars() */

/*
 * print alignment of described in struct path pp[]
 */
static
pr_align()
{
        int             nn;     /* char count */
        int             more;
        register        i;

        for (i = 0, lmax = 0; i < 2; i++) {
                nn = stripname(namex[i]);
                if (nn > lmax)
                        lmax = nn;

                nc[i] = 1;
                ni[i] = 1;
                siz[i] = ij[i] = 0;
                ps[i] = seqx[i];
                po[i] = out[i];         }
```

pr_align

## Table 1 (cont')

...pr_align

```
        for (nn = nm = 0, more = 1; more; ) {
                for (i = more = 0; i < 2; i++) {
                        /*
                         * do we have more of this sequence?
                         */
                        if (!*ps[i])
                                continue;

                        more++;

                        if (pp[i].spc) {        /* leading space */
                                *po[i]++ = ' ';
                                pp[i].spc--;
                        }
                        else if (siz[i]) {      /* in a gap */
                                *po[i]++ = '-';
                                siz[i]--;
                        }
                        else {                  /* we're putting a seq element
                                                 */
                                *po[i] = *ps[i];
                                if (islower(*ps[i]))
                                        *ps[i] = toupper(*ps[i]);
                                po[i]++;
                                ps[i]++;

                                /*
                                 * are we at next gap for this seq?
                                 */
                                if (ni[i] == pp[i].x[ij[i]]) {
                                        /*
                                         * we need to merge all gaps
                                         * at this location
                                         */
                                        siz[i] = pp[i].n[ij[i]++];
                                        while (ni[i] == pp[i].x[ij[i]])
                                                siz[i] += pp[i].n[ij[i]++];
                                }
                                ni[i]++;
                        }
                }
                if (++nn == olen || !more && nn) {
                        dumpblock();
                        for (i = 0; i < 2; i++)
                                po[i] = out[i];
                        nn = 0;
                }
        }
}

/*
 * dump a block of lines, including numbers, stars: pr_align()
 */
static
dumpblock()
{
        register i;

        for (i = 0; i < 2; i++)
                *po[i]-- = '\0';
```

dumpblock

## Table 1 (cont')

**...dumpblock**

```
        (void) putc('\n', fx);
        for (i = 0; i < 2; i++) {
                if (*out[i] && (*out[i] != ' ' || *(po[i]) != ' ')) {
                        if (i == 0)
                                nums(i);
                        if (i == 0 && *out[1])
                                stars();
                        putline(i);
                        if (i == 0 && *out[1])
                                fprintf(fx, star);
                        if (i == 1)
                                nums(i);
                }
        }
}

/*
 * put out a number line: dumpblock()
 */
static
nums(ix)
        int     ix;     /* index in out[] holding seq line */
{
        char            nline[P_LINE];
        register        i, j;
        register char   *pn, *px, *py;

        for (pn = nline, i = 0; i < lmax+P_SPC; i++, pn++)
                *pn = ' ';
        for (i = nc[ix], py = out[ix]; *py; py++, pn++) {
                if (*py == ' ' || *py == '-')
                        *pn = ' ';
                else {
                        if (i%10 == 0 || (i == 1 && nc[ix] != 1)) {
                                j = (i < 0)? -i : i;
                                for (px = pn; j; j /= 10, px--)
                                        *px = j%10 + '0';
                                if (i < 0)
                                        *px = '-';
                        }
                        else
                                *pn = ' ';
                        i++;
                }
        }
        *pn = '\0';
        nc[ix] = i;
        for (pn = nline; *pn; pn++)
                (void) putc(*pn, fx);
        (void) putc('\n', fx);
}

/*
 * put out a line (name, [num], seq, [num]): dumpblock()
 */
static
putline(ix)
        int     ix;             {
```

**nums**

**putline**

## Table 1 (cont')

...putline

```
	int		i;
	register char	*px;

	for (px = namex[ix], i = 0; *px && *px != ':'; px++, i++)
		(void) putc(*px, fx);
	for (; i < lmax+P_SPC; i++)
		(void) putc(' ', fx);

	/* these count from 1:
	 * ni[] is current element (from 1)
	 * nc[] is number at start of current line
	 */
	for (px = out[ix]; *px; px++)
		(void) putc(*px&0x7F, fx);
	(void) putc('\n', fx);
}


/*
 * put a line of stars (seqs always in out[0], out[1]): dumpblock()
 */
static
stars()
{
	int		i;
	register char	*p0, *p1, cx, *px;

	if (!*out[0] || (*out[0] == ' ' && *(po[0]) == ' ') ||
	    !*out[1] || (*out[1] == ' ' && *(po[1]) == ' '))
		return;
	px = star;
	for (i = lmax+P_SPC; i; i--)
		*px++ = ' ';

	for (p0 = out[0], p1 = out[1]; *p0 && *p1; p0++, p1++) {
		if (isalpha(*p0) && isalpha(*p1)) {

			if (xbm[*p0-'A']&xbm[*p1-'A']) {
				cx = '*';
				nm++;
			}
			else if (!dna && _day[*p0-'A'][*p1-'A'] > 0)
				cx = '.';
			else
				cx = ' ';
		}
		else
			cx = ' ';
		*px++ = cx;
	}
	*px++ = '\n';
	*px = '\0';
}
```

stars

## Table 1 (cont')

stripname

```
/*
 * strip path or prefix from pn, return len: pr_align()
 */
static
stripname(pn)
        char    *pn;    /* file name (may be path) */
{
        register char   *px, *py;

        py = 0;
        for (px = pn; *px; px++)
                if (*px == '/')
                        py = px + 1;
        if (py)
                (void) strcpy(pn, py);
        return(strlen(pn));

}
```

## Table 1 (cont')

```
/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>

char    *jname = "/tmp/homgXXXXXX";          /* tmp file for jmps */
FILE    *fj;

int     cleanup();                           /* cleanup tmp file */
long    lseek();

/*
 * remove any tmp file if we blow
 */
cleanup(i)                                                              cleanup
        int     i;
{
        if (fj)
                (void) unlink(jname);
        exit(i);
}

/*
 * read, return ptr to seq, set dna, len, maxlen
 * skip lines starting with ';', '<', or '>'
 * seq in upper or lower case
 */
char    *
getseq(file, len)                                                       getseq
        char    *file;  /* file name */
        int     *len;   /* seq len */
{
        char            line[1024], *pseq;
        register char   *px, *py;
        int             natgc, tlen;
        FILE            *fp;

        if ((fp = fopen(file,"r")) == 0) {
                fprintf(stderr,"%s: can't read %s\n", prog, file);
                exit(1);
        }
        tlen = natgc = 0;
        while (fgets(line, 1024, fp)) {
                if (*line == ';' || *line == '<' || *line == '>')
                        continue;
                for (px = line; *px != '\n'; px++)
                        if (isupper(*px) || islower(*px))
                                tlen++;
        }
        if ((pseq = malloc((unsigned)(tlen+6))) == 0) {
                fprintf(stderr,"%s: malloc() failed to get %d bytes for %s\n", prog, tlen+6, file);
                exit(1);
        }
        pseq[0] = pseq[1] = pseq[2] = pseq[3] = '\0';
```

Table 1 (cont')

**...getseq**

```
        py = pseq + 4;
        *len = tlen;
        rewind(fp);

        while (fgets(line, 1024, fp)) {
                if (*line == ';' || *line == '<' || *line == '>')
                        continue;
                for (px = line; *px != '\n'; px++) {
                        if (isupper(*px))
                                *py++ = *px;
                        else if (islower(*px))
                                *py++ = toupper(*px);
                        if (index("ATGCU",*(py-1)))
                                natgc++;
                }
        }
        *py++ = '\0';
        *py = '\0';
        (void) fclose(fp);
        dna = natgc > (tlen/3);
        return(pseq+4);
}

char    *
g_calloc(msg, nx, sz)
        char    *msg;           /* program, calling routine */
        int     nx, sz;         /* number and size of elements */
{
        char            *px, *calloc();

        if ((px = calloc((unsigned)nx, (unsigned)sz)) == 0) {
                if (*msg) {
                        fprintf(stderr, "%s: g_calloc() failed %s (n=%d, sz=%d)\n", prog, msg, nx, sz);
                        exit(1);
                }
        }
        return(px);
}
```

**g_calloc**

```
/*
 * get final jmps from dx[] or tmp file, set pp[], reset dmax: main()
 */
readjmps()
{
        int             fd = -1;
        int             siz, i0, i1;
        register i, j, xx;

        if (fj) {
                (void) fclose(fj);
                if ((fd = open(jname, O_RDONLY, 0)) < 0) {
                        fprintf(stderr, "%s: can't open() %s\n", prog, jname);
                        cleanup(1);
                }
        }
        for (i = i0 = i1 = 0, dmax0 = dmax, xx = len0; ; i++) {
                while (1) {
                        for (j = dx[dmax].ijmp; j >= 0 && dx[dmax].jp.x[j] >= xx; j--)
                                ;
```

**readjmps**

## Table 1 (cont')

**...readjmps**

```
                        if (j < 0 && dx[dmax].offset && fj) {
                                (void) lseek(fd, dx[dmax].offset, 0);
                                (void) read(fd, (char *)&dx[dmax].jp, sizeof(struct jmp));
                                (void) read(fd, (char *)&dx[dmax].offset, sizeof(dx[dmax].offset));
                                dx[dmax].ijmp = MAXJMP-1;
                        }
                        else
                                break;
                }
                if (i >= JMPS) {
                        fprintf(stderr, "%s: too many gaps in alignment\n", prog);
                        cleanup(1);
                }
                if (j >= 0) {
                        siz = dx[dmax].jp.n[j];
                        xx = dx[dmax].jp.x[j];
                        dmax += siz;
                        if (siz < 0) {                  /* gap in second seq */
                                pp[1].n[i1] = -siz;
                                xx += siz;
                                /* id = xx - yy + len1 - 1
                                 */
                                pp[1].x[i1] = xx - dmax + len1 - 1;
                                gapy++;
                                ngapy -= siz;
/* ignore MAXGAP when doing endgaps */
                                siz = (-siz < MAXGAP || endgaps)? -siz : MAXGAP;
                                i1++;
                        }
                        else if (siz > 0) { /* gap in first seq */
                                pp[0].n[i0] = siz;
                                pp[0].x[i0] = xx;
                                gapx++;
                                ngapx += siz;
/* ignore MAXGAP when doing endgaps */
                                siz = (siz < MAXGAP || endgaps)? siz : MAXGAP;
                                i0++;
                        }
                }
                else
                        break;
        }

        /* reverse the order of jmps
         */
        for (j = 0, i0--; j < i0; j++, i0--) {
                i = pp[0].n[j]; pp[0].n[j] = pp[0].n[i0]; pp[0].n[i0] = i;
                i = pp[0].x[j]; pp[0].x[j] = pp[0].x[i0]; pp[0].x[i0] = i;
        }
        for (j = 0, i1--; j < i1; j++, i1--) {
                i = pp[1].n[j]; pp[1].n[j] = pp[1].n[i1]; pp[1].n[i1] = i;
                i = pp[1].x[j]; pp[1].x[j] = pp[1].x[i1]; pp[1].x[i1] = i;
        }
        if (fd >= 0)
                (void) close(fd);
        if (fj) {
                (void) unlink(jname);
                fj = 0;
                offset = 0;
        }               }
```

## Table 1 (cont')

```
/*
 * write a filled jmp struct offset of the prev one (if any): nw()
 */
writejmps(ix)                                                   writejmps
        int     ix;
{
        char    *mktemp();

        if (!fj) {
                if (mktemp(jname) < 0) {
                        fprintf(stderr, "%s: can't mktemp() %s\n", prog, jname);
                        cleanup(1);
                }
                if ((fj = fopen(jname, "w")) == 0) {
                        fprintf(stderr, "%s: can't write %s\n", prog, jname);
                        exit(1);
                }
        }
        (void) fwrite((char *)&dx[ix].jp, sizeof(struct jmp), 1, fj);
        (void) fwrite((char *)&dx[ix].offset, sizeof(dx[ix].offset), 1, fj);
}
```

Table 2

| | | |
|---|---|---|
| PRO | XXXXXXXXXXXXXXX | (Length = 15 amino acids) |
| Comparison Protein | XXXXXYYYYYYY | (Length = 12 amino acids) |

% amino acid sequence identity =

(the number of identically matching amino acid residues

between the two polypeptide sequences as

determined by ALIGN-2) divided by (the total number of amino acid residues of the PRO polypeptide) =

5 divided by 15 = 33.3%

Table 3

| | | |
|---|---|---|
| PRO | XXXXXXXXXX | (Length = 10 amino acids) |
| Comparison Protein | XXXXXYYYYYYZZYZ | (Length = 15 amino acids) |

% amino acid sequence identity =

(the number of identically matching

amino acid residues between the two polypeptide sequences as

determined by ALIGN-2) divided by (the total number of amino acid residues of the PRO polypeptide) =

5 divided by 10 = 50%

Table 4

| | | |
|---|---|---|
| PRO-DNA<br>Comparison DNA | NNNNNNNNNNNNNN<br>NNNNNNLLLLLLLLLL | (Length = 14 nucleotides)<br>(Length = 16 nucleotides) |

% nucleic acid sequence identity =

(the number of identically matching nucleotides

between the two nucleic acid sequences as determined by

ALIGN-2) divided by (the total number of nucleotides of the PRO-DNA nucleic acid sequence) =

6 divided by 14 = 42.9%

Table 5

| | | |
|---|---|---|
| PRO-DNA<br>Comparison DNA | NNNNNNNNNNNN<br>NNNNLLLVV | (Length = 12 nucleotides)<br>(Length = 9 nucleotides) |

% nucleic acid sequence identity =

(the number of identically matching nucleotides

between the two nucleic acid sequences as determined by

ALIGN-2) divided by (the total number of nucleotides of the PRO-DNA nucleic acid sequence) =

4 divided by 12 = 33.3%

II. Compositions and Methods of the Invention

1. Preparation of the polypeptides of the invention

The present invention provides newly identified and isolated nucleotide sequences encoding polypeptides referred to in the present application as PRO polypeptides. In particular, cDNAs encoding various PRO polypeptides have been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the protein encoded by the full length native nucleic acid molecules disclosed herein as well as all further native homologues and variants included in the foregoing definition of PRO, will be referred to as "PRO" or "PRO/number", regardless of their origin or mode of preparation.

As disclosed in the Examples below, various cDNA clones have been deposited with the ATCC. The actual nucleotide sequences of those clones can readily be determined by the skilled artisan by sequencing of the deposited clone using routine methods in the art. The predicted amino acid sequence can be determined from the nucleotide sequence using routine skill. For the PRO polypeptides and encoding nucleic acids described herein, Applicants have identified what is believed to be the reading frame best identifiable with the sequence information available at the time.

A. PRO Polypentides

The description below relates primarily to production of the PRO polypeptide of the invention by culturing cells transformed or transfected with a vector containing nucleic acid which encodes of the PRO polypeptide of the invention . It is, of course, contemplated that alternative methods, which are well known in the art, may be employed to prepare of the PRO polypeptide of the invention. For instance, the polypeptide sequence, or portions thereof, may be produced by direct peptide synthesis using solid-phase techniques [see, e.g., Stewart et al., Solid-Phase Peptide Synthesis, W. H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO polypeptide of the invention may be chemically synthesized separately and combined using chemical or enzymatic methods to produce the full-length polypeptide.

B. PRO Polypeptide Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO, such as changing the number or position of glycosylation sites or altering the membrane anchoring characteristics.

Variations in the native full-length sequence PRO or in various domains of the PRO described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO that results in a change in the amino acid sequence of the PRO as compared with the native sequence PRO. Optionally the variation is by substitution of at least one amino acid with any other amino acid in one or more of the domains of the PRO. Guidance in determining which amino acid residue may be inserted, substituted or deleted without adversely affecting the desired activity may be found by comparing the sequence of the PRO with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide.

PRO fragments may be prepared by any of a number of conventional techniques. Desired peptide fragments may be chemically synthesized. An alternative approach involves generating PRO fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide disclosed herein.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

Table 6

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |

Table 6 (continued)

| Original Residue | Exemplary Substitutions | Preferred Substitutions |
|---|---|---|
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13: 4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

C. Modifications of PRO

Covalent modifications of PRO are included within the scope of this invention. One type of covalent modification includes reacting targeted amino acid residues of a PRO polypeptide with an organic derivatizing agent that is capable of reacting with selected side chains or the N- or C- terminal residues of the PRO. Derivatization with bifunctional agents is useful, for instance, for crosslinking PRO to a water-insoluble support matrix or surface for use in the method for purifying anti-PRO antibodies, and vice-versa. Commonly used crosslinking agents include, e.g., 1,1-bis (diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO (either by removing the underlying glycosylation site or by deleting the glycosylation by chemical and/or enzymatic means), and/or adding one or more glycosylation sites that are not present in the native sequence PRO. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO (for O-linked glycosylation sites). The PRO amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO comprises linking the PRO polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO of the present invention may also be modified in a way to form a chimeric molecule comprising PRO fused to another, heterologous polypeptide or amino acid sequence.

In one embodiment, such a chimeric molecule comprises a fusion of the PRO with a tag polypeptide which provides an epitope to which an anti-tag antibody can selectively bind. The epitope tag is generally placed at the amino- or carboxyl- terminus of the PRO. The presence of such epitope-tagged forms of the PRO can be detected using an antibody against the tag polypeptide. Also, provision of the epitope tag enables the PRO to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8: 2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

D. Preparation of PRO

The description below relates primarily to production of PRO by culturing cells transformed or transfected with a vector containing PRO nucleic acid. It is, of course, contemplated that alternative methods, which are well known in the art, may be employed to prepare PRO. For instance, the PRO sequence, or portions thereof, may be produced by direct peptide synthesis using solid-phase techniques [see, e.g., Stewart et al., Solid-Phase Peptide Synthesis, W. H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO may be chemically synthesized separately and combined using chemical or enzymatic methods to produce the full-length PRO.

1. Isolation of DNA Encoding PRO

DNA encoding PRO may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures (e.g., automated nucleic acid synthesis).

Libraries can be screened with probes (such as antibodies to the PRO or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like $^{32}P$-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

2. Selection and Transformation of Host Cells

Host cells are transfected or transformed with expression or cloning vectors described herein for PRO production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, $CaCl_2$, $CaPO_4$, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23: 315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli*. Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia*, e.g., *E. coli*, *Enterobacter*, *Erwinia*, *Klebsiella*, *Proteus, Salmonella,* e.g., *Salmonella typhimurium*, *Serratia*, e.g., *Serratia marcescans*, and *Shigella,* as well as *Bacilli* such as *B. subtilis* and *B. licheniformis* (e.g., *B. licheniformis* 41 disclosed in DD 266,710 published 12 April 1989), *Pseudomonas* such as *P. aeruginosa*, and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA* ; *E. coli* W3110 strain 9E4, which has the complete genotype *tonA ptr3*; *E. coli* W3110 strain 27C7 (ATCC 55,244), which has the complete genotype *tonA ptr3 phoA E15 (argF-lac)169 degP ompT* $kan^r$; *E. coli* W3110 strain 37D6, which has the complete genotype *tonA ptr3 phoA E15 (argF-lac)169 degP ompT rbs7 ilvG* $kan^r$; *E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus (ATCC* 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans*, and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida*, *Kloeckera*, *Pichia*, *Saccharomyces. Torulopsis,* and *Rhodotorula*. A list of specific species that are exemplary of this class of yeasts may be found in C. Anthony, The Biochemistry of Methylotrophs, 269 (1982).

Suitable host cells for the expression of glycosylated PRO are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7. ATCC CRL 1651): human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

3. Selection and Use of a Replicable Vector

The nucleic acid (e.g., cDNA or genomic DNA) encoding PRO may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site (s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7: 141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36, 776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the genomes of viruses such as polyoma virus, fowlpox virus (UK 2.211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO by higher eukaryotes may be increased by inserting an enhancer sequence into the vector. Enhancers are cis-acting elements of DNA. usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO.

Still other methods, vectors, and host cells suitable for adaptation to the synthesis of PRO in recombinant vertebrate cell culture are described in Gething et al., Nature, 293:620-625 (1981); Mantei et al., Nature, 281:40-46 (1979); EP 117,060; and EP 117,058.

4. Detecting Gene Expression

Gene expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

5. Purification of Polypeptide

Forms of PRO may be recovered from culture medium or from host cell lysates. If membrane-bound, it can be released from the membrane using a suitable detergent solution (e.g. Triton-X 100) or by enzymatic cleavage. Cells employed in expression of PRO can be disrupted by various physical or chemical means, such as freeze-thaw cycling, sonication, mechanical disruption, or cell lysing agents.

It may be desired to purify PRO from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG: and metal chelating columns to bind epitope-tagged forms of the PRO. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO produced.

E. Tissue Distribution

The location of tissues expressing the PRO polypeptides of the invention can be identified by determining mRNA expression in various human tissues. The location of such genes provides information about which tissues are most likely to be affected by the stimulating and inhibiting activities of the PRO polypeptides of the invention. The location of a gene in a specific tissue also provides sample tissue for the activity blocking assays discussed below.

As noted before, gene expression in various tissues may be measured by conventional Southern blotting, Northern blotting to quantitate the transcription of mRNA (Thomas, Proc. Natl. Acad. Sci. USA, 77:5201-5205 [1980]), dot blotting (DNA analysis), or in situ hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence of a PRO polypeptide of the invention or against a synthetic peptide based on the DNA sequences encoding the PRO polypeptide of the invention or against an exogenous sequence fused to a DNA encoding a PRO polypeptide of the invention and encoding a specific antibody epitope. General techniques for generating antibodies, and special protocols for Northern blotting and *in situ* hybridization are provided below.

F. Antibody Binding Studies

The activity of the polypeptides of the invention can be further verified by antibody binding studies, in which the ability of anti-PRO179, PRO175, PRO182, PRO366, PRO240, PRO256, PRO306, PRO364, PRO356, PRO826, PRO1068, PRO1343 or PRO1375 antibodies to inhibit the effect of the PRO179, PRO175, PRO182, PRO366, PRO240, PRO256, PRO306, PRO364, PRO356, PRO826, PRO1068, PRO1343 or PRO1375 polypeptides on tissue cells is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques, pp. 147-158 (CRC Press, Inc., 1987).

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, e.g., US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tissue sample may be fresh or frozen or may be embedded in paraffin and fixed with a preservative such as formalin, for example.

G. Cell-Based Assays

Cell-based assays and animal models for immune related diseases can be used to further understand the relationship between the genes and polypeptides identified herein and the development and pathogenesis of immune related disease.

In a different approach, cells of a cell type known to be involved in a particular immune related disease are transfected with the cDNAs described herein, and the ability of these cDNAs to stimulate or inhibit immune function is analyzed. Suitable cells can be transfected with the desired gene, and monitored for immune function activity. Such transfected cell lines can then be used to test the ability of poly- or monoclonal antibodies or antibody compositions to inhibit or stimulate immune function, for example to modulate T-cell proliferation or inflammatory cell infiltration. Cells transfected with the coding sequences of the genes identified herein can further be used to identify drug candidates for the treatment of immune related diseases.

In addition, primary cultures derived from transgenic animals (as described below) can be used in the cell-based assays herein, although stable cell lines are preferred. Techniques to derive continuous cell lines from transgenic animals are well known in the art (see, e.g. Small et al., Mol. Cell. Biol. 5, 642-648 [1985]).

One suitable cell based assay is the mixed lymphocyte reaction (MLR). Current Protocols in Immunology, unit 3.12; edited by JE Coligan, AM Kruisbeek, DH Marglies, EM Shevach, W Strober, National Insitutes of Health, Published by John Wiley & Sons, Inc. In this assay, the ability of a test compound to stimulate or inhibit the proliferation of activated T cells is assayed. A suspension of responder T cells is cultured with allogeneic stimulator cells and the proliferation of T cells is measured by uptake of tritiated thymidine. This assay is a general measure of T cell reactivity. Since the majority of T cells respond to and produce IL-2 upon activation, differences in responsiveness in this assay in part reflect differences in IL-2 production by the responding cells. The MLR results can be verified by a standard lymphokine (IL-2) detection assay. Current Protocols in Immunology, above, 3.15, 6.3.

A proliferative T cell response in an MLR assay may be due to direct mitogenic properties of an assayed molecule or to external antigen induced activation. Additional verification of the T cell stimulatory activity of the polypeptides of the invention can be obtained by a costimulation assay. T cell activation requires an antigen specific signal mediated through the T-cell receptor (TCR) and a costimulatory signal mediated through a second ligand binding interaction, for example, the B7(CD80, CD86)/CD28 binding interaction. CD28 crosslinking increases lymphokine secretion by activated T cells. T cell activation has both negative and positive controls through the binding of ligands which have a negative or positive effect. CD28 and CTLA-4 are related glycoproteins in the Ig superfamily which bind to B7. CD28 binding to B7 has a positive costimulation effect of T cell activation; conversely, CTLA-4 binding to B7 has a negative T cell deactivating effect. Chambers and Allison, Curr. Opin. Immunol. (1997) 9:396. Schwartz, Cell (1992) 71:1065; Linsey, and Ledbetter, Annu. Rev. Immunol. (1993) 11:191; June et al, Immunol. Today (1994) 15: 321; Jenkins, Immunity (1994) 1:405. In a costimulation assay, the PRO polypeptides of the invention are assayed for T cell costimulatory or inhibitory activity.

PRO polypeptides of the invention, as well as other compounds of the invention, which are stimulators (costimulators) ofT cell proliferation and agonists , e.g. agonist antibodies, thereto as determined by MLR and costimulation assays, for example, are useful in treating immune related diseases characterized by poor, suboptimal or inadequate immune function. These diseases are treated by stimulating the proliferation and activation of T cells (and T cell mediated immunity) and enhancing the immune response in a mammal through administration of a stimulatory compound, such as the stimulating polypeptides of the invention. The stimulating polypeptide may, for example, be a PRO179, PRO175, PRO182, PRO366, PRO240, PRO256, PRO306, PRO364, PRO356, PRO826, PRO1068, PRO1343 or PRO1375 polypeptide or an agonist antibody therefor.

Direct use of a stimulating compound as in the invention has been validated in experiments with 4-1BB glycoprotein, a member of the tumor necrosis factor receptor family, which binds to a ligand (4-1BBL) expressed on primed T cells and signals T cell activation and growth. Alderson et al., J. Immunol. (1994) 24:2219.

The use of an agonist stimulating compound has also been validated experimentally. Activation of 4-1BB by treatment with an agonist anti-4-1BB antibody enhances eradication of tumors. Hellstrom and Hellstrom, Crit. Rev. Immunol. (1998) 18:1. Immunoadjuvant therapy for treatment of tumors, described in more detail below, is another example of the use of the stimulating compounds of the invention.

An immune stimulating or enhancing effect can also be achieved by antagonizing or blocking the activity of a protein which has been found to be inhibiting in the MLR assay. Negating the inhibitory activity of the compound produces a net stimulatory effect. Suitable antagonists/blocking compounds are antibodies or fragments thereof which recognize and bind to the inhibitory protein, thereby blocking the effective interaction of the protein with its receptor and inhibiting signaling through the receptor. This effect has been validated in experiments using anti-CTLA-4 antibodies which enhance T cell proliferation, presumably by removal of the inhibitory signal caused by CTLA-4 binding. Walunas et al, Immunity (1994) 1:405.

On the other hand, PRO polypeptides of the invention, as well as other compounds of the invention, which are direct inhibitors of T cell proliferation/activation and/or lymphokine secretion, can be directly used to suppress the immune response. These compounds are useful to reduce the degree of the immune response and to treat immune related diseases characterized by a hyperactive, superoptimal, or autoimmune response. This use of the compounds of the invention has been validated by the experiments described above in which CTLA-4 binding to receptor B7 deactivates T cells. The direct inhibitory compounds of the invention function in an analogous manner.

Alternatively, compounds, e.g. antibodies, which bind to stimulating PRO polypeptides of the invention and block the stimulating effect of these molecules produce a net inhibitory effect and can be used to suppress the T cell mediated immune response by inhibiting T cell proliferation/activation and/or lymphokine secretion. Blocking the stimulating effect of the PRO polypeptides suppresses the immune response of the mammal. This use has been validated in experiments using an anti-IL2 antibody . In these experiments, the antibody binds to IL2 and blocks binding of IL2 to its receptor thereby achieving a T cell inhibitory effect.

H. Animal Models

The results of the cell based *in vitro* assays can be further verified using *in vivo* animal models and assays for T-cell function. A variety of well known animal models can be used to further understand the role of the genes identified herein in the development and pathogenesis of immune related disease, and to test the efficacy of candidate therapeutic agents, including antibodies, and other antagonists of the native polypeptides, including small molecule antagonists. The *in vivo* nature of such models makes them predictive of responses in human patients. Animal models of immune related diseases include both non-recombinant and recombinant (transgenic) animals. Non-recombinant animal models include, for example, rodent, e.g., murine models. Such models can be generated by introducing cells into syngeneic mice using standard techniques, e.g. subcutaneous injection, tail vein injection, spleen implantation, intraperitoneal implantation, implantation under the renal capsule, etc.

Graft-versus-host disease occurs when immunocompetent cells are transplanted into immunosuppressed or tolerant patients. The donor cells recognize and respond to host antigens. The response can vary from life threatening severe inflammation to mild cases of diarrhea and weight loss. Graft-versus-host disease models provide a means of assessing T cell reactivity against MHC antigens and minor transplant antigens. A suitable procedure is described in detail in Current Protocols in Immunology, above, unit 4.3.

An animal model for skin allograft rejection is a means of testing the ability of T cells to mediate *in vivo* tissue destruction and a measure of their role in transplant rejection. The most common and accepted models use murine tail-skin grafts. Repeated experiments have shown that skin allograft rejection is mediated by T cells, helper T cells and killer-effector T cells, and not antibodies. Auchincloss and Sachs, Fundamental Immunology, 2nd ed., W. E. Paul ed., Raven Press, NY, 1989, 889-992. A suitable procedure is described in detail in Current Protocols in Immunology, above, unit 4.4. Other transplant rejection models which can be used to test the compounds of the invention are the allogeneic heart transplant models described by Tanabe et al., Transplantation (1994) 58:23 and Tinubu et al, J. Immunol. (1994) 4330-4338.

Animal models for delayed type hypersensitivity provides an assay of cell mediated immune function as well. Delayed type hypersensitivity reactions are a T cell mediated in vivo immune response characterized by inflammation which does not reach a peak until after a period of time has elapsed after challenge with an antigen. These reactions also occur in tissue specific autoimmune diseases such as multiple sclerosis (MS) and experimental autoimmune encephalomyelitis (EAE, a model for MS). A suitable procedure is described in detail in Current Protocols in Immunology, above, unit 4.5.

EAE is a T cell mediated autoimmune disease characterized by T cell and mononuclear cell inflammation and subsequent demyelination of axons in the central nervous system. EAE is generally considered to be a relevant animal model for MS in humans. Bolton, Multiple Sclerosis (1995) 1:143. Both acute and relapsing-remitting models have been developed. The compounds of the invention can be tested for T cell stimulatory or inhibitory activity against immune mediated demyelinating disease using the protocol described in Current Protocols in Immunology, above, units 15.1 and 15.2. See also the models for myelin disease in which oligodendrocytes or Schwann cells are grafted into the central nervous system as described in Duncan et al, Molec. Med. Today (1997) 554-561.

Contact hypersensitivity is a simple delayed type hypersensitivity *in vivo* assay of cell mediated immune function. In this procedure, cutaneous exposure to exogenous haptens which gives rise to a delayed type hypersensitivity reaction which is measured and quantitated. Contact sensitivity involves an initial sensitizing phase followed by an elicitation phase. The elicitation phase occurs when the T lymphocytes encounter an antigen to which they have had previous contact. Swelling and inflammation occur, making this an excellent model of human allergic contact dermatitis. A suitable procedure is described in detail in Current Protocols in Immunology, Eds. J E Cologan, AM Kruisbeek, DH Margulies, EM Shevach and Strober, John Wiley & Sons, Inc., 1994, unit 4.2. See also Grabbe and Schwarz, Immun. Today 19(1):37-44 (1998).

An animal model for arthritis is collagen-induced arthritis. This model shares clinical, histological and immunological characteristics of human autoimmune rheumatoid arthritis and is an acceptable model for human autoimmune arthritis. Mouse and rat models are characterized by synovitis, erosion of cartilage and subchondral bone. The PRO polypeptides and compounds of the invention can be tested for activity against autoimmune arthritis using the protocols described in Current Protocols in Immunology, above, units 15.5. See also the model using a monoclonal antibody to CD18 and VLA-4 integrins described in Issekutz et al., Immunology (1996) 88:569.

A model of asthma has been described in which antigen-induced airway hyper-reactivity, pulmonary eosinophilia and inflammation are induced by sensitizing an animal with ovalbumin and then challenging the animal with the same protein delivered by aerosol. Several animal models (guinea pig, rat, non-human primate) show symptoms similar to atopic asthma in humans upon challenge with aerosol antigens. Murine models have many of the features of human asthma. Suitable procedures to test the PRO polypeptides and compounds of the invention for activity and effectiveness in the treatment of asthma are described by Wolyniec et al, Am. J. Respir. Cell Mol. Biol. (1998) 18:777 and the references cited therein.

Additionally, the PRO polypeptides and compounds of the invention can be tested on animal models for psoriasis like diseases. Evidence suggests a T cell pathogenesis for psoriasis. The compounds of the invention can be tested in the scid/scid mouse model described by Schon et al, Nat. Med. (1997) 3:183, in which the mice demonstrate histopathologic skin lesions resembling psoriasis. Another suitable model is the human skin/scid mouse chimera prepared as described by Nickoloff et al, Am. J. Path. (1995) 146:580.

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, e.g. baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (e.g., Van der Putten et al., Proc. Natl. Acad. Sci. USA 82, 6148-615 [1985]); gene targeting in embryonic stem cells (Thompson et al., Cell 56, 313-321 [1989]); electroporation of embryos (Lo, Mol. Cel., Biol. 3, 1803-1814 [1983]); sperm-mediated gene transfer (Lavitrano et al., Cell 57, 717-73 [1989]). For review, see, for example, U.S. Patent No. 4,736,866.

For the purpose of the present invention, transgenic animals include those that carry the transgene only in part of their cells ("mosaic animals"). The transgene can be integrated either as a single transgene, or in concatamers, e.g., head-to-head or head-to-tail tandems. Selective introduction of a transgene into a particular cell type is also possible by following, for example, the technique of Lasko et al., Proc. Natl. Acad. Sci. USA 89, 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as in situ hybridization, Northern blot analysis, PCR, or immunocytochemistry.

The animals may be further examined for signs of immune disease pathology, for example by histological examination to determine infiltration of immune cells into specific tissues. Blocking experiments can also be performed in which the transgenic animals are treated with the compounds of the invention to determine the extent of the T cell proliferation stimulation or inhibition of the compounds. In these experiments, blocking antibodies which bind to the PRO polypeptide of the invention, prepared as described above, are administered to the animal and the effect on immune function is determined.

Alternatively, "knock out" animals can be constructed which have a defective or altered gene encoding a polypeptide identified herein, as a result of homologous recombination between the endogenous gene encoding the polypeptide and altered genomic DNA encoding the same polypeptide introduced into an embryonic cell of the animal. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically. several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, Teratocarcinomas and Embryonic Stem Cells: A Practical Approach, Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

I. Immunoadiuvant Therapy

In one embodiment, the immunostimulating PRO polypeptides and compounds of the invention can be used in immunoadjuvant therapy for the treatment of tumors (cancer). It is now well established that T cells recognize human tumor specific antigens. One group of tumor antigens, encoded by the MAGE, BAGE and GAGE families of genes, are silent in all adult normal tissues, but are expressed in significant amounts in tumors, such as melanomas, lung tumors, head and neck tumors, and bladder carcinomas. DeSmet et al, (1996) Proc. Natl. Acad. Sci. USA, 93:7149. It has been shown that costimulation of T cells induces tumor regression and an antitumor response both *in vitro* and *in vivo*. Melero et al, Nature Medicine (1997) 3:682; Kwon et al, Proc. Natl. Acad. Sci. USA (1997) 94:8099; Lynch et al, Nature Medicine (1997) 3:625; Finn and Lotze, J. Immunol. (1998) 21:114. The stimulatory PRO polypeptides and compounds of the invention can be administered as adjuvants, alone or together with a growth regulating agent, cytotoxic agent or chemotherapeutic agent, to stimulate T cell proliferation/activation and an antitumor response to tumor antigens. The growth regulating, cytotoxic, or chemotherapeutic agent may be administered in conventional amounts using known administration regimes. Immunostimulating activity by the PRO polypeptides and compounds of the invention allows reduced amounts of the growth regulating, cytotoxic, or chemotherapeutic agents thereby potentially lowering the toxicity to the patient.

J. Screening Assays for Drug Candidates

Screening assays for drug candidates are designed to identify compounds that bind to or complex with the polypeptides encoded by the genes identified herein or a biologically active fragment thereof, or otherwise interfere with the interaction of the encoded polypeptides with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies. and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art.

All assays are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide encoded by the gene identified herein or the drug candidate is immobilized on a solid phase, e.g. on a microtiter plate, by covalent or non-covalent attachments. Non-covalent attachment generally is accomplished by coating the solid surface with a solution of the polypeptide and drying. Alternatively, an immobilized antibody, e.g. a monoclonal antibody, specific for the polypeptide to be immobilized can be used to anchor it to a solid surface. The assay is performed by adding the non-immobilized component, which may be labeled by a detectable label, to the immobilized component, e.g. the coated surface containing the anchored component. When the reaction is complete, the non-reacted components are removed, e.g. by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labelled antibody specifically binding the immobilized complex.

If the candidate compound interacts with but does not bind to a particular protein encoded by a gene identified herein, its interaction with that protein can be assayed by methods well known for detecting protein-protein interactions. Such assays include traditional approaches, such as, cross-linking. co-immunoprecipitation, and co-purification through gradients or chromatographic columns. In addition, protein-protein interactions can be monitored by using a yeast-based genetic system described by Fields and co-workers [Fields and Song, Nature (London) 340, 245-246 (1989); Chien et al., Proc. Natl. Acad. Sci. USA 88, 9578-9582 (1991)] as disclosed by Chevray and Nathans [Proc. Natl. Acad. Sci. USA 89, 5789-5793 (1991)]. Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-lacZ reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

In order to find compounds that interfere with the interaction of a gene identified herein and other intra- or extracellular components can be tested, a reaction mixture is usually prepared containing the product of the gene and the intra- or extracellular component under conditions and for a time allowing for the interaction and binding of the two products. To test the ability of a test compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described above. The formation of a complex in the control reaction(s) but not in the reaction mixture containing the test compound indicates that the test compound interferes with the interaction of the test compound and its reaction partner.

K. Compositions and Methods for the Treatment of Immune Related Diseases

The compositions useful in the treatment of immune related diseases include, without limitation, proteins, antibodies, small organic molecules, peptides, phosphopeptides, antisense and ribozyme molecules, triple helix molecules, etc. that inhibit or stimulate immune function, for example, T cell proliferation/activation, lymphokine release, or immune cell infiltration.

For example, antisense RNA and RNA molecules act to directly block the translation of mRNA by hybridizing to targeted mRNA and preventing protein translation. When antisense DNA is used, oligodeoxyribonucleotides derived from the translation initiation site, e.g. between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e.g. Rossi, Current Biology 4, 469-471 (1994), and PCT publication No. WO 97/33551 (published September 18, 1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizeable stretches of purines or pyrimidines on one strand of a duplex. For further details see, e.g. PCT publication No. WO 97/33551, supra.

These molecules can be identified by any or any combination of the screening assays discussed above and/or by any other screening techniques well known for those skilled in the art.

L. Anti-PRO Antibodies

The present invention further provides anti-PRO antibodies. Exemplary antibodies include polyclonal, monoclonal, humanized, bispecific, and heteroconjugate antibodies. Some of the most promising drug candidates according to the present invention are antibodies and antibody fragments which may inhibit (antagonists) or stimulate (agonists) T cell proliferation, eosinophil infiltration, etc.

1. Polyclonal Antibodies

The anti-PRO antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

2. Monoclonal Antibodies

The anti-PRO antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro*.

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT). the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U. S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

3. Human and Humanized Antibodies

The anti-PRO antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321: 522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boemer et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks *et al*., Bio/Technology 10, 779-783 (1992); Lonberg *et al*., Nature 368 856-859 (1994); Morrison, Nature 368, 812-13 (1994); Fishwild *et al*., Nature Biotechnology 14, 845-51 (1996); Neuberger, Nature Biotechnology 14, 826 (1996); Lonberg and Huszar, Intern. Rev. Immunol. 13 65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

4. Bispecific Antibodies

Bispecific antibodies are monoclonal, preferably human or humanized, antibodies that have binding specificities for at least two different antigens. In the present case, one of the binding specificities is for the PRO, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH 1) containing the site necessary for light-chain binding present in at least one of the fusions. DNAs encoding the immunoglobulin heavy-chain fusions and, if desired, the immunoglobulin light chain, are inserted into separate expression vectors, and are cotransfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. $F(ab')_2$ bispecific antibodies). Techniques for generating bispecific antibodies from antibody fragments have been described in the literature. For example, bispecific antibodies can be prepared can be prepared using chemical linkage. Brennan *et al*., Science 229:81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby *et al*., J. Exp. Med. 175:217-225 (1992) describe the production of a fully humanized bispecific antibody $F(ab')_2$ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny *et al*., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger *et al*., Proc. Natl. Acad. Sci. USA

90:6444-6448 (1993) has provided an alternative mechanism for making bispecific antibody fragments. The fragments comprise a heavy-chain variable domain ($V_H$) connected to a light-chain variable domain ($V_L$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See, Gruber *et al*., J. Immunol. 152:5368 (1994). Antibodies with more than two valencies are contemplated. For example, trispecific antibodies can be prepared. Tutt *et al*., J. Immunol. 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

5. Heteroconjugate Antibodies

Heteroconjugate antibodies are also within the scope of the present invention. Heteroconjugate antibodies are composed of two covalently joined antibodies. Such antibodies have. for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

6. Effector Function Engineering

It may be desirable to modify the antibody of the invention with respect to effector function, so as to enhance, e.g., the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron *et al*., J. Exp Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff *et al*. Cancer Research, 53: 2560-2565 (1993). Alternatively, an antibody can be engineered that has dual Fc regions and may thereby have enhanced complement lysis and ADCC capabilities. See Stevenson *et al*., Anti-Cancer Drug Design, 3: 219-230 (1989).

7. Immunoconjugates

The invention also pertains to immunoconjugates comprising an antibody conjugated to a cytotoxic agent such as a chemotherapeutic agent, toxin (e.g., an enzymatically active toxin of bacterial, fungal, plant, or animal origin, or fragments thereof), or a radioactive isotope (*i.e*., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*). ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin. enomycin, and the tricothecenes. A variety of radionuclides are available for the production of radioconjugated antibodies. Examples include $^{212}Bi$, $^{131}I$, $^{131}In$, $^{90}Y$, and $^{186}Re$. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde). bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta *et al.,* Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See W094/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) that is conjugated to a cytotoxic agent (*e.g.*, a radionucleotide).

8. Immunoliposomes

The antibodies disclosed herein may also be formulated as immunoliposomes. Liposomes containing the antibody are prepared by methods known in the art, such as described in Epstein *et al*., Proc. Natl. Acad. Sci. USA, 82: 3688 (1985); Hwang *et al*., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin *et al* ., J. Biol. Chem., 257: 286-288 (1982) via a disulfide-interchange reaction. A chemotherapeutic agent (such as Doxorubicin) is optionally contained within the liposome. See Gabizon *et al*., J. National Cancer Inst., 81(19): 1484 (1989).

M. Pharmaceutical Compositions

The active molecules of the invention, PRO polypeptides and antibodies, as well as other molecules identified by the screening assays disclosed above, can be administered for the treatment of immune related diseases, in the form of pharmaceutical compositions.

Therapeutic formulations of the active molecule, preferably a PRO polypeptide or anti-PRO antibody of the invention, are prepared for storage by mixing the active molecule having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

Compounds identified by the screening assays of the present invention can be formulated in an analogous manner, using standard techniques well known in the art.

Lipofections or liposomes can also be used to deliver the polypeptide, antibody, or an antibody fragment, into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (see, e.g. Marasco et al., Proc. Natl. Acad. Sci. USA 90, 7889-7893 [1993]).

The formulation herein may also contain more than one active polypeptide or compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active molecules may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and g ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

N. Methods of Treatment

It is contemplated that the PRO polypeptides, antibodies and other active compounds of the present invention may be used to treat various immune related diseases and conditions, such as T cell mediated diseases, including those characterized by infiltration of inflammatory cells into a tissue, stimulation of T-cell proliferation, inhibition of T-cell proliferation, increased or decreased vascular permeability or the inhibition thereof.

Exemplary conditions or disorders to be treated with the PRO polypeptides, antibodies and other compounds of the invention, include, but are not limited to systemic lupus erythematosis, rheumatoid arthritis, juvenile chronic arthritis, osteoarthritis, spondyloarthropathies, systemic sclerosis (scleroderma), idiopathic inflammatory myopathies (dermatomyositis, polymyositis), Sjogren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, diopathic demyelinating polyneuropathy or Guillain-Barré syndrome, and chronic inflammatory lemyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

In systemic lupus erythematosus, the central mediator of disease is the production of auto-reactive antibodies to self proteins/tissues and the subsequent generation of immune-mediated inflammation. Though T lymphocytes have not been shown to be directly involved in tissue damage, T lymphocytes are required for the development of auto-reactive antibodies. The genesis of the disease is thus T lymphocyte dependent. Multiple organs and systems are affected clinically including kidney, lung, musculoskeletal system, mucocutaneous, eye, central nervous system, cardiovascular system, gastrointestinal tract, bone marrow and blood.

Rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG, with the resultant formation of immune complexes that attain high levels in joint fluid and blood. These complexes in the joint may induce the marked infiltrate of lymphocytes and monocytes into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, intestitial pneumonitis with pulmonary fibrosis, keratoconjunctivitis sicca, and rhematoid nodules.

Juvenile chronic arthritis is a chronic idiopathic inflammatory disease which begins often at less than 16 years of age. Its phenotype has some similarities to RA; some patients which are rhematoid factor positive are classified as juvenile rheumatoid arthritis. The disease is sub-classified into three major categories: pauciarticular, polyarticular, and systemic. The arthritis can be severe and is typically destructive and leads to joint ankylosis and retarded growth. Other manifestations can include chronic anterior uveitis and systemic amyloidosis.

Spondyloarthropathies are a group of disorders with some common clinical features and the common association with the expression of HLA-B27 gene product. The disorders include: ankylosing sponylitis, Reiter's syndrome (reactive arthritis), arthritis associated with inflammatory bowel disease, spondylitis associated with psoriasis, juvenile onset spondyloarthropathy and undifferentiated spondyloarthropathy. Distinguishing features include sacroileitis with or without spondylitis; inflammatory asymmetric arthritis; association with HLA-B27 (a serologically defined allele of the HLA-B locus of class I MHC); ocular inflammation, and absence of autoantibodies associated with other rheumatoid disease. The cell most implicated as key to induction of the disease is the $CD8^+$ T lymphocyte, a cell which targets antigen presented by class I MHC molecules. $CD8^+$ T cells may react against the class I MHC allele HLA-B27 as if it were a foreign peptide expressed by MHC class I molecules. It has been hypothesized that an epitope of HLA-B27 may mimic a bacterial or other microbial antigenic epitope and thus induce a $CD8^+$ T cells response.

Systemic sclerosis (scleroderma) has an unknown etiology. A hallmark of the disease is induration of the skin; likely this is induced by an active inflammatory process. Scleroderma can be localized or systemic; vascular lesions are common and endothelial cell injury in the microvasculature is an early and important event in the development of systemic sclerosis; the vascular injury may be immune mediated. An immunologic basis is implied by the presence of mononuclear cell infiltrates in the cutaneous lesions and the presence of anti-nuclear antibodies in many patients. ICAM-1 is often upregulated on the cell surface of fibroblasts in skin lesions suggesting that T cell interaction with these cells may have a role in the pathogenesis of the disease. Other organs involved include: the gastrointestinal tract: smooth muscle atrophy and fibrosis resulting in abnormal peristalsis/motility; kidney: concentric subendothelial intimal proliferation affecting small arcuate and interlobular arteries with resultant reduced renal cortical blood flow, results in proteinuria, azotemia and hypertension; skeletal muscle: atrophy, interstitial fibrosis; inflammation; lung: interstitial pneumonitis and interstitial fibrosis; and heart: contraction band necrosis, scarring/fibrosis.

Idiopathic inflammatory myopathies including dermatomyositis, polymyositis and others are disorders of chronic muscle inflammation of unknown etiology resulting in muscle weakness. Muscle injury/inflammation is often symmetric and progressive. Autoantibodies are associated with most forms. These myositis-specific autoantibodies are directed against and inhibit the function of components, proteins and RNA's, involved in protein synthesis.

Sjogren's syndrome is due to immune-mediated inflammation and subsequent functional destruction of the tear glands and salivary glands. The disease can be associated with or accompanied by inflammatory connective tissue diseases. The disease is associated with autoantibody production against Ro and La antigens, both of which are small RNA-protein complexes. Lesions result in keratoconjunctivitis sicca, xerostomia, with other manifestations or associations including bilary cirrhosis, peripheral or sensory neuropathy, and palpable purpura.

Systemic vasculitis are diseases in which the primary lesion is inflammation and subsequent damage to blood vessels which results in ischemia/necrosis/degeneration to tissues supplied by the affected vessels and eventual end-organ dysfunction in some cases. Vasculitides can also occur as a secondary lesion or sequelae to other immune-inflammatory mediated diseases such as rheumatoid arthritis, systemic sclerosis, etc., particularly in diseases also associated with the formation of immune complexes. Diseases in the primary systemic vasculitis group include: systemic necrotizing vasculitis: polyarteritis nodosa, allergic angiitis and granulomatosis, polyangiitis; Wegener's granulomatosis; lymphomatoid granulomatosis; and giant cell arteritis. Miscellaneous vasculitides include: mucocutaneous lymph node syndrome (MLNS or Kawasaki's disease), isolated CNS vasculitis, Behet's disease, thromboangiitis obliterans (Buerger's disease) and cutaneous necrotizing venulitis. The pathogenic mechanism of most of the types of vasculitis listed is believed to be primarily due to the deposition of immunoglobulin complexes in the vessel wall and subsequent induction of an inflammatory response either via ADCC, complement activation, or both.

Sarcoidosis is a condition of unknown etiology which is characterized by the presence of epithelioid granulomas in nearly any tissue in the body; involvement of the lung is most common. The pathogenesis involves the persistence of activated macrophages and lymphoid cells at sites of the disease with subsequent chronic sequelae resultant from the release of locally and systemically active products released by these cell types.

Autoimmune hemolytic anemia including autoimmune hemolytic anemia, immune pancytopenia, and paroxysmal noctural hemoglobinuria is a result of production of antibodies that react with antigens expressed on the surface of red blood cells (and in some cases other blood cells including platelets as well) and is a reflection of the removal of those antibody coated cells via complement mediated lysis and/or ADCC/Fc-receptor-mediated mechanisms.

In autoimmune thrombocytopenia including thrombocytopenic purpura, and immune-mediated thrombocytopenia in other clinical settings, platelet destruction/removal occurs as a result of either antibody or complement attaching to platelets and subsequent removal by complement lysis, ADCC or FC-receptor mediated mechanisms.

Thyroiditis including Grave's disease, Hashimoto's thyroiditis, juvenile lymphocytic thyroiditis, and atrophic thyroiditis, are the result of an autoimmune response against thyroid antigens with production of antibodies that react with proteins present in and often specific for the thyroid gland. Experimental models exist including spontaneous models: rats (BUF and BB rats) and chickens (obese chicken strain); inducible models: immunization of animals with either thyroglobulin, thyroid microsomal antigen (thyroid peroxidase).

Type I diabetes mellitus or insulin-dependent diabetes is the autoimmune destruction of pancreatic islet β-cells; this destruction is mediated by auto-antibodies and auto-reactive T cells. Antibodies to insulin or the insulin receptor can also produce the phenotype of insulin-non-responsiveness.

Immune mediated renal diseases, including glomerulonephritis and tubulointerstitial nephritis, are the result of antibody or T lymphocyte mediated injury to renal tissue either directly as a result of the production of autoreactive antibodies or T cells against renal antigens or indirectly as a result of the deposition of antibodies and/or immune complexes in the kidney that are reactive against other, non-renal antigens. Thus other immune-mediated diseases that result in the formation of immune-complexes can also induce immune mediated renal disease as an indirect sequelae. Both direct and indirect immune mechanisms result in inflammatory response that produces/induces lesion development in renal tissues with resultant organ function impairment and in some cases progression to renal failure. Both humoral and cellular immune mechanisms can be involved in the pathogenesis of lesions.

Demyelinating diseases of the central and peripheral nervous systems, including Multiple Sclerosis; idiopathic demyelinating polyneuropathy or Guillain-Barr syndrome; and Chronic Inflammatory Demyelinating Polyneuropathy, are believed to have an autoimmune basis and result in nerve demyelination as a result of damage caused to oligodendrocytes or to myelin directly. In MS there is evidence to suggest that disease induction and progression is dependent on T lymphocytes. Multiple Sclerosis is a demyelinating disease that is T lymphocyte-dependent and has either a relapsing-remitting course or a chronic progressive course. The etiology is unknown; however, viral infections, genetic predisposition, environment, and autoimmunity all contribute. Lesions contain infiltrates of predominantly T lymphocyte mediated, microglial cells and infiltrating macrophages; $CD4^{+}$T lymphocytes are the predominant cell type at lesions. The mechanism of oligodendrocyte cell death and subsequent demyelination is not known but is likely T lymphocyte driven.

Inflammatory and Fibrotic Lung Disease, including Eosinophilic Pneumonias; Idiopathic Pulmonary Fibrosis, and Hypersensitivity Pneumonitis may involve a disregulated immune-inflammatory response. Inhibition of that response would be of therapeutic benefit.

Autoimmune or Immune-mediated Skin Disease including Bullous Skin Diseases, Erythema Multiforme, and Contact Dermatitis are mediated by auto-antibodies, the genesis of which is T lymphocyte-dependent.

Psoriasis is a T lymphocyte-mediated inflammatory disease. Lesions contain infiltrates of T lymphocytes, macrophages and antigen processing cells, and some neutrophils.

Allergic diseases, including asthma; allergic rhinitis; atopic dermatitis; food hypersensitivity; and urticaria are T lymphocyte dependent. These diseases are predominantly mediated by T lymphocyte induced inflammation, IgE mediated-inflammation or a combination of both.

Transplantation associated diseases, including Graft rejection and Graft-Versus-Host-Disease (GVHD) are T lymphocyte-dependent; inhibition of T lymphocyte function is ameliorative.

Other diseases in which intervention of the immune and/or inflammatory response have benefit are infectious disease including but not limited to viral infection (including but not limited to AIDS, hepatitis A, B, C, D, Eand herpes) bacterial infection, fungal infections, and protozoal and parasitic infections (molecules (or derivatives/agonists) which stimulate the MLR can be utilized therapeutically to enhance the immune response to infectious agents), diseases of immunodeficiency (molecules/derivatives/agonists) which stimulate the MLR can be utilized therapeutically to enhance the immune response for conditions of inherited, acquired, infectious induced (as in HIV infection), or iatrogenic (i.e. as from chemotherapy) immunodeficiency), and neoplasia.

It has been demonstrated that some human cancer patients develop an antibody and/or T lymphocyte response to antigens on neoplastic cells. It has also been shown in animal models of neoplasia that enhancement of the immune response can result in rejection or regression of that particular neoplasm. Molecules that enhance the T lymphocyte response in the MLR have utility *in vivo* in enhancing the immune response against neoplasia. Molecules which enhance the T lymphocyte proliferative response in the MLR (or small molecule agonists or antibodies that affected the same receptor in an agonistic fashion) can be used therapeutically to treat cancer. Molecules that inhibit the lymphocyte response in the MLR also function in vivo during neoplasia to suppress the immune response to a neoplasm; such molecules can either be expressed by the neoplastic cells themselves or their expression can be induced by the neoplasm in other cells. Antagonism of such inhibitory molecules (either with antibody, small molecule antagonists or other measn) enhances immune-mediated tumor rejection.

Additionally, inhibition of molecules with proinflammatory properties may have therapeutic benefit in reperfusion injury; stroke; myocardial infarction; atherosclerosis; acute lung injury; hemorrhagic shock; burn; sepsis/septic shock; acute tubular necrosis; endometriosis; degenerative joint disease and pancreatis.

The PRO polypeptides and compounds of the present invention, e.g. PRO polypeptides or antibodies, are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation (intranasal, intrapulmonary) routes. Intravenous or inhaled administration of polypeptides and antibodies is preferred.

In immunoadjuvant therapy, other therapeutic regimens, such administration of an anti-cancer agent, may be combined with the administration of the proteins, antibodies or compounds of the instant invention. For example, the patient to be treated with a the immunoadjuvant of the invention may also receive an anti-cancer agent (chemotherapeutic agent) or radiation therapy. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the immunoadjuvant or may be given simultaneously therewith. Additionally, an anti-oestrogen compound such as tamoxifen or an anti-progesterone such as onapristone (see, EP 616812) may be given in dosages known for such molecules.

It may be desirable to also administer antibodies against other immune disease associated or tumor associated antigens, such as antibodies which bind to CD20, CD11a, CD18, ErbB2, EGFR, ErbB3, ErbB4, or vascular endothelial factor (VEGF). Alternatively, or in addition, two or more antibodies binding the same or two or more different antigens disclosed herein may be coadministered to the patient. Sometimes, it may be beneficial to also administer one or more cytokines to the patient. In one embodiment, the polypeptides of the invention are coadministered with a growth inhibitory agent. For example, the growth inhibitory agent may be administered first, followed by a PRO polypeptide of the invention. However, simultaneous administration or administration first is also contemplated. Suitable dosages for the growth inhibitory agent are those presently used and may be lowered due to the combined action (synergy) of the growth inhibitory agent and the PRO polypeptide of the invention.

For the treatment or reduction in the severity of immune related disease, the appropriate dosage of an a PRO polypeptide or compound of the invention will depend on the type of disease to be treated, as defined above, the severity and course of the disease, whether the agent is administered for preventive or therapeutic purposes, previous therapy, the patient's clinical history and response to the compound, and the discretion of the attending physician. The compound is suitably administered to the patient at one time or over a series of treatments.

For example, depending on the type and severity of the disease, about 1 µg/kg to 15 mg/kg (e.g. 0.1-20mg/kg) of polypeptide or antibody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

O. Articles of Manufacture

In another embodiment of the invention, an article of manufacture containing materials useful for the diagnosis or treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually a PRO polypeptide or an antibody of the invention. The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

P. Diagnosis and Prognosis of Immune Related Disease

Cell surface proteins, such as proteins which are overexpressed in certain immune related diseases, are excellent targets for drug candidates or disease treatment. The same proteins along with secreted proteins encoded by the genes amplified in immune related disease states find additional use in the diagnosis and prognosis of these diseases. For example, antibodies directed against the protein products of genes amplified in multiple sclerosis, rheumatoid arthritis, or another immune related disease, can be used as diagnostics or prognostics.

For example, antibodies, including antibody fragments, can be used to qualitatively or quantitatively detect the expression of proteins encoded by amplified or overexpressed genes ("marker gene products"). The antibody preferably is equipped with a detectable, e.g. fluorescent label, and binding can be monitored by light microscopy, flow cytometry, fluorimetry, or other techniques known in the art. These techniques are particularly suitable, if the overexpressed gene encodes a cell surface protein Such binding assays are performed essentially as decribed above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for in situ detection.

The following examples are offered for illustrative purposes only, and are not intended to limit the scope of the present invention in any way. All patent and literature references cited in the present specification are hereby incorporated by reference in their entirety.

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA. Unless otherwise noted, the present invention uses standard procedures of recombinant DNA technology, such as those described hereinabove and in the following textbooks: Sambrook et al., Molecular Cloning: A Laboratory Manual, Cold Spring Harbor Press N.Y., 1989; Ausubel et al., Current Protocols in Molecular Biology, Green Publishing Associates and Wiley Interscience, N.Y., 1989; Innis et al., PCR Protocols: A Guide to Methods and Applications, Academic Press, Inc., N.Y., 1990; Harlow et al., Antibodies: A Laboratory Manual, Cold Spring Harbor Press, Cold Spring Harbor, 1988; Gait, Oligonucleotide Synthesis, IRL Press, Oxford, 1984; Freshney, Animal Cell Culture, 1987; Coligan et al., Current Protocols in Immunology, 1991.

## EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1: Isolation of cDNA clones Encoding Human PRO179, PRO175, PRO182, PRO366, PRO240, PRO256, PRO306, PRO364, PRO356, PRO826, PRO1068, PRO1343 or PRO1375(UNQ153, UNQ149, UNQ156, UNQ321, UNQ214, UN0223, UNQ269, UNQ319, UNQ313, UNQ467, UNQ525, UNQ698 or UNQ712)

### A. Isolation of cDNA clones Encoding Human PRO179 (UNQ153)

#### 1. Preparation of oligo dT primed cDNA library

mRNA was isolated from a human tissue of interest using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites.

#### 2. Preparation of random primed cDNA library

A secondary cDNA library was generated in order to preferentially represent the 5' ends of the primary cDNA clones. Sp6 RNA was generated from the primary library (described above), and this RNA was used to generate a random primed cDNA library in the vector pSST-AMY.0 using reagents and protocols from Life Technologies (Super Script Plasmid System, referenced above). In this procedure the double stranded cDNA was sized to 500-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with amylase sequence will lead to the secretion of amylase from appropriately transfected yeast colonies.

#### 3. Transformation and Detection

DNA from the library described in paragraph 2 above was chilled on ice to which was added electrocompetent DH10B bacteria (Life Technologies, 20 ml). The bacteria and vector mixture was then electroporated as recommended by the manufacturer. Subsequently, SOC media (Life Technologies, 1 ml) was added and the mixture was incubated at 37°C for 30 minutes. The transformants were then plated onto 20 standard 150 mm LB plates containing ampicillin and incubated for 16 hours (37°C). Positive colonies were scraped off the plates and the DNA was isolated from the bacterial pellet using standard protocols, e.g. CsCl-gradient. The purified DNA was then carried on to the yeast protocols below.

The yeast methods were divided into three categories: (1) Transformation of yeast with the plasmid/cDNA combined vector; (2) Detection and isolation of yeast clones secreting amylase; and (3) PCR amplification of the insert directly from the yeast colony and purification of the DNA for sequencing and further analysis.

The yeast strain used was HD56-5A (ATCC-90785). This strain has the following genotype: MAT alpha, ura3-52, leu2-3, leu2-112, his3-11, his3-15, $MAL^+$, $SUC^+$, $GAL^+$. Preferably, yeast mutants can be employed that have deficient post-translational pathways. Such mutants may have translocation deficient alleles in *sec*71, *sec*72, *sec*62, with truncated *sec*71 being most preferred. Alternatively, antagonists (including antisense nucleotides and/or ligands) which interfere with the normal operation of these genes, other proteins implicated in this post translation pathway (e. g., SEC61p, SEC72p, SEC62p, SEC63p, TDJ 1p or SSA1p-4p) or the complex formation of these proteins may also be preferably employed in combination with the amylase-expressing yeast.

Transformation was performed based on the protocol outlined by Gietz et al., Nucl. Acid. Res., 20: 1425 (1992). Transformed cells were then inoculated from agar into YEPD complex media broth (100 ml) and grown overnight at 30°C. The YEPD broth was prepared as described in Kaiser et al., Methods in Yeast Genetics, Cold Spring Harbor Press, Cold Spring Harbor, NY, p. 207 (1994). The overnight culture was then diluted to about 2 x $10^6$ cells/ml (approx. $OD_{600}$=0.1) into fresh YEPD broth (500 ml) and regrown to 1 x $10^7$ cells/ml (approx. $OD_{600}$=0.4-0.5).

The cells were then harvested and prepared for transformation by transfer into GS3 rotor bottles in a Sorval GS3 rotor at 5,000 rpm for 5 minutes, the supernatant discarded, and then resuspended into sterile water, and centrifuged again in 50 ml falcon tubes at 3,500 rpm in a Beckman GS-6KR centrifuge. The supernatant was discarded and the cells were subsequently washed with LiAc/TE (10 ml, 10 mM Tris-HCl, 1 mM EDTA pH 7.5, 100 mM $Li_2OOCCH_3$), and resuspended into LiAc/TE (2.5 ml).

Transformation took place by mixing the prepared cells (100 µl) with freshly denatured single stranded salmon testes DNA (Lofstrand Labs, Gaithersburg, MD) and transforming DNA (1 µg, vol. < 10 µl) in microfuge tubes. The mixture was mixed briefly by vortexing, then 40% PEG/TE (600 µl, 40% polyethylene glycol-4000, 10 mM Tris-HCl. 1 mM EDTA, 100 mM $Li_2OOCCH_3$, pH 7.5) was added. This mixture was gently mixed and incubated at 30°C while agitating for 30 minutes. The cells were then heat shocked at 42°C for 15 minutes, and the reaction vessel centrifuged in a microfuge at 12,000 rpm for 5-10 seconds, decanted and resuspended into TE (500 µl, 10 mM Tris-HCl, 1 mM EDTA pH 7.5) followed by recentrifugation. The cells were then diluted into TE (1 ml) and aliquots (200 µl) were spread onto the selective media previously prepared in 150 mm growth plates (VWR).

Alternatively, instead of multiple small reactions, the transformation was performed using a single, large scale reaction, wherein reagent amounts were scaled up accordingly.

The selective media used was a synthetic complete dextrose agar lacking uracil (SCD-Ura) prepared as described in Kaiser et al., Methods in Yeast Genetics, Cold Spring Harbor Press, Cold Spring Harbor, NY, p. 208-210 (1994). Transformants were grown at 30°C for 2-3 days.

The detection of colonies secreting amylase was performed by including red starch in the selective growth media. Starch was coupled to the red dye (Reactive Red-120, Sigma) as per the procedure described by Biely et al., Anal. Biochem., 172:176-179 (1988). The coupled starch was incorporated into the SCD-Ura agar plates at a final concentration of 0.15 % (w/v), and was buffered with potassium phosphate to a pH of 7.0 (50-100 mM final concentration).

The positive colonies were picked and streaked across fresh selective media (onto 150 mm plates) in order to obtain well isolated and identifiable single colonies. Well isolated single colonies positive for amylase secretion were detected by direct incorporation of red starch into buffered SCD-Ura agar. Positive colonies were determined by their ability to break down starch resulting in a clear halo around the positive colony visualized directly.

4. Isolation of DNA by PCR Amplification

When a positive colony was isolated, a portion of it was picked by a toothpick and diluted into sterile water (30 µl) in a 96 well plate. At this time, the positive colonies were either frozen and stored for subsequent analysis or immediately amplified. An aliquot of cells (5 µl) was used as a template for the PCR reaction in a 25 µl volume containing: 0.5 µl Klentaq (Clontech, Palo Alto, CA); 4.0 µl 10 mM dNTP's (Perkin Elmer-Cetus); 2.5 µl Kentaq buffer (Clontech); 0.25 µl forward oligo 1; 0.25 µl reverse oligo 2; 12.5 µl distilled water. The sequence of the forward oligonucleotide 1 was:

5'-TGTAAAACGACGGCCAGTTAAATAGACCTGCAATTATTAATCT-3' (SEQ ID NO:27)

The sequence of reverse oligonucleotide 2 was:

5'-CAGGAAACAGCTATGACCACCTGCACACCTGCAAATCCATT-3' (SEQ ID NO:28)

PCR was then performed as follows:

| | | | | |
|---|---|---|---|---|
| a. | | Denature | 92°C, | 5 minutes |
| b. | 3 cycles of | Denature | 92°C, | 30 seconds |
| | | Anneal | 59°C, | 30 seconds |
| | | Extend | 72°C, | 60 seconds |
| c. | 3 cycles of | Denature | 92°C, | 30 seconds |
| | | Anneal | 57°C, | 30 seconds |
| | | Extend | 72°C, | 60 seconds |
| d. | 25 cycles of | Denature | 92°C, | 30 seconds |
| | | Anneal | 55°C, | 30 seconds |
| | | Extend | 72°C, | 60 seconds |
| e. | | Hold | 4°C | |

The underlined regions of the oligonucleotides annealed to the ADH promoter region and the amylase region, respectively, and amplified a 307 bp region from vector pSST-AMY.0 when no insert was present. Typically, the first 18 nucleotides of the 5' end of these oligonucleotides contained annealing sites for the sequencing primers. Thus, the total product of the PCR reaction from an empty vector was 343 bp. However, signal sequence-fused cDNA resulted in considerably longer nucleotide sequences.

Following the PCR, an aliquot of the reaction (5 µl) was examined by agarose gel electrophoresis in a 1% agarose gel using a Tris-Borate-EDTA (TBE) buffering system as described by Sambrook et al., supra. Clones resulting in a single strong PCR product larger than 400 bp were further analyzed by DNA sequencing after purification with a 96 Qiaquick PCR clean-up column (Qiagen Inc., Chatsworth, CA).

A cDNA sequence isolated in the above screen was found, by BLAST and FastA sequence alignment, to have sequence homology to a nucleotide sequence encoding various angiopoietin proteins. This cDNA sequence is herein designated DNA 10028 and/or DNA25250. Based on the sequence homology, probes were generated from the sequence of the DNA 10028 molecule and used to screen a human fetal liver (LIB6) library prepared as described in paragraph 1 above. The cloning vector was pRK5B (pRK5B is a precursor of pRK5D that does not contain the SfiI site; see, Holmes et al., Science, 253:1278-1280 (1991)), and the cDNA size cut was less than 2800 bp.

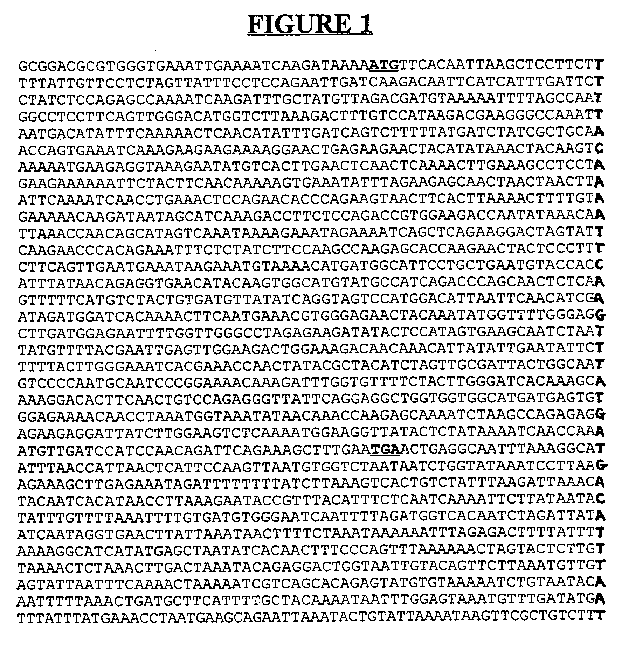

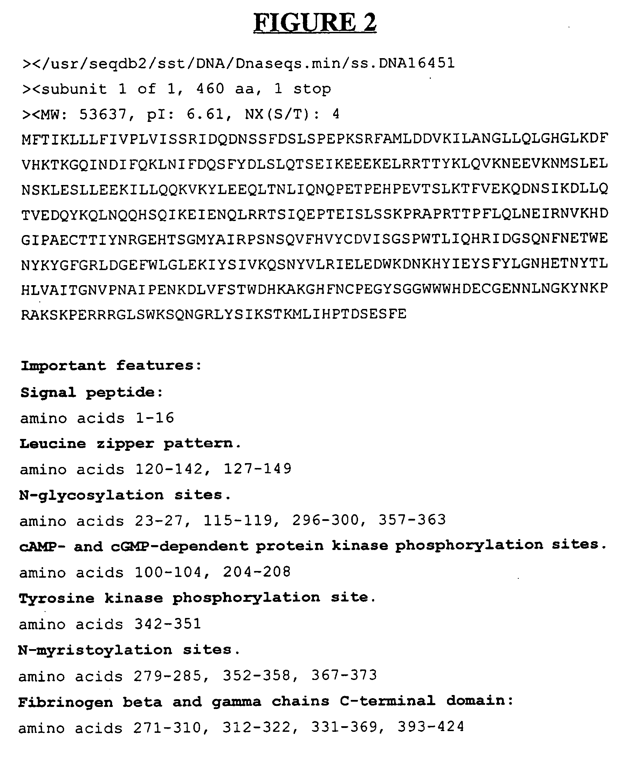

A full length clone was identified that contained a single open reading frame with an apparent translational initiation site at nucleotide positions 37-39 and ending at the stop codon found at nucleotide positions 1417-1419 (Figure 1; SEQ ID NO:1). The predicted polypeptide precursor is 460 amino acids long, has a calculated molecular weight of approximately 53,637 daltons and an estimated pI of approximately 6.61. Analysis of the full-length PRO179 sequence shown in Figure 2 (SEQ ID NO:2) evidences the presence of the following: a signal peptide from about amino acid 1 to about amino acid 16, leucine zipper pattern sequences from about amino acid 120 to about amino acid 141 and from about amino acid 127 to about amino acid 148, potential N-glycosylation sites from about amino acid 23 to about amino acid 26, from about amino acid 115 to about amino acid 118, from about amino acid 296 to about amino acid 299 and from about amino acid 357 to about amino acid 360 and fibrinogen beta and gamma chain C-terminal domain sequence homologies from about amino acid 271 to about amino acid 309, from about amino acid 312 to about amino acid 321, from about amino acid 331 to about amino acid 368 and from about amino acid 393 to about amino acid 423. Clone DNA16451-1388 has been deposited with ATCC on April 14, 1998 and is assigned ATCC deposit no. 209776.

Analysis of the amino acid sequence of the full-length PRO 179 polypeptide suggests that it possesses significant sequence similarity to the angiopoietin family of proteins, thereby indicating that PRO179 may be a novel angiopoietin family member. More specifically, an analysis of the Dayhoff database (version 35.45 SwissProt 35) evidenced significant homology between the PRO179 amino acid sequence and the following Dayhoffsequences, AF004326_1, P_R94605, HSU83508_1, P_R94603, P_R94317, AF025818_1, HSY16132_1, P_R65760, I37391 and HUMRSC192_1.

B. Isolation of Human cDNA encoding PRO175 (UNQ149)

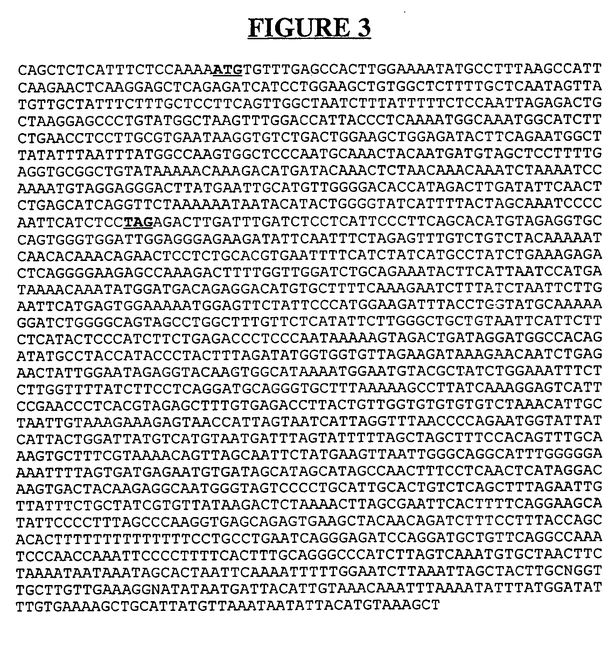

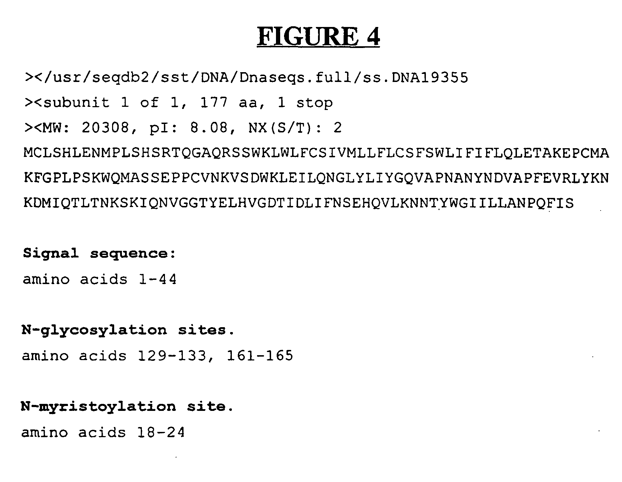

A yeast screen was employed to provide the DNA19355-1150 molecule. An identified cDNA clone was sequenced in entirety. A nucleotide sequence of DNA19355-1150 is shown in Figure 3 (SEQ ID NO:3). Clone DNA 19355-1150 contains a single open reading frame with an apparent translational initiation site at nucleotide positions 21-23 (Figure 3). The predicted polypeptide precursor is 177 amino acids long and has a calculated molecular weight of approximately 20,308 daltons. Hydropathy analysis suggests a type II transmembrane protein typology, with a putative cytoplasmic region (amino acids 1-25); transmembrane region (amino acids 26-51); and extracellular region (amino acids 52-177). Two potential N-linked glycosylation sites have been identified at position 129 (Asn) and position 161 (Asn) of the sequence shown in Figure 4 (SEQ ID NO:4). Clone DNA 19355-1150 has been deposited with ATCC and is assigned ATCC deposit no. 209466. DNA19355 polypeptide is obtained or obtainable by expressing the molecule encoded by the cDNA insert of the deposited ATCC deposit no. 209466 vector. Digestion of the vector with XbaI and NotI restriction enzymes will yield a 1411 bp fragment and 668 bp fragment.

Based on a BLAST and FastA sequence alignment analysis (using the ALIGN computer program) of extracellular sequence, DNA19355-1150 shows amino acid sequence identity to several members of the TNF cytokine family, and particularly, to human Apo-2L (19.8%), Fas/Apo1-ligand (19.0%), TNF-alpha (20.6 % ) and Lymphotoxin (17.5%).

C. Isolation of cDNA clones Encoding Human PRO182 (UNQ156; DNA27865-1091)

A cDNA library was constructed from human uterus mRNA obtained from Clontech Laboratories, Inc. Palo Alto, CA USA, catalog no. 6537-1.

The following protocol is described in "Instruction Manual: SUPERSCRIPT® Lamda System for cDNA Synthesis and 1 cloning," cat. No. 19643-014, Life Technologies, Gaithersburg, MD, USA which is herein incorporated by reference. Unless otherwise noted, all reagents were also obtained from Life Technologies. The overall procedure can be summarized into the following steps: (1) First strand synthesis; (2) Second strand synthesis; (3) Adaptor addition; (4) Enzymatic digestion; (5) Gel isolation of cDNA; (6) Ligation into vector; and (7) Transformation.

First strand synthesis:

NotI primer-adapter (Life Tech., 2 µl, 0.5 g/µl) was added to a sterile 1.5 ml microcentrifuge tube to which was added poly $A^+$ mRNA (7 1, 5µg). The reaction tube was heated to 70°C for 5 minutes or time sufficient to denature the secondary structure of the mRNA. The reaction was then chilled on ice and 5X First strand buffer (Life Tech., 4 µl), 0.1 M DTT (2µl) and 10 mM dNTP Mix (Life Tech., 1 µl) were added and then heated to 37°C for 2 minutes to equilibrate the temperature. SUPERSCRIPT II® reverse transcriptase (Life Tech., 5 µl) was then added, the reaction tube mixed well and incubated at 37°C for 1 hour, and terminated by placement on ice. The final concentration of the reactants was the following: 50 mM Tris-HCl (pH 8.3); 75 mM KCl; 3 mM $MgCl_2$; 10 mM DTT; 500 mM each dATP, dCTP, dGTP and dTTP; 50 mg/ml NotI primer-adapter; 5 mg (250 mg/ml) mRNA; 50,000 U/ml SUPERSCRIPT II® reverse transcriptase.

Second strand synthesis:

While on ice, the following reagents were added to the reaction tube from the first strand synthesis, the reaction well mixed and allowed to react at 16°C for 2 hours, taking care not to allow the temperature to go above 16°C: distilled water (93 ml); 5X Second strand buffer (30 ml); dNTP mix (3 ml); 10 U/ml E. Coli DNA ligase (1 ml); 10 U/ml E. coli DNA polymerase I (4 ml); 2 U/ml E. coli RNase H (1 ml). 10 U T4 DNA Polymerase (2 ml) was added and the reaction continued to incubate at 16°C for another 5 minutes. The final concentration of the reaction was the following: 25 mM Tris-HCl (pH 7.5); 100 mM KCl; 5 mM $MgCl_2$; 10 mM $(NH_4)_2SO_4$; 0.15 mM β-NAD+; 250 mM each dATP, dCTP, dGTP, dTTP; 1.2 mM DTT; 65 U/ml DNA ligase; 250 U/ml DNA polymerase I; 13 U/ml RNase H. The reaction was halted by placement on ice and by addition of 0.5 M EDTA (10 ml), then extracted through phenol:chloroform: isoamyl alcohol (25:24:1, 150 ml). The aqueous phase was removed, collected and diluted into 5M NaCl (15 ml) and absolute ethanol (-20°C, 400 ml) and centrifuged for 2 minutes at 14,000 x g. The supernatant was carefully removed from the resulting DNA pellet, the pellet resuspended in 70% ethanol (0.5 ml) and centrifuged again for 2 minutes at 14,000 x g. The supernatant was again removed and the pellet dried in a SPEEDVAC™ drier.

Adapter addition:

The following reagents were added to the cDNA pellet from the second strand synthesis above, and the reaction was gently mixed and incubated at 16°C for 16 hours: distilled water (25 ml); 5X T4 DNA ligase buffer (10 ml); SalI adapters (10 ml); T4 DNA ligase (5 ml). The final composition of the reaction was the following: 50 mM Tris-HCl (pH 7.6); 10 mM $MgCl_2$; 1 mM ATP; 5% (w/v) PEG 8000; 1 mM DTT; 200 mg/ml Sal I adapters; 100 U/ml T4 DNA ligase. The reaction was extracted through phenol:chloroform:isoamyl alcohol (25:24:1, 50 ml), the aqueous phase removed, collected and diluted into 5M NaCl (8 ml) and absolute ethanol (-20°C, 250 ml). This was then centrifuged for 20 minutes at 14,000 x g, the supernatant removed and the pellet was resuspended in 0.5 ml 70% ethanol, and centrifuged again for 2 minutes at 14,000 x g. Subsequently, the supernatant was removed and the resulting pellet dried in a SPEEDVAC™ drier and carried on into the next procedure.

Enzymatic digestion:

To the cDNA prepared with the Sal I adapter from the previous paragraph was added the following reagents and the mixture was incubated at 37°C for 2 hours: DEPC-treated water (41 ml); Not I restriction buffer (REACT, Life Tech., 5 ml), Not I (4 ml). The final composition of this reaction was the following: 50 mM Tris-HCl (pH 8.0); 10 mM $MgCl_2$; 100 mM NaCl; 1,200 U/ml Not I.

Gel isolation of cDNA:

The cDNA was size fractionated by acrylamide gel electrophoresis on a 5 % acrylamide gel, and any fragments which were larger than 1 kb, as determined by comparison with a molecular weight marker, were excised from the gel. The cDNA was then electroeluted from the gel into 0.1 x TBE buffer (200 ml) and extracted with phenol:chloroform: isoamyl alcohol (25:24:1, 200 ml). The aqueous phase was removed, collected and centrifuged for 20 minutes at 14,000 x g. The supernatant was removed from the DNA pellet which was resuspended in 70% ethanol (0.5 ml) and centrifuged again for 2 minutes at 14,000 x g. The supernatant was again discarded, the pellet dried in a speedvac and resuspended in distilled water (15 ml).

Ligation of cDNA into pRK5B vector:

The following reagents were added together and incubated at 16°C for 16 hours: 5X T4 ligase buffer (3 ml); pRK5B, XhoI, NotI digested vector, 0.5 mg, 1 ml); cDNA prepared from previous paragraph (5 ml) and distilled water (6 ml). Subsequently, additional distilled water (70 ml) and 10 mg/ml tRNA (0.1 ml) were added and the entire reaction was extracted through phenol:chloroform:isoamyl alcohol (25:24:1). The aqueous phase was removed, collected and diluted into 5M NaCl (10 ml) and absolute ethanol (-20°C, 250 ml). This was then centrifuged for 20 minutes at 14,000 x g, decanted, and the pellet resuspended into 70% ethanol (0.5 ml) and centrifuged again for 2 minutes at 14,000 x g. The DNA pellet was then dried in a speedvac and eluted into distilled water (3 ml) for use in the subsequent procedure.

Transformation of library ligation into bacteria:

The ligated cDNA/pRK5B vector DNA prepared previously was chilled on ice to which was added electrocompetent DH10B bacteria (Life Tech., 20 ml). The bacteria vector mixture was then electroporated as per the manufacturers recommendation. Subsequently SOC media (1 ml) was added and the mixture was incubated at 37 C for 30 minutes. The transformants were then plated onto 20 standard 150 mm LB plates containing ampicillin and incubated for 16 hours (37 C) to allow the colonies to grow. Positive colonies were then scraped off and the DNA isolated from the bacterial pellet using standard CsCl-gradient protocols (see, for example, Ausubel et al., supra, 2.3.1.).

Isolation of full-length ilp

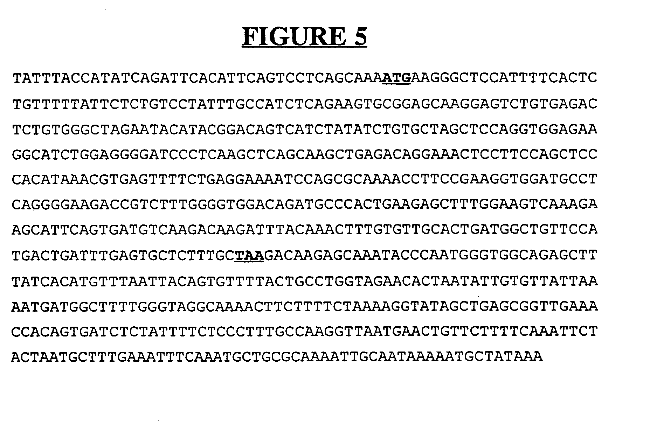

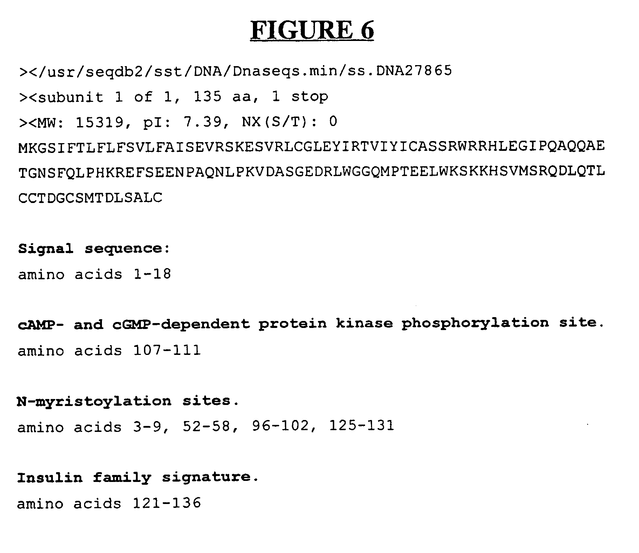

The full length nucleic acid sequence of ilp was obtained by screening a plasmid cDNA library (prepared from uterine mRNA as described above) by colony hybridization using oligonucleotides designed based on the EST sequences from Incyte, Inc (Incyte EST INC2328985) and Incyte EST INC778319). The primer oligonucleotide sequences were 5'-CACATTCAGTCCTCAGCAAAATGAA-3' (SEQ ID NO:29); 5'-GAGAATAAAAACAGAGTGAAAATGGAGCCCTTCATTTTGC-3' (SEQ ID NO:30); and 5'-CTCAGCTTGCTGAGCTTGAGGGA-3' (SEQ ID NO:31). The sequence of the cDNA obtained by this procedure were determined by standard techniques. The nucleic acid of the cDNA is shown in Figure 5 and amino acid sequences of PRO182 is shown in Figure 6.

D. Isolation of cDNA clones Encoding PRO366 (UNQ321; Human Apo-2DcR)

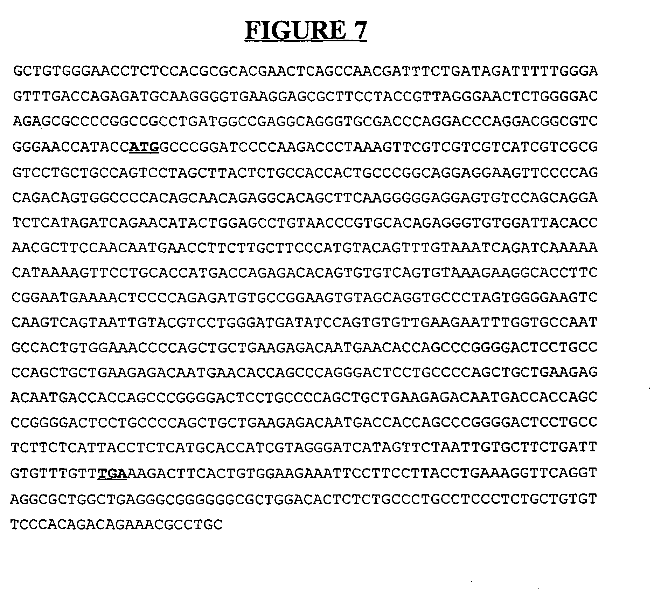

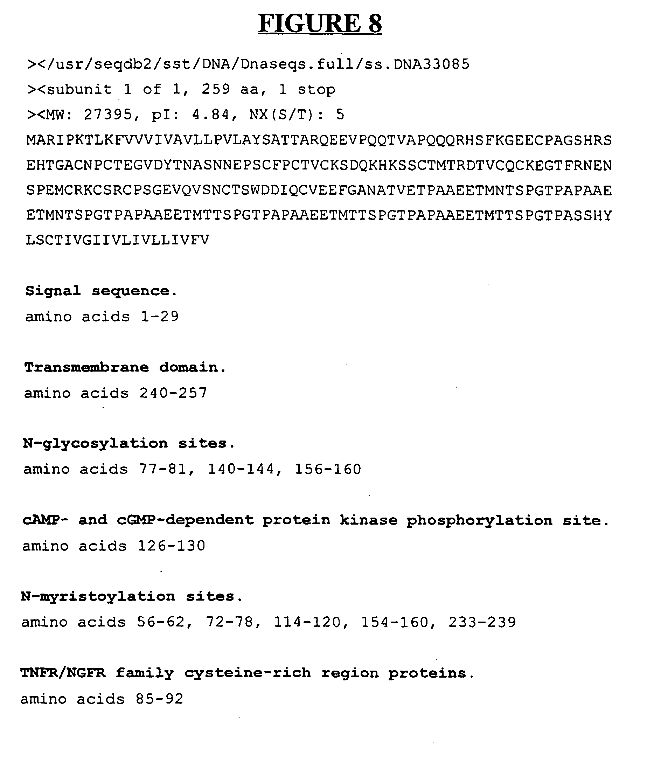

Using the yeast screen described in Example 1, part A above, the DNA33085-1110 molecule was isolated from a human breast carcinoma library. Specifically, a full length clone was identified (DNA33085-1110) (pRK5-hApo-2DcR) (also referred to as Apo2-DcR deposited as ATCC 209087, as indicated below) that contained a single open reading frame with an apparent translational initiation site at nucleotide positions 193-195 and ending at the stop codon found at nucleotide positions 970-972 (Figure 7; SEQ ID NO:7). The predicted polypeptide precursor is 259 amino acids long and has a calculated molecular weight of approximately 27.4 kDa. Sequence analysis indicated an N-terminal signal peptide, two cysteine-rich domains, a sequence that contains four nearly identical 15 amino acid tandem repeats, and a hydrophobic C-terminal region (amino acid residues 41-299 in Figure 8). The hydrophobic sequence at the C-terminus is preceded by a pair of small amino acids (Ala223 and Ala224); this structure and the absence of an apparent cytoplasmic domain suggests that PRO366 (Apo-2DcR) may be a glycosylphosphatydilinositol (GPI) anchored protein [see, Moran, J. Biol. Chem., 266:1250-1257 (1991)]. Apo-2DcR contains five potential N-linked glycosylation sites.

TNF receptor family proteins are typically characterized by the presence of multiple (usually four) cysteine-rich domains in their extracellular regions -- each cysteine-rich domain being approximately 45 amino acids long and containing approximately 6, regularly spaced, cysteine residues. Based on the crystal structure of the type 1 TNF receptor, the cysteines in each domain typically form three disulfide bonds in which usually cysteines 1 and 2, 3 and 5, and 4 and 6 are paired together. Like DR4 and Apo-2 (described further below), Apo-2DcR contains two extracellular cysteine-rich pseudorepeats, whereas other identified mammalian TNFR family members contain three or more such domains [Smith et al., Cell, 76:959 (1994)].

Based on an alignment analysis of the full-length sequence shown in Figure 7 (SEQ ID NO:7), Apo-2DcR shows more sequence identity to DR4 and Apo-2 than to other apoptosis-linked receptors, such as Apo-3, TNFR1, or Fas/Apo-1.

Applicants have shown that the apparent translational initiation site may alternatively be assigned at nucleotide positions 93-95 (amino acid residue 1 in Figure8). The Apo-2DcR shown in Figure 8 includes amino acid residues 1 to 299.

E. Isolation of cDNA Clones Encoding Human PRO240 (UNQ214)

The extracellular domain (ECD) sequences (including the secretion signal, if any) of from about 950 known secreted proteins from the Swiss-Prot public protein database were used to search expressed sequence tag (EST) databases. The EST databases included public EST databases (e.g., GenBank) and a proprietary EST DNA database (LIFESEQTM, Incyte Pharmaceuticals, Palo Alto, CA). The search was performed using the computer program BLAST or BLAST2 (Altshul et al., Methods in Enzymology 266:460-480 (1996)) as a comparison of the ECD protein sequences to a 6 frame translation of the EST sequence. Those comparisons resulting in a BLAST score of 70 (or in some cases 90) or greater that did not encode known proteins were clustered and assembled into consensus DNA sequences with the program "phrap" (Phil Green, University of Washington, Seattle, Washington).

Oligonucleotides were synthesized to identify by PCR a cDNA library that contained the sequence of interest and for use as probes to isolate a clone of the full-length coding sequence for PRO240.

A pair of PCR primers (forward and reverse) were synthesized:

forward PCR primer: 5'-TCAGCTCCAGACTCTGATACTGCC-3' (SEQ ID NO:32)

reverse PCR primer: 5'-TGCCTTTCTAGGAGGCAGAGCTCC-3' (SEQ ID NO:33)

Additionally, a synthetic oligonucleotide hybridization probe was constructed which had the following nucleotide sequence:

hybridization probe:

5'-GGACCCAGAAATGTGTCCTGAGAATGGATCTTGTGTACCTGATGGTCCAG-3' (SEQ ID NO:34)

In order to screen several libraries for a source of a full-length clone, DNA from the libraries was screened by PCR amplification with the PCR primer pair identified above. A positive library was then used to isolate clones encoding the PRO240 gene using the probe oligonucleotide and one of the PCR primers.

RNA for construction of the cDNA libraries was isolated from human fetal liver tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

DNA sequencing of the clones isolated as described above gave the full-length DNA sequence for PRO240 [herein designated as DNA34387-1138] and the derived protein sequence for PRO240.

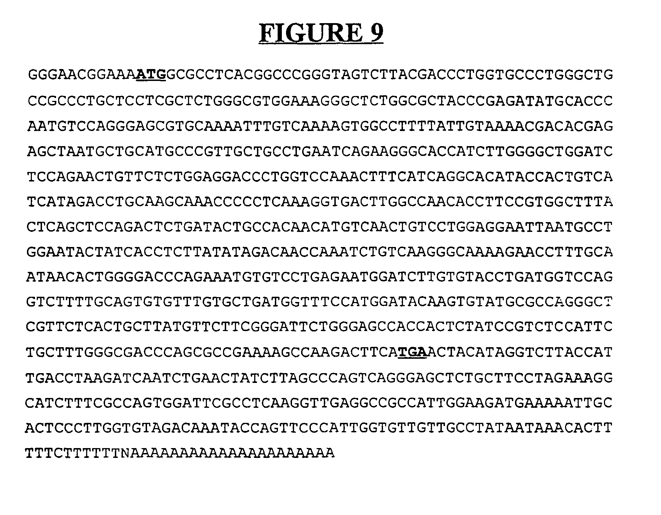

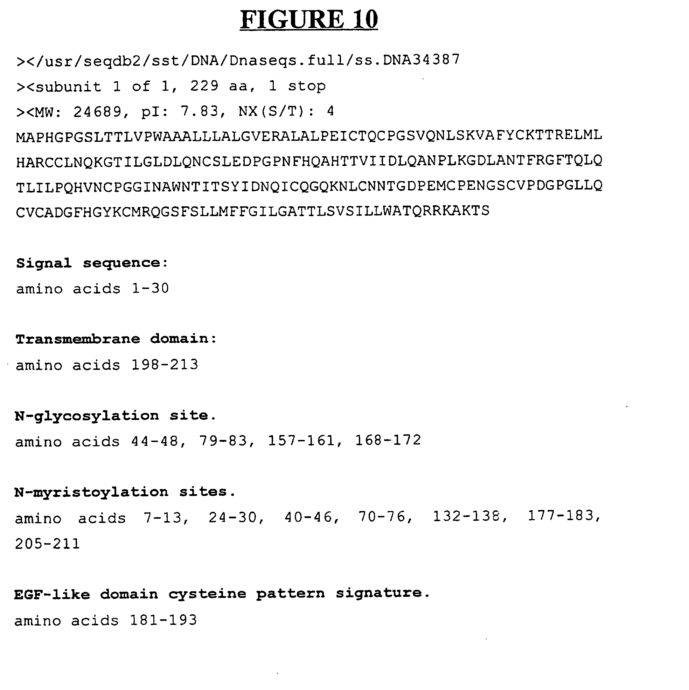

The entire nucleotide sequence of DNA34387-1138 is shown in Figure 9 (SEQ ID NO:9). Clone DNA34387-1138 contains a single open reading frame with an apparent translational initiation site at nucleotide positions 12-14 and ending at the stop codon at nucleotide positions 699-701. The predicted polypeptide precursor is 229 amino acids long (Figure 10). Clone DNA34387-1138 has been deposited with ATCC on September 16, 1997 and is assigned ATCC deposit no. 209260.

Analysis of the amino acid sequence of the full-length PRO240 suggests that it possesses 30% and 35% amino acid identity with the serrate precursor protein from Drospohilia melanogaster and the C-serrate-1 protein from Gallus gallus.

F. Isolation of cDNA Clones Encoding Human PRO256 (UNQ223)

A consensus DNA sequence was assembled relative to other EST sequences using phrap as described in Example 1, part E above. This consensus sequence is herein designated DNA28725. Based on the DNA28725 consensus sequence, oligonucleotides were synthesized: 1) to identify by PCR a cDNA library that contained the sequence of interest, and 2) for use as probes to isolate a clone of the full-length coding sequence for PRO256. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, with the PCR primer pair. A positive library was then used to isolate clones encoding the gene of interest using the probe oligonucleotide and one of the primer pairs.

A pair of PCR primers (forward and reverse) were synthesized:

forward PCR primer: 5'-TGTCCACCAAGCAGACAGAAG-3' (SEQ ID NO:35)

reverse PCR primer: 5'-ACTGGATGGCGCCTTTCCATG-3' (SEQ ID NO:36)

Additionally, two synthetic oligonucleotide hybridization probes were constructed from the consensus DNA28725 sequence which had the following nucleotide sequence:

hybridization probes:

5'-CTGACAGTGACTAGCTCAGACCACCCAGAGGACACGGCCAACGTCACAGT-3' (SEQ ID NO:37)

5'-GGGCTCTTTCCCACGCTGGTACTATGACCCCACGGAGCAGATCTG-3' (SEQ ID NO:38)

In order to screen several libraries for a source of a full-length clone, DNA from the libraries was screened by PCR amplification with the PCR primer pair identified above. A positive library was then used to isolate clones encoding the PRO256 gene using one of the probe oligonucleotides and one of the PCR primers.

RNA for construction of the cDNA libraries was isolated from human placenta tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

DNA sequencing of the clones isolated as described above gave the full-length DNA sequence for PRO256 [herein designated as DNA35880-1160] (SEQ ID NO:11) and the derived protein sequence for PRO256.

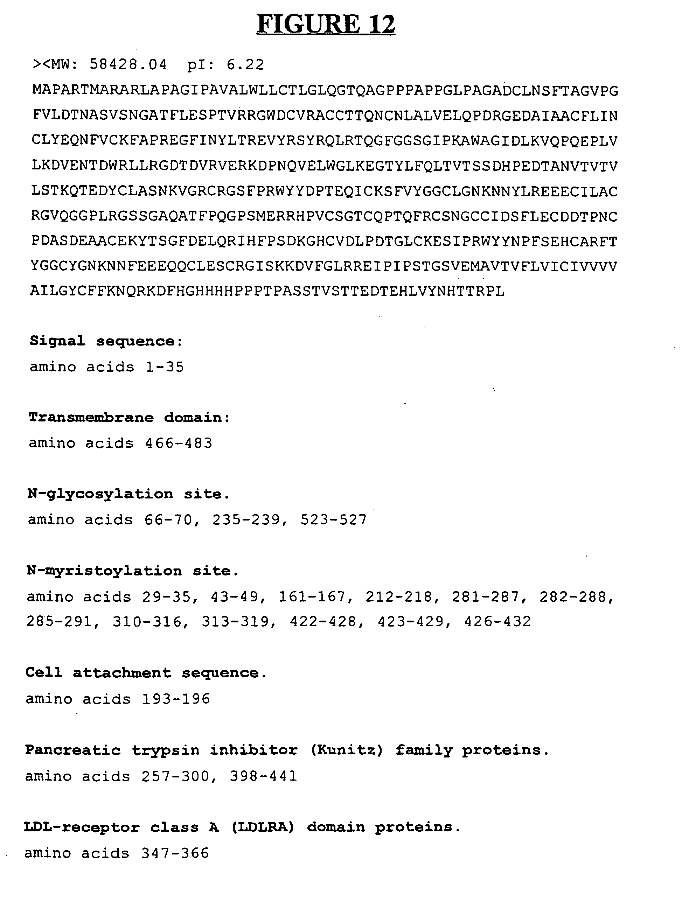

The entire nucleotide sequence of DNA35880-1160 is shown in Figure I 1 (SEQ ID NO:11). Clone DNA35880-1160 contains a single open reading frame with an apparent translational initiation site at nucleotide positions 188-190 and ending at the stop codon at nucleotide positions 1775-1777. The predicted polypeptide precursor is 529 amino acids long (Figure 12). Clone DNA35880-1160 has been deposited with ATCC and is assigned ATCC deposit no. 209379.

Analysis of the amino acid sequence of the full-length PRO256 polypeptide suggests that portions of it possess significant homology to the human bikunin protein, thereby indicating that PRO256 may be a novel proteinase inhibitor.

G. Isolation of cDNA Clones Encoding Human PRO306 (UNQ269)

A consensus DNA sequence was assembled relative to other EST sequences using phrap as described in Example 1, part E above. This consensus sequence is herein designated DNA36458. The consensus DNA sequence was extended using repeated cycles of BLAST and phrap to extend the consensus sequence as far as possible using the sources of EST sequences discussed above.

Based on the DNA36458 consensus sequence, oligonucleotides were synthesized: 1) to identify by PCR a cDNA library that contained the sequence of interest, and 2) for use as probes to isolate a clone of the full-length coding sequence for PRO306. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as ber Ausubel et al., Current Protocols in Molecular Biology, with the PCR primer pair. A positive library was then used to isolate clones encoding the gene of interest using the probe oligonucleotide and one of the primer pairs.

Forward and reverse PCR primers were synthesized:

forward PCR primer: 5'-CAGGTCGAACCCAGACCACGATGC-3' (SEQ ID NO:39)

forward PCR primer: 5'-GCCACATGGCCCAGCTTG-3' (SEQ ID NO:40)

forward PCR primer: 5'-GAGACGGAGGAAGCAGGC-3' (SEQ ID NO:41)

forward PCR primer: 5'-GGCCACACTTACAGCTCTG-3' (SEQ ID NO:42)

reverse PCR primer: 5'-AGCCGGCTTCTGAGGGCGTCTACC-3' (SEQ ID NO:43)

Additionally, a synthetic oligonucleotide hybridization probe was constructed from the consensus DNA36458 sequence which had the following nucleotide sequence:

hybridization probe:

5'-TGGTGCTGCCGCTGCTGCTCCTGGCCGCGGCAGCCCTGGCCGAAG-3' (SEQ ID NO:44)

In order to screen several libraries for a source of a full-length clone, DNA from the libraries was screened by PCR amplification with one of the PCR primer pairs identified above. A positive library was then used to isolate clones encoding the PRO306 gene using the probe oligonucleotide and one of the PCR primers.

RNA for construction of the cDNA libraries was isolated from human fetal kidney tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

DNA sequencing of the clones isolated as described above gave the full-length DNA sequence for PRO306 [herein designated as DNA39984-1221] (SEQ ID NO:13) and the derived protein sequence for PRO306.

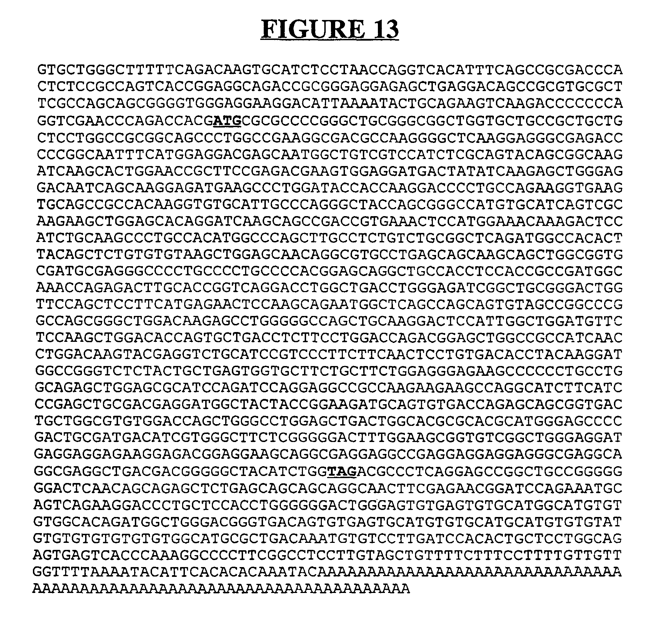

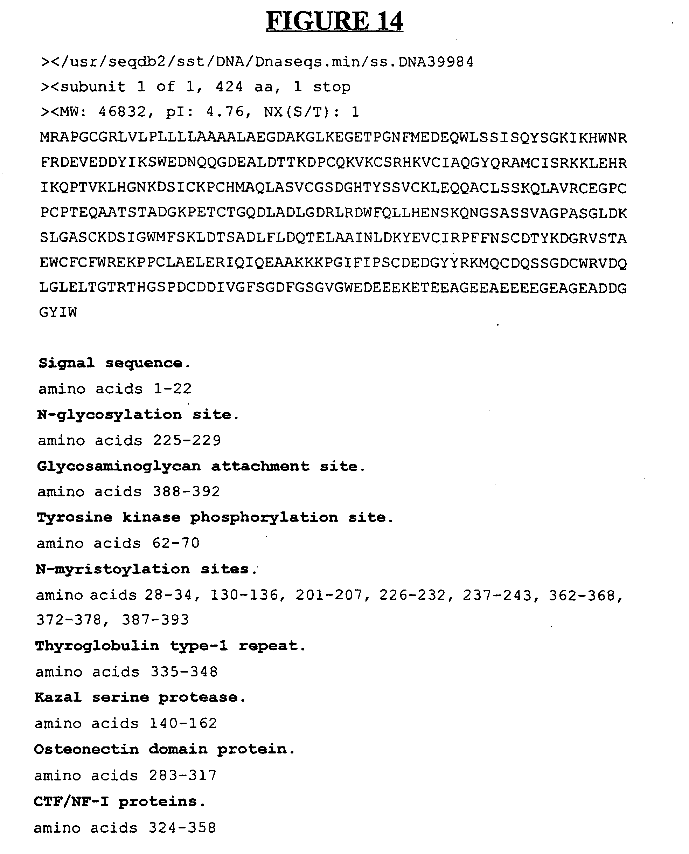

The entire nucleotide sequence of DNA39984-1221 is shown in Figure 13 (SEQ ID NO:13). Clone DNA39984-1221 contains a single open reading frame with an apparent translational initiation site at nucleotide positions 199-201 and ending at the stop codon at nucleotide positions 1471-1473. The predicted polypeptide precursor is 424 amino acids long (Figure 14). Clone DNA39984-1221 has been deposited with ATCC and is assigned ATCC deposit no. 209435.

Analysis of the amino acid sequence of the full-length PRO306 polypeptide suggests that portions of it possess significant homology to the testican protein, and to the IGF binding protein thereby indicating that PRO306 may be a novel testican or IGF binding protein.

H. Isolation of cDNA Clones Encoding Human PRO364 (UNQ319)

An expressed sequence tag (EST) DNA database (LIFESEQ™, Incyte Pharmaceuticals, Palo Alto, CA) was searched and an EST (Incyte EST No. 3003460) was identified that encoded a polypeptide which showed homology to members of the tumor necrosis factor receptor (TNFR) familiy of polypeptides.

A consensus DNA sequence was then assembled relative to the Incyte 3003460 EST and other EST sequences using repeated cycles of BLAST (Altshul et al., Methods in Enzymology 266:460-480 (1996)) and "phrap" (Phil Green, University of Washington, Seattle, Washington). This consensus sequence is herein designated DNA44825.

Based upon the DNA44825 consensus sequence, oligonucleotides were synthesized: 1) to identify by PCR a cDNA library that contained the sequence of interest, and 2) for use as probes to isolate a clone of the full-length coding sequence for PRO364. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al. , Current Protocols in Molecular Biology, with the PCR primer pair. A positive library was then used to isolate clones encoding the gene of interest using the probe oligonucleotide and one of the primer pairs.

Pair of PCR primers (forward and reverse) were synthesized:

forward PCR primer: 5'-CACAGCACGGGGCGATGGG-3' (SEQ ID NO:45)

forward PCR primer: 5'-GCTCTGCGTTCTGCTCTG-3' (SEQ ID NO:46)

forward PCR primer: 5'-GGCACAGCACGGGGCGATGGGCGCGTTT-3' (SEQ ID NO:47)

reverse PCR primer: 5'-CTGGTCACTGCCACCTTCCTGCAC-3' (SEQ ID NO:48)

reverse PCR primer: 5'-CGCTGACCCAGGCTGAG-3' (SEQ ID NO:49)

reverse PCR primer: 5'-GAAGGTCCCCGAGGCACAGTCGATACA-3' (SEQ ID NO:50)

Additionally, synthetic oligonucleotide hybridization probes were constructed from the consensus DNA44825 sequence which had the following nucleotide sequences:

hybridization probes:

5'-GAGGAGTGCTGTTCCGAGTGGGACTGCATGTGTGTCCAGC-3' (SEQ ID NO:51)

5'-AGCCTGGGTCAGCGCCCCACCGGGGGTCCCGGGTGCGGCC-3' (SEQ ID NO:52)

In order to screen several libraries for a source of a full-length clone, DNA from the libraries was screened by PCR amplification with the PCR primer pairs identified above. A positive library was then used to isolate clones encoding the PRO364 gene using the probe oligonucleotides and one of the PCR primers.

RNA for construction of the cDNA libraries was isolated from human small intestine tissue (LIB231). The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

DNA sequencing of the clones isolated as described above gave the full-length DNA sequence for PRO364 [herein designated as DNA47365-1206] (SEQ ID NO:15) and the derived protein sequence for PRO364.

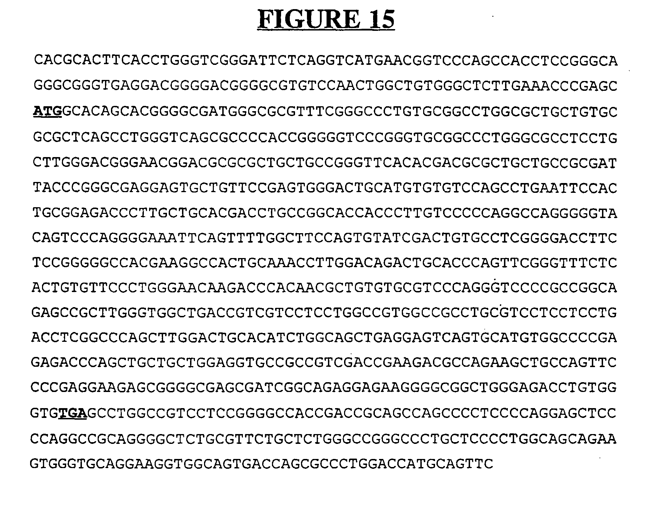

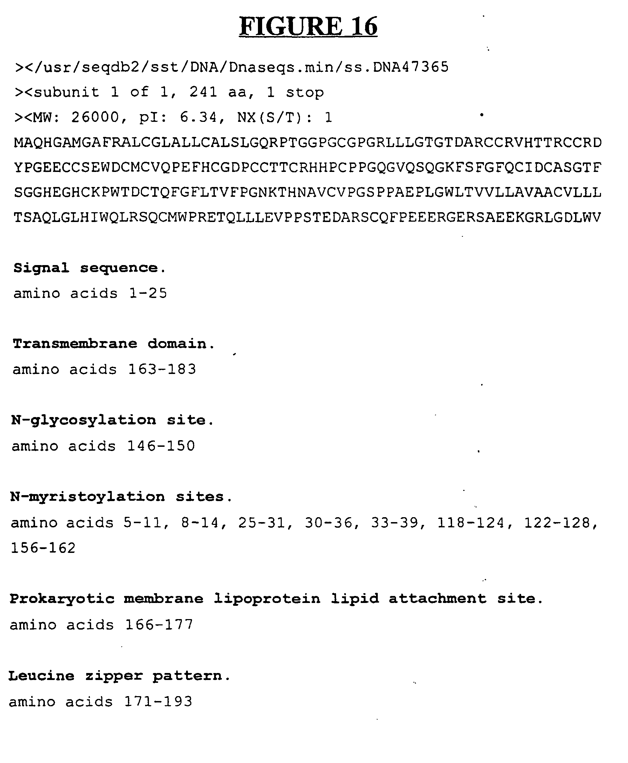

The entire nucleotide sequence of DNA47365-1206 is shown in Figure 15 (SEQ ID NO:15). Clone DNA47365-1206 contains a single open reading frame with an apparent translational initiation site at nucleotide positions 121-123 and ending at the stop codon at nucleotide positions 844-846. The predicted polypeptide precursor is 241 amino acids long (Figure 16). The full-length PRO364 protein has an estimated molecular weight of about 26,000 daltons and a pI of about 6.34. A potential N-glycosylation sites exists between amino acids 146 and 149 of the amino acid sequence shown in Figure 16. A putative signal sequence is from amino acids 1 to 25 and a potential transmembrane domain exists between amino acids 162 to 180. Clone DNA47365-1206 has been deposited with ATCC and is assigned ATCC Deposit No. ATCC 209436.

Analysis of the amino acid sequence of the full-length PRO364 polypeptide suggests that portions of it possess significant homology to members of the tumor necrosis factor receptor family, thereby indicating that PRO364 may be a novel member of the tumor necrosis factor receptor family.

A detailed review of the amino acid sequence of the full-length native PRO364 polypeptide and the nucleotide sequence that encodes that amino acid sequence evidences sequence homology with the mouse GITR protein reported by Nocentini et al., Proc. Natl. Acad. Sci. USA 94:6216-6221 (1997). It is possible, therefore, that PRO364 represents the human couterpart to the mouse GITR protein reported by Nocentini et al.

I. Isolation of cDNA clones Encoding Human NL4 (PRO356; UNQ313)

An expressed sequence tag (EST) DNA database (LIFESEQ™, Incyte Pharmaceuticals, Palo Alto, CA) was searched and an EST (#2939340) was identified which showed homology to human TIE-2 L1 and TIE-2 L2.

Based on the EST, a pair of PCR primers (forward and reverse), and a probe were synthesized:

NL4,5-1: 5'-TTCAGCACCAAGGACAAGGACAATGACAACT-3' (SEQ ID NO:53)

NL4,3-1: 5'-TGTGCACACTTGTCCAAGCAGTTGTCATTGTC-3' (SEQ ID NO:54)

NL4,3-3: 5'-GTAGTACACTCCATTGAGGTTGG-3' (SEQ ID NO:55).

Oligo dT primed cDNA libraries were prepared from uterus mRNA purchased from Clontech, Inc. (Palo Alto, CA, USA, catalog # 6537-1) in the vector pRK5D using reagents and protocols from Life Technologies, Gaithersburg, MD (Super Script Plasmid System). pRK5D is a cloning vector that has an sp6 transcription initiation site followed by an SfiI restriction enzyme site preceding the XhoI/NotI cDNA cloning sites. The cDNA was primed with oligo dT containing a NotI site, linked with blunt to SalI hemikinased adaptors, cleaved with NotI, sized to greater than 1000 bp appropriately by gel electrophoresis, and cloned in a defined orientation into XhoI/NotI-cleaved pRK5D.

In order to screen several libraries for a source of a full-length clone, DNA from the libraries was screened by PCR amplification with the PCR primer pair identified above. A positive library was then used to isolate clones encoding the NL4 gene using the probe oligonucleotide and one of the PCR primers.

DNA sequencing of the clones isolated as described above gave the full-length DNA sequence for NL4 and the derived protein sequence.

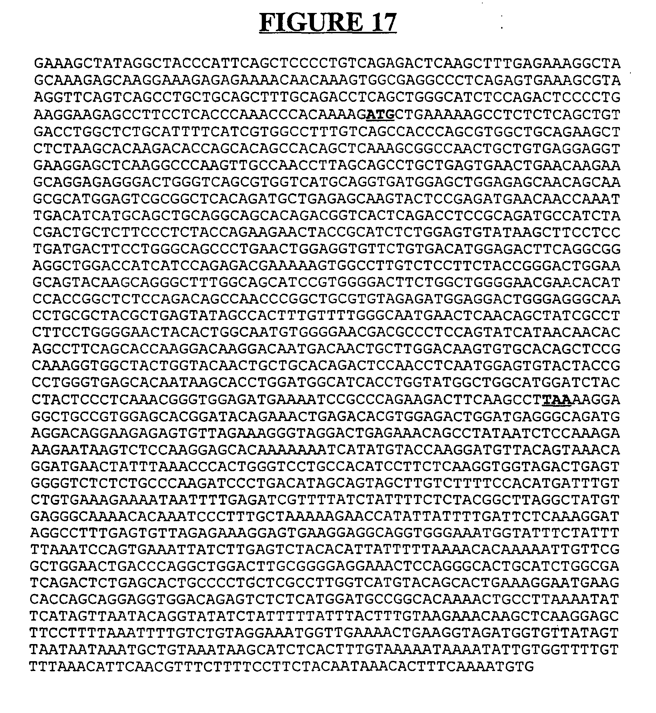

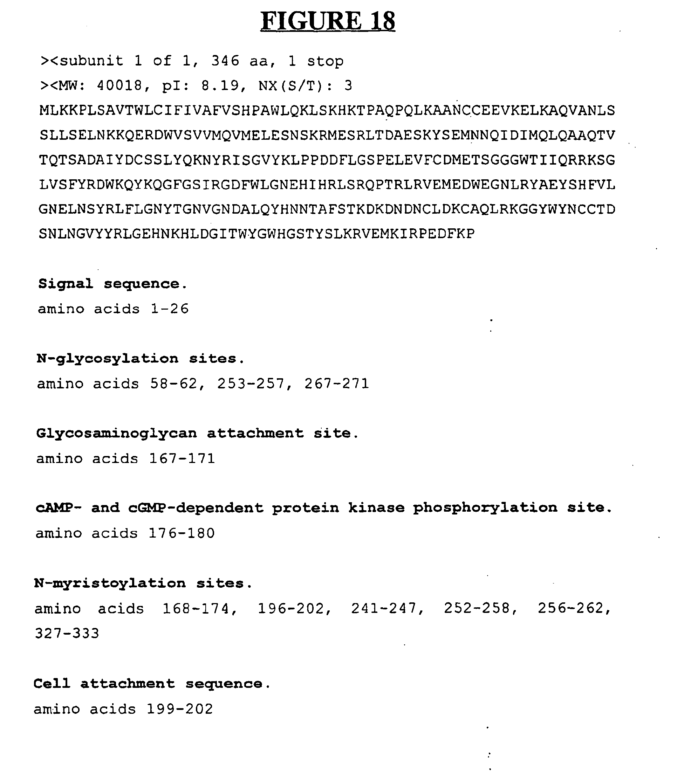

The entire nucleotide sequence of PRO356 is shown in Figure 17 (SEQ ID NO:17). Clone DNA47470-1130 contains a single open reading frame with an apparent translational initiation site at nucleotide positions 215-217. In Figure 17, the TAA stop codon is at nucleotide positions 1039-1041. The predicted polypeptide is 346 amino acids long (Figure 18). Clone DNA47470-1130 has been deposited with ATCC and is assigned ATCC deposit no. 209422.

Based on a BLAST and FastA sequence alignment analysis of the full-length sequence, PRO356 shows amino acid sequence identity to TIE2L1 (32%) and TIE2L2 (34%).

J. Isolation of cDNA clones Encoding Human PRO826 (UNQ467)

DNA57694-1341 was identified by applying a proprietary signal sequence finding algorithm developed by Genentech, Inc. (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST cluster sequence from the Incyte database, designated 47283. This EST cluster sequence was then compared to a variety of expressed sequence tag (EST) databases which included public EST databases (e.g., GenBank) and a proprietary EST DNA database (Lifeseq®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA56000.

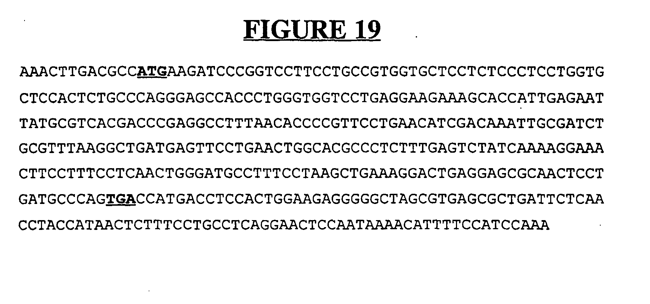

In light of an observed sequence homology between the DNA56000 sequence and an EST sequence contained within the Merck EST clone no. W69233, the Merck EST clone W69233 was purchased and the cDNA insert was obtained and sequenced. The sequence of this cDNA insert is shown in Figure 19 and is herein designated as DNA57694-1341.

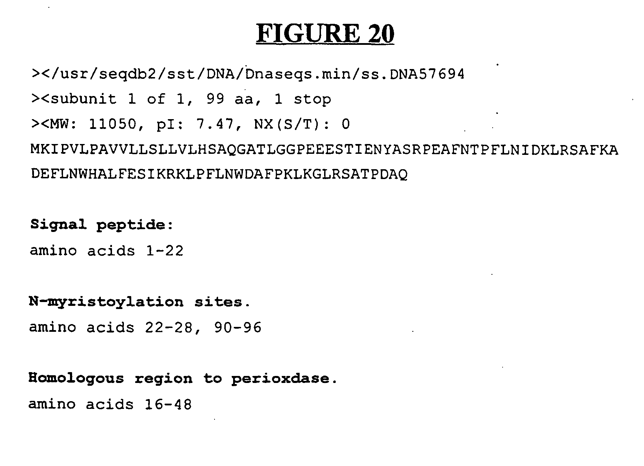

Clone DNA57694-1341 contains a single open reading frame with an apparent translational initiation site at nucleotide positions 13-15 and ending at the stop codon at nucleotide positions 310-312. The predicted polypeptide precursor is 99 amino acids long (Figure 20). The full-length PRO826 protein has an estimated molecular weight of about 11,050 daltons and a pI of about 7.47. Analysis of the full-length PRO826 sequence shown in Figure 20 (SEQ ID NO:20) evidences the presence of the following: a signal peptide from about amino acid 1 to about amino acid 22, potential N-myristoylation sites from about amino acid 22 to about amino acid 27 and from about amino acid 90 to about amino acid 95 and an amino acid sequence block having homology to peroxidase from about amino acid 16 to about amino acid 48. Clone DNA57694-1341 has been deposited with ATCC on June 23, 1998 and is assigned ATCC deposit no.203017.

An analysis of the Dayhoff database (version 35.45 SwissProt 35), using a WU-BLAST2 sequence alignment analysis of the full-length sequence shown in Figure 20, evidenced significant homology between the PRO826 amino acid sequence and the following Dayhoff sequences: CCU12315_1, SCU96108_6, CELF39F10_4 4 and HELT_HELHO.

K. Isolation of cDNA clones Encoding Human PRO1068 (UNQ525)

Use of the signal sequence algorithm described in Example 1, part J above allowed identification of an EST cluster sequence from the LIFESEQ® database, designated Incyte cluster no. 141736. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. One or more of the ESTs was derived from a human mast cell line from a patient with mast cell leukemia. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA56094.

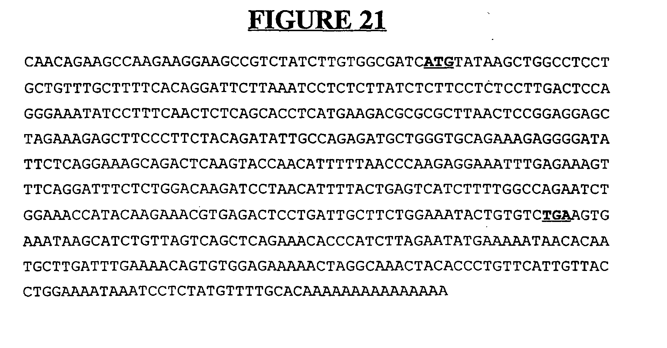

In light of an observed sequence homology between the DNA56094 sequence and an EST sequence contained within Incyte EST clone no. 004974, Incyte EST clone no. 004974 was purchased and the cDNA insert was obtained and sequenced. The sequence of this cDNA insert is shown in Figure 21 and is herein designated as DNA59214-1449 (SEQ ID NO:21).

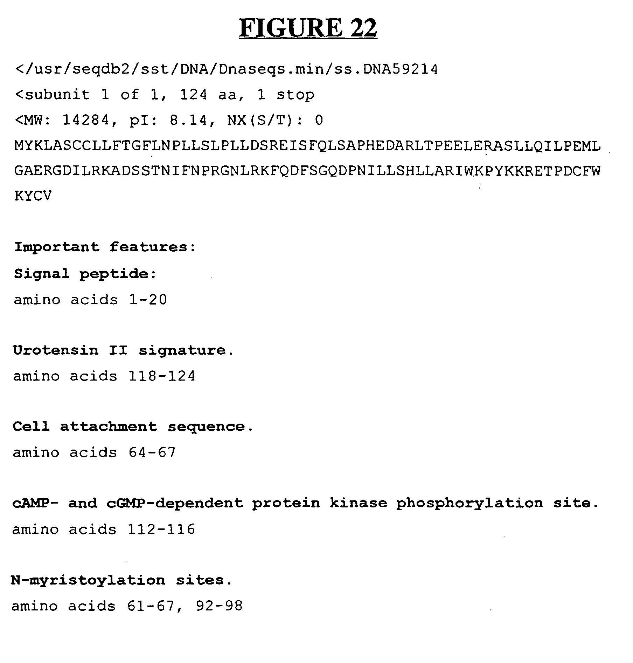

The full length clone shown in Figure 21 contained a single open reading frame with an apparent translational initiation site at nucleotide positions 42-44 and ending at the stop codon found at nucleotide positions 14-16. The predicted polypeptide precursor (Figure 22, SEQ ID NO:22) is 124 amino acids long. PRO1068 has a calculated molecular weight of approximately 14,284 Daltons and an estimated pI of approximately 8.14. The PRO1068 polypeptide has the following additional features, as indicated in Figure 22: a signal peptide sequence at about amino acids 1-20, a urotensin II signature sequence at about amino acids 118-123, a cell attachment sequence at about amino acids 64-66, and a potential cAMP- and cGMP-dependent protein kinase phosphorylation site at about amino acids 112-115.

An analysis of the Dayhoff database (version 35.45 SwissProt 35), using a WU-BLAST2 sequence alignment analysis of the full-length sequence shown in Figure 22, revealed homology between the PRO 1068 amino acid sequence and the following Dayhoff sequences: HALBOP_1, MTV043_36, I50498, and P_R78445

Clone DNA59214-1449 was deposited with the ATCC on July 1, 1998 and is assigned ATCC deposit no. 203046.

L. Isolation of cDNA clones Encoding Human PRO1343 (UN0698)

A cDNA sequence isolated in the amylase screen described ion Example 1, part A above was found, by the WU-BLAST2 sequence alignment computer program, to have no significant sequence identity to any known human encoding nucleic acid. This cDNA sequence obtained is herein designated DNA48921. Probes were generated from the sequence of the DNA48921 molecule and used to screen a human smooth muscle cell tissue library prepared as described in paragraph 1 of Example 1, part A above. The cloning vector was pRK5B (pRK5B is a precursor of pRK5D that does not contain the SfiI site; see, Holmes et al., Science, 253:1278-1280 (1991)), and the cDNA size cut was less than 2800 bp.

The oligonucleotide probes employed were as follows:

forward PCR primer: 5'-CAATATGCATCTTGCACGTCTGG-3' (SEQ ID NO:56)

reverse PCR primer: 5'-AAGCTTCTCTGCTTCCTTTCCTGC-3' (SEQ ID NO:57)

hybridization probe:

5'-TGACCCCATTGAGAAGGTCATTGAAGGGATCAACCGAGGGCTG-3' (SEQ ID NO:58)

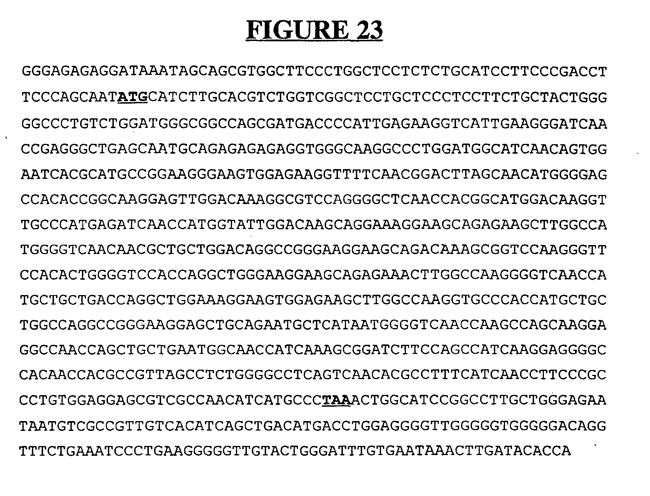

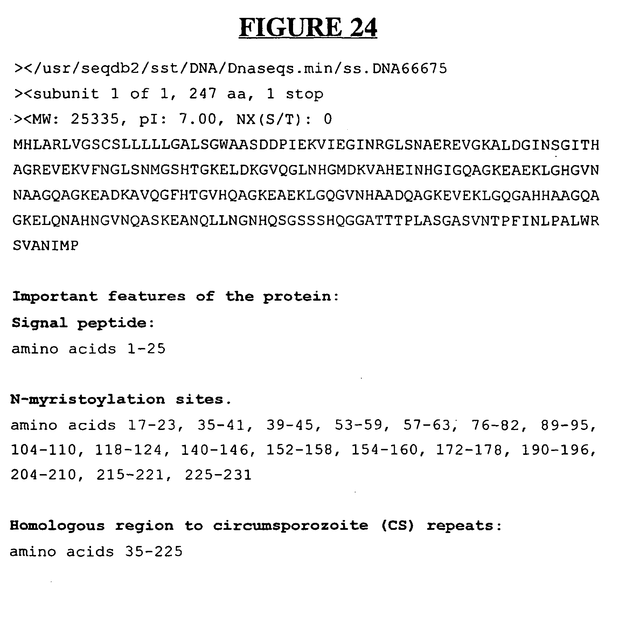

A full length clone was identified that contained a single open reading frame with an apparent translational initiation site at nucleotide positions 71-73, and a stop signal at nucleotide positions 812-814 (Figure 23, SEQ ID NO: 23). The predicted polypeptide precursor is 247 amino acids long, has a calculated molecular weight of approximately 25,335 daltons and an estimated pI of approximately 7.0. Analysis of the full-length PRO1343 sequence shown in Figure 24 (SEQ ID NO:24) evidences the presence of the following: a signal peptide from about amino acid 1 to about amino acid 25 and a homologous region to circumsporozoite repeats from about amino acid 35 to about amino acid 225. Clone DNA66675-1587 has been deposited with ATCC on September 22, 1998 and is assigned ATCC deposit no. 203115.

An analysis of the Dayhoff database (version 35.45 SwissProt 35), using a WU-BLAST2 sequence alignment analysis of the full-length sequence shown in Figure 24, evidenced significant homology between the PRO1343 amino acid sequence and the following Dayhoff sequences: CSP_PLACC, CEF25H8_2, U88974_40, BNAMRNAA_1, BOBOPC3_1, S58135, AF061832_1, BHU52040_1, HUMPROFILE_1 and MTV023_14.

Additionally, an Incyte EST clone (Incyte EST clone no. 4701148) having homology to the DNA48921 sequence was obtained and the insert sequence, thereby giving rise to the DNA66675-1587 sequence shown in Figure 23.

M. Isolation of cDNA clones Encoding Human PRO1375 (UNQ712)

A Merck/Washington University database was searched and a Merck EST was identified. This sequence was then put in a program which aligns it with other seeequences from the Swiss-Prot public database, public EST databases (e.g., GenBank, Merck/Wash. U.), and a proprietary EST database (LIFESEQ®, Incyte Pharmaceuticals, Palo Alto, CA). The search was performed using the computer program BLAST or BLAST2 [Altschul et al., Methods in Enzymology, 266:460-480 (1996)] as a comparison of the extracellular domain (ECD) protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington).

A consensus DNA sequence was assembled relative to other EST sequences using phrap. This consensus sequence is designated herein "DNA67003". Based on the DNA67003 consensus sequence, a nucleic acid was identified in a human pancreas library. DNA sequencing of the clone gave the full-length DNA sequence and the derived protein sequence for PRO1375.

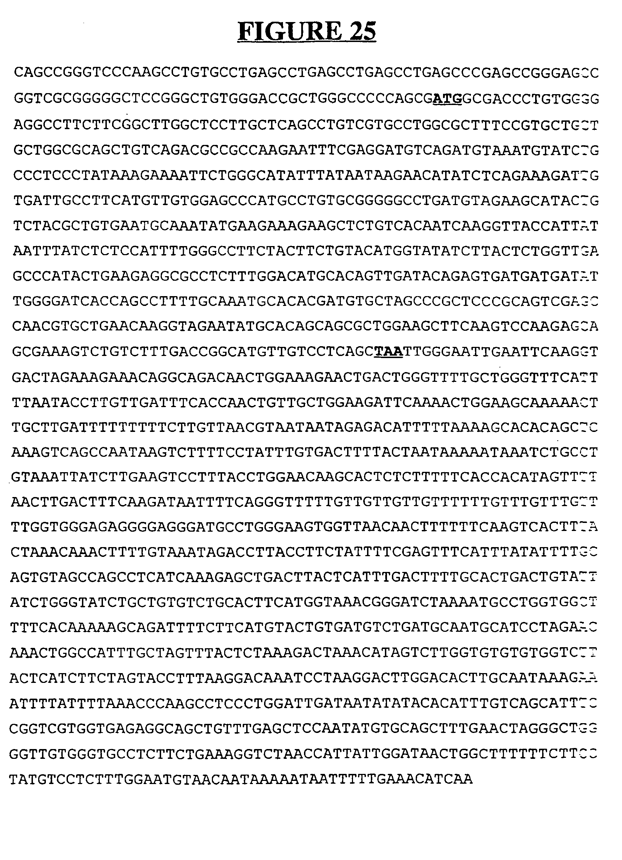

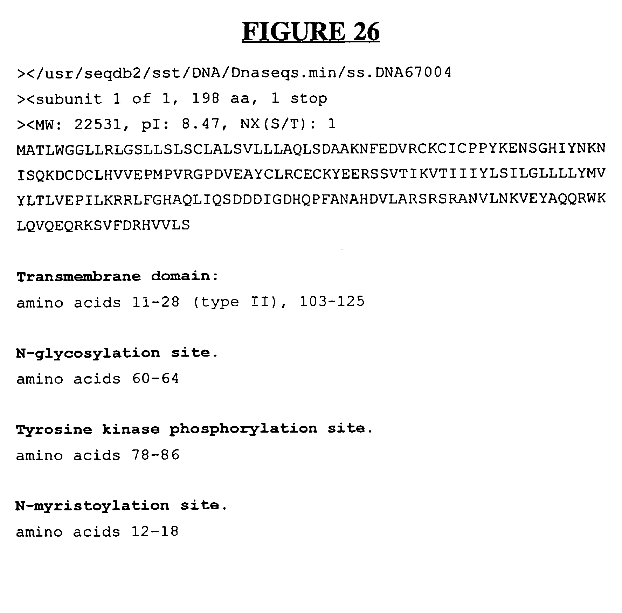

The entire coding sequence of PRO1375 is shown in Figure 25 (SEQ ID NO:25). Clone DNA67004-1614 contains a single open reading frame with an apparent translational initiation site at nucleotide positions 104-106 and an apparent stop codon at nucleotide positions 698-700. The predicted polypeptide precursor is 198 amino acids long and is shown in Figure 26 (SEQ ID NO:26). The transmembrane domains are at about amino acids 11-28 (type II) and 103-125. Clone DNA67004-1614 has been deposited with ATCC and is assigned ATCC deposit no. 203115. The full-length PRO1375 protein shown in Figure 26 has an estimated molecular weight of about 22,531 daltons and a pI of about 8.47.

An analysis of the Dayhoff database (version 35.45 SwissProt 35), using a WU-BLAST2 sequence alignment analysis of the full-length sequence shown in Figure 26, revealed sequence identity between the PRO1375 amino acid sequence and the following Dayhoff sequences: AF026198_5, CELR12C12_5, S73465, Y011_MYCPN, S64538_1, P_P8150, MUVSHPO10_1, VSH_MUMPL and CVU59751_5.

EXAMPLE 2: Stimulatory Activity in Mixed Lymphocyte Reaction (MLR) Assay (Assay 24)

This example shows that certain polypeptides of the invention are active as a stimulator of the proliferation of stimulated T-lymphocytes. Compounds which stimulate proliferation of lymphocytes are useful therapeutically where enhancement of an immune response is beneficial. A therapeutic agent may take the form of antagonists of the PRO polypeptide of the invention, for example, murine-human chimeric, humanized or human antibodies against the polypeptide.

The basic protocol for this assay is described in Current Protocols in Immunology, unit 3.12; edited by JE Coligan, AM Kruisbeek, DH Marglies, EM Shevach, W Strober, National Insitutes of Health, Published by John Wiley & Sons, Inc.

More specifically, in one assay variant, peripheral blood mononuclear cells (PBMC) are isolated from mammalian individuals, for example a human volunteer, by leukopheresis (one donor will supply stimulator PBMCs, the other donor will supply responder PBMCs). If desired, the cells are frozen in fetal bovine serum and DMSO after isolation. Frozen cells may be thawed overnight in assay media (37°C, 5% $CO_2$) and then washed and resuspended to $3x10^6$ cells/ml of assay media (RPMI; 10% fetal bovine serum, 1% penicillin/streptomycin, 1% glutamine, 1% HEPES, 1% non-essential amino acids, 1% pyruvate). The stimulator PBMCs are prepared by irradiating the cells (about 3000 Rads).

The assay is prepared by plating in triplicate wells a mixture of:

100:1 of test sample diluted to 1% or to 0.1%,
50 :1 of irradiated stimulator cells, and
50 :1 of responder PBMC cells.

100 microliters of cell culture media or 100 microliter of CD4-IgG is used as the control. The wells are then incubated at 37°C, 5% $CO_2$ for 4 days. On day 5, each well is pulsed with tritiated thymidine (1.0 mC/well; Amersham). After 6 hours the cells are washed 3 times and then the uptake of the label is evaluated.

In another variant of this assay, PBMCs are isolated from the spleens of Balb/c mice and C57B6 mice. The cells are teased from freshly harvested spleens in assay media (RPMI; 10% fetal bovine serum, 1% penicillin/streptomycin, 1% glutamine, 1% HEPES, 1% non-essential amino acids, 1% pyruvate) and the PBMCs are isolated by overlaying these cells over Lympholyte M (Organon Teknika), centrifuging at 2000 rpm for 20 minutes, collecting and washing the mononuclear cell layer in assay media and resuspending the cells to $1x10^7$ cells/ml of assay media. The assay is then conducted as described above.

Positive increases over control are considered positive with increases of greater than or equal to 180% being preferred. However, any value greater than control indicates a stimulatory effect for the test protein. The results are shown in Table 7 below.

Table 7

| PRO | PRO Concentration | Percent Increase Over Control |
|---|---|---|
| PR0256 | 1.80nM | 261.0 |
| " | 0.225nM | 202.0 |
| " | 0.028nM | 150.0 |
| PR0306 | 0.55nM | 204.1 |
| " | 5.46nM | 267.7 |
| PR0364 | 2.72nM | 120.2 |
| " | 27.23nM | 278.4 |
| PRO826 | 0.9nM | 144.8 |
| " | 0.9nM | 249.4 |
| " | 9.03nM | 191.9 |
| " | 9.03nM | 213.6 |
| PRO1068 | 1.67nM | 121.0 |
| " | 1.67nM | 229.9 |
| " | 16.72nM | 224.8 |
| " | 16.72nM | 276.3 |
| PRO1343 | 2.3nM | 205.1 |
| " | 2.3nM | 245.6 |
| " | 230nM | 107.7 |
| " | 230nM | 122.0 |
| PRO1375 | 5.9nM | 103.1 |
| " | 5.9nM | 141.2 |
| " | 59nM | 212.5 |
| " | 59nM | 221.7 |

EXAMPLE 3: Skin Vascular Permeability Assay (Assay 64)

This assay shows that certain polypeptides of the invention stimulate an immune response and induce inflammation by inducing mononuclear cell, eosinophil and PMN infiltration at the site of injection of the animal. Compounds which stimulate an immune response are useful therapeutically where stimulation of an immune response is beneficial. This skin vascular permeability assay is conducted as follows. Hairless guinea pigs weighing 350 grams or more are anesthetized with ketamine (75-80 mg/Kg) and 5 mg/Kg xylazine intramuscularly (IM). A sample of purified PRO polypeptide of the invention or a conditioned media test sample is injected intradermally onto the backs of the test animals with 100 µl per injection site. It is possible to have about 10-30, preferably about 16-24, injection sites per animal. One µl of Evans blue dye (1% in physiologic buffered saline) is injected intracardially. Blemishes at the injection sites are then measured (mm diameter) at 1 hr and 6 hr post injection. Animals were sacrificed at 6 hrs after injection. Each skin injection site is biopsied and fixed in formalin. The skins are then prepared for histopathologic evaluation. Each site is evaluated for inflammatory cell infiltration into the skin. Sites with visible inflammatory cell inflammation are scored as positive. Inflammatory cells may be neutrophilic, eosinophilic, monocytic or lymphocytic. At least a minimal perivascular infiltrate at the injection site is scored as positve, no infiltrate at the site of injection is scored as negative. The following polypeptides tested positive in this assay: PRO175, PRO179, PRO182, PRO240, PRO366 and PRO1375.

EXAMPLE 4: Human Co-Stimulation Assay

In addition to the activation signal mediated by the T cell receptor, T cell activation requires a costimulatory signal. One costimulatory signal is generated by the interaction of B7 (CD3) with CD28. In this assay, 96 well plates are coated with CD3 with or without CD28 and then human peripheral blood lymphocytes followed by a test protein, are added. Proliferation of the lymphocytes is determined by tritiated thymidine uptake. A positive assay indicates that the test protein provided a stimulatory signal for lymphocyte proliferation.

Material:

1) Hyclone D-PBS without Calcium, Magnesium
2) Nunc 96 well certified plates #4-39454
3) Anti-human CD3 Amac 0178 200 μg/ml stock
4) Anti-human CD28 Biodesign P42235M
5) Media: Gibco RPMI 1640 + 10% Intergen #1020-90 FBS, 1% Glu, 1 % P/S, 50 μg/ml Gentamycin. 10 mM Hepes. Fresh for each assay.
6) Tritiated Thymidine
7) Frozen human peripheral blood lymphocytes (PBL) collected via a leukophoresis procedure.

Plates are prepared by coating 96 well flat bottom plates with anti-CD3 antibody (Amac) or anti-CD28 antibody (Biodesign) or both in Hyclone D-PBS without calcium and magnesium. Anti-CD3 antibody is used at 10 ng/well (50:1 of 200 ng/ml) and anti-CD28 antibody at 25 ng/well (50:1 of 0.5 μg/ml) in 100:1 total volume.

PBLs are isolated from human donors using standard leukophoresis methods. The cell preparations are frozen in 50% fetal bovine serum and 50% DMSO until the assay is conducted. Cells are prepared by thawing and washing PBLs in media, resuspending PBLs in 25 mls of media and incubating at 37°C. 5% $CO_2$ overnight.

In the assay procedure, the coated plate is washed twice with PBS and the PBLs are washed and resuspended to $1x10^6$ cells/ml using 100μl/well. 100 μl of a test protein or control media are added to the plate making a total volume per well of 200 μl. The plate is incubated for 72 hours. The plate is then pulsed for 6 hours with tritiated thymidine (1 mC/well; Amersham) and the PBLs are harvested from the plates and evaluated for uptake of the tritiated thymidine.

The results of this assay for compounds of the invention are shown in Table 8 below. Positive increases over control are considered positive with increases of greater than or equal to 180% being preferred. However, any value greater than control indicates a stimulatory effect for the test protein. PBMC means peripheral blood mononuclear cells.

Table 8

| PRO | PRO Concentration | Percent Increase Over Control |
|---|---|---|
| PR0256 | 0.4% of stock (CD4) | 254 (stock=180nM) |
| PR0256 | 0.04% of stock (CD4) | 338 |
| PR0256 | 0.7% of stock (PBMC) | 243 |
| PR0256 | 0.07% of stock (PBMC) | 659 |
| PR0306 | 0.4% of stock (CD4) | 226 (stock=546nM) |
| PR0306 | 0.04% of stock (CD4) | 136 |
| PR0306 | 1 % of stock (PBMC) | 69 |
| PR0306 | 0.5% of stock (PBMC) | 540 |
| PR0306 | 0.17% of stock (PBMC) | 388 |
| PR0306 | 0.06% of stock (PBMC) | 225 |
| PR0306 | 0.02 % of stock (PBMC) | 145 |
| PR0356 | 0.4% of stock (CD4) | 287 (stock=110nM) |
| PR0356 | 0.04% of stock (CD4) | 218 |
| PR0356 | 1% of stock (PBMC) | 69 |
| PR0356 | 0.7% of stock (PBMC) | 46 |
| PR0356 | 0.07% of stock (PBMC) | 205 |
| PR0356 | 0.5% of stock (PBMC) | 196 |
| PR0356 | 0.17% of stock (PBMC) | 186 |
| PR0356 | 0.06% of stock (PBMC) | 327 |
| PR0356 | 0.02 % of stock (PBMC) | 225 |
| PR0364 | 0.4% of stock (CD4) | 141 (stock=0.25mg/ml) |
| PR0364 | 0.04% of stock (CD4) | 117 |
| PR0364 | 0.5% of stock (PBMC) | 201 |

Table 8 (continued)

| PRO | PRO Concentration | Percent Increase Over Control |
|---|---|---|
| PR0364 | 0.17% of stock (PBMC) | 142 |
| PR0364 | 0.06% of stock (PBMC) | 99 |
| PR0364 | 0.02% of stock (PBMC) | 296 |
| PR0826 | 0.4% of stock (CD4) | 268 (stock=903nM) |
| PR0826 | 0.04% of stock (CD4) | 171 |
| PR0826 | 0.5% of stock (PMBC) | 489 |
| PR0826 | 0.17 % of stock (PMBC) | 260 |
| PR0826 | 0.06% of stock (PMBC) | 307 |
| PR0826 | 0.02% of stock (PMBC) | 338 |
| PRO1068 | 0.4% of stock (CD4) | 392 (stock = 1672 nM) |
| PRO1068 | 0.04% of stock (CD4) | 197 |
| PRO1068 | 0.5 % of stock (PMBC) | 673 |
| PRO1068 | 0.17% of stock (PMBC) | 260 |
| PRO1068 | 0.06 % of stock (PMBC) | 429 |
| PRO1068 | 0.02% of stock (PMBC) | 192 |
| PRO1343 | 0.4% of stock (CD4) | 364 (stock=0.23mg/ml) |
| PRO1343 | 0.04% of stock (CD4) | 129 |
| PRO1343 | 0.5 % of stock (PMBC) | 470 |
| PRO1343 | 0.17 % of stock (PMBC) | 277 |
| PRO1343 | 0.06% of stock (PMBC) | 193 |
| PRO1343 | 0.02 % of stock (PMBC) | 230 |
| PRO1375 | 0.4% of stock (CD4) | 255 (stock=0.0059mg/ml) |
| PRO1375 | 0.04% of stock (CD4) | 156 |
| PRO1375 | 0.5 % of stock (PMBC) | 578 |
| PRO1375 | 0.17% of stock (PMBC) | 397 |
| PRO1375 | 0.06% of stock (PMBC) | 422 |
| PRO1375 | 0.02% of stock (PMBC) | 377 |

EXAMPLE 5: Inhibitory Activity in Mixed Lymphocyte Reaction (MLR) Assay (Assay 67)

This example shows that one or more of the polypeptides of the invention are active as inhibitors of the proliferation of stimulated T-lymphocytes. Compounds which inhibit proliferation of lymphocytes are useful therapeutically where suppression of an immune response is beneficial.

The basic protocol for this assay is described in Current Protocols in Immunology, unit 3.12; edited by JE Coligan, AM Kruisbeek, DH Marglies, EM Shevach, W Strober, National Insitutes of Health, Published by John Wiley & Sons, Inc.

More specifically, in one assay variant, peripheral blood mononuclear cells (PBMC) are isolated from mammalian individuals, for example a human volunteer, by leukopheresis (one donor will supply stimulator PBMCs, the other donor will supply responder PBMCs). If desired, the cells are frozen in fetal bovine serum and DMSO after isolation. Frozen cells may be thawed overnight in assay media (37°C, 5 % $CO_2$) and then washed and resuspended to $3x10^6$ cells/ml of assay media (RPMI; 10% fetal bovine serum, 1 % penicillin/streptomycin, I % glutamine, 1% HEPES, 1% non-essential amino acids, 1% pyruvate). The stimulator PBMCs are prepared by irradiating the cells (about 3000 Rads).

The assay is prepared by plating in triplicate wells a mixture of:

100:1 of test sample diluted to 1% or to 0.1%,
50 :1 of irradiated stimulator cells, and
50 :1 of responder PBMC cells.

100 microliters of cell culture media or 100 microliter of CD4-IgG is used as the control. The wells are then incubated at 37°C, 5% $CO_2$ for 4 days. On day 5, each well is pulsed with tritiated thymidine (1.0 mC/well; Amersham). After 6 hours the cells are washed 3 times and then the uptake of the label is evaluated.

In another variant of this assay, PBMCs are isolated from the spleens of Balb/c mice and C57B6 mice. The cells are teased from freshly harvested spleens in assay media (RPMI; 10 % fetal bovine serum, 1% penicillin/streptomycin, 1% glutamine, 1% HEPES, 1% non-essential amino acids, 1% pyruvate) and the PBMCs are isolated by overlaying these cells over Lympholyte M (Organon Teknika), centrifuging at 2000 rpm for 20 minutes, collecting and washing the mononuclear cell layer in assay media and resuspending the cells to $1x10^7$ cells/ml of assay media. The assay is then conducted as described above.

Any decreases below control is considered to be a positive result for an inhibitory compound, with decreases of less than or equal to 80% being preferred. However, any value less than control indicates an inhibitory effect for the test protein.

The following PRO polypeptides tested positive in this assay: PRO182.

EXAMPLE 6: *In situ* Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis and aid in chromosome mapping.

*In situ* hybridization was performed following an optimized version of the protocol by Lu and Gillett, Cell Vision 1:169-176 (1994), using PCR-generated $^{33}$P-labeled riboprobes. Briefly, formalin-fixed, paraffin-embedded human tissues were sectioned, deparaffinized, deproteinated in proteinase K (20 g/ml) for 15 minutes at 37°C, and further processed for *in situ* hybridization as described by Lu and Gillett, *supra*. A [$^{33}$-P] UTP-labeled antisense riboprobe was generated from a PCR product and hybridized at 55°C overnight. The slides were dipped in Kodak NTB2 nuclear track emulsion and exposed for 4 weeks.

## $^{33}$P-Riboprobe synthesis

6.0 µl (125 mCi) of $^{33}$P-UTP (Amersham BF 1002, SA < 2000 Ci/mmol) were speed vac dried. To each tube containing dried $^{33}$P-UTP, the following ingredients were added:
2.0 µl 5x transcription buffer
1.0 µl DTT (100 mM)
2.0 µl NTP mix (2.5 mM : 10 µ; each of 10 mM GTP, CTP & ATP + 10 µl $H_2O$)
1.0 µl UTP (50 µM)
1.0 µl Rnasin
1.0 µl DNA template (1µg)
1.0 µl $H_2O$
1.0 µl RNA polymerase (for PCR products T3 = AS, T7 = S, usually)

The tubes were incubated at 37°C for one hour. 1.0 µl RQ1 DNase were added, followed by incubation at 37°C for 15 minutes. 90 µl TE (10 mM Tris pH 7.6/1mM EDTA pH 8.0) were added, and the mixture was pipetted onto DE81 paper. The remaining solution was loaded in a Microcon-50 ultrafiltration unit, and spun using program 10 (6 minutes). The filtration unit was inverted over a second tube and spun using program 2 (3 minutes). After the final recovery spin, 100 µl TE were added. 1 µl of the final product was pipetted on DE81 paper and counted in 6 ml of Biofluor II.

The probe was run on a TBE/urea gel. 1-3 µl of the probe or 5 µl of RNA Mrk III were added to 3 µl of loading buffer. After heating on a 95 °C heat block for three minutes, the gel was immediately placed on ice. The wells of gel were flushed, the sample loaded, and run at 180-250 volts for 45 minutes. The gel was wrapped in saran wrap and exposed to XAR film with an intensifying screen in -70°C freezer one hour to overnight.

## $^{33}$P-Hybridization

A. Pretreatment of frozen sections

The slides were removed from the freezer, placed on aluminium trays and thawed at room temperature for 5 minutes. The trays were placed in 55 °C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ $H_2O$). After deproteination in 0.5 µg/ml proteinase K for 10 minutes at 37°C (12.5 µl of 10 mg/ml stock in 250 ml prewarmed RNase-free RNAse buffer), the sections were washed in 0.5 x SSC for 10 minutes at room temperature. The sections were dehydrated in 70%, 95%, 100 % ethanol, 2 minutes each.

B. Pretreatment of paraffin-embedded sections

The slides were deparaffinized, placed in SQ $H_2O$, and rinsed twice in 2 x SSC at room temperature, for 5 minutes each time. The sections were deproteinated in 20 µg/ml proteinase K (500 µl of 10 mg/ml in 250 ml RNase-free RNase buffer; 37°C, 15 minutes) - human embryo, or 8 x proteinase K (100 µl in 250 ml Rnase buffer, 37°C, 30 minutes) - formalin tissues. Subsequent rinsing in 0.5 x SSC and dehydration were performed as described above.

C. Prehybridization

The slides were laid out in a plastic box lined with Box buffer (4 x SSC, 50% formamide) - saturated filter paper. The tissue was covered with 50 µl of hybridization buffer (3.75g Dextran Sulfate + 6 ml SQ $H_2O$), vortexed and heated in the microwave for 2 minutes with the cap loosened. After cooling on ice, 18.75 ml formamide, 3.75 ml 20 x SSC and 9 ml SQ $H_2O$ were added, the tissue was vortexed well. and incubated at 42°C for 1-4 hours.

D. Hybridization

1.0 x $10^6$ cpm probe and 1.0 µl tRNA (50 mg/ml stock) per slide were heated at 95 °C for 3 minutes. The slides were cooled on ice, and 48 µl hybridization buffer were added per slide. After vortexing, 50 µl $^{33}P$ mix were added to 50 µl prehybridization on slide. The slides were incubated overnight at 55°C.

E. Washes

Washing was done 2 x 10 minutes with 2xSSC, EDTA at room temperature (400 ml 20 x SSC + 16 ml 0.25M EDTA, $V_f$=4L), followed by RNaseA treatment at 37 °C for 30 minutes (500 µl of 10 mg/ml in 250 ml Rnase buffer = 20 µg/ml), The slides were washed 2 x 10 minutes with 2 x SSC, EDTA at room temperature. The stringency wash conditions were as follows: 2 hours at 55°C, 0.1 x SSC, EDTA (20 ml 20 x SSC + 16 ml EDTA, $V_f$=4L).

F. Results

DNA19355-1150

Expression in human fetal tissues. Adrenal - Specific positive signal over population of endothelial cells in the fetal adrenal.
Oligonucleotides used:

5'-TCTAATACGACTCACTATAGCTCAGGGGAAGAGCCAAAGA-3' (SEQ ID NO:59)

5'-TGAATTAACCCTCACTAAAGCAGTGCAATGCAGGGGACTA-3' (SEQ ID NO:60)

DNA47365-1206

Expression in human and fetal tissues. In the fetus, there is expression in the fascia lining the anterior surface of the vertebral body. There is expression over the fetal retina and low level expression over fetal neurones.
Oligonucleotides used:

5'-GGATTCTAATACGACTCACTATAGGGCAACCCGAGCATGGCACAGCAC-3' (SEQ ID NO:61)

5'-CTATGAAATTAACCCTCACTAAAGGGATCTCCCAGCCGCCCCTTCTC-3' (SEQ ID NO:62)

DNA34387-1138

Expression in human adult and fetal tissues. Generalized low level expression. Elevated signal was observed at the following sites: Fetal - thyroid epithelium, small intestinal epithelium, gonad, pancreatic epithelium, hepatocytes in liver and renal tubules. Expression also seen in vascular tissue in developing long bones. Adult - Moderate signal in placental cytotrophoblast, renal tubular epithelium, bladder epithelium, parathyroid and epithelial tumors.
Oligonucleotides used:

5'-GGATTCTAATACGACTCACTATAGGGCCCGAGATATGCACCCAATGTC-3' (SEQ ID NO:63)

5'-CTATGAAATTAACCCTCACTAAAGGGATCCCAGAATCCCGAAGAACA-3' (SEQ ID NO:64)

EXAMPLE 7: *In situ* Hybridization in Cells and Diseased Tissues

The *in situ* hybridization method of Example 6 is used to determine gene expression. analyze the tissue distribution of transcription, and follow changes in specific mRNA synthesis for the genes/DNAs and the proteins of the invention in diseased tissues isolated from human individuals suffering from a specific disease. These results show more specifically where in diseased tissues the genes of the invention are expressed and are more predictive of the particular localization of the therapeutic effect of the inhibitory or stimulatory compounds of the invention (and agonists or antagonists thereof) in a disease. Hybridization is performed according to the method of Example 6 using one or more of the following tissue and cell samples:

(a) lymphocytes and antigen presenting cells (dendritic cells, langherhans cells, macrophages and monocytes, NK cells);
(b) lymphoid tissues: normal and reactive lymph node, thymus, Bronchial Associated Lymphoid Tissues, (BALT), Mucosal Associated Lymphoid Tissues (MALT);
(c) human disease tissues: Synovium and joint of patients with arthritis and degenerative joint disease, colon from patients with inflammatory bowel disease including ulcerative colitis and Crohns' disease, skin lesions from psoriasis and other forms of dermatitis, lung tissue including BALT and tissue lymph nodes from chronic and acute bronchitis, pneumonia, pneumonitis, pleuritis, lung tissue including BALT and tissue lymph nodes from asthma, nasal and sinus tissue from patients with rhinitis or sinusitis, brain and spinal cord from multiple sclerosis, alzheimer's disease and stroke, kidney from nephritis, glomerulonephritis and systemic lupus erythematosis, liver from infectious and non-infectious hepatitis and tissues from neoplasms/cancer.

Expression is observed in one or more cell or tissue samples indicating localization of the therapeutic effect of the compounds of the invention (and agonists or antagonists thereof) in the disease associated with the cell or tissue sample.

DNA34387-1138

Expression was observed in lung cancer. The gene was amplified in Taqman analysis of a lung tumor panel. Expression was observed in eight squamous carcinomas and in 6/8 adenocarcinomas. Expression was seen in *in situ* and infiltrating components. Expression levels were low to moderate in the adenocarcinomas. In general expression was higher in the squamous carcinomas and in two the expression was strong. Possible low level expression in lymph nodes.
Oligonucleotides used:

5'-GGATTCTAATACGACTCACTATAGGGCCCGAGATATGCACCCAATGTC-3' (SEQ ID NO:65)

5'-CTATGAAATTAACCCTCACTAAAGGGATCCCAGAATCCCGAAGAACA-3' (SEQ ID NO:66)

EXAMPLE 8: Use of PRO as a hybridization probe

The following method describes use of a nucleotide sequence encoding PRO as a hybridization probe.

DNA comprising the coding sequence of full-length or mature PRO as disclosed herein is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high stringency conditions. Hybridization of radiolabeled PRO-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence PRO can then be identified using standard techniques known in the art.

EXAMPLE 9: Expression of PRO in *E. coli*

This example illustrates preparation of an unglycosylated form of PRO by recombinant expression in *E. coli.*

The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli*; see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO coding region, lambda transcriptional terminator, and an argU gene.

The ligation mixture is then used to transform a selected *E. coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in *E. coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) lon galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g $(NH_4)_2SO_4$, 0.71 g sodium citrate·2H2O, 1.07 g KCl, 5.36 g Difco yeast extract, 5.36 g Sheffield hycase SF in 500 mL water, as well as 110 mM MPOS, pH 7.3, 0.55 % (w/v) glucose and 7 mM $MgSO_4$) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4 °C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1 % TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Many of the PRO polypeptides disclosed herein were successfully expressed as described above.

EXAMPLE 10: Expression of PRO in mammalian cells

This example illustrates preparation of a potentially glycosylated form of PRO by recombinant expression in mammalian cells.

The vector, pRK5 (see EP 307,247, published March 15, 1989), is employed as the expression vector. Optionally, the PRO DNA is ligated into pRK5 with selected restriction enzymes to allow insertion of the PRO DNA using ligation methods such as described in Sambrook et al., supra. The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 μg pRK5-PRO DNA is mixed with about 1 μg DNA encoding the VA RNA gene [Thimmappaya et al., Cell, 31:543 (1982)] and dissolved in 500 μl of 1 mM Tris-HCl, 0.1 mM EDTA, 0.227 M $CaCl_2$. To this mixture is added, dropwise, 500 μl of 50 mM HEPES (pH 7.35), 280 mM NaCl, 1.5 mM $NaPO_4$, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 μCi/ml $^{35}$S-cysteine and 200 μCi/ml $^{35}$S-methionine. After a 12 hour incubation, the conditioned medium is collected, concentrated on a spin filter, and loaded onto a 15% SDS gel. The processed gel may be dried and exposed to film for a selected period of time to reveal the presence of PRO polypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 μg pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 μg/ml bovine insulin and 0.1 μg/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as $CaPO_4^-$ or DEAE-dextran. As described above, the cell cultures can be incubated, and the medium replaced with culture medium (alone) or medium containing a radiolabel such as $^{35}$S-methionine. After determining the presence of PRO polypeptide, the culture medium may be replaced with serum free medium. Preferably, the cultures are incubated for about 6 days, and then the conditioned medium is harvested. The medium containing the expressed PRO can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-his tag into a Baculovirus expression vector. The poly-his tagged PRO insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO can then be concentrated and purified by any selected method, such as by $Ni^{2+}$-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgGl constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect" (Quiagen), Dosper" or Fugene" (Boehringer Mannheim). The cells are grown as described in Lucas et al., supra. Approximately 3 x $10^{-7}$ cells are frozen in an ampule for further growth and production as described below.

The ampules containing the plasmid DNA are thawed by placement into water bath and mixed by vortexing. The contents are pipetted into a centrifuge tube containing 10 mLs of media and centrifuged at 1000 rpm for 5 minutes. The supernatant is aspirated and the cells are resuspended in 10 mL of selective media (0.2 μm filtered PS20 with 5% 0.2 μm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x $10^5$ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x $10^6$ cells/mL. On day 0, the cell number pH ie determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Coming 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 μm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4 % mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 μL of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as described above.

EXAMPLE 11: Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

EXAMPLE 12: Expression of PRO in Baculovirus-Infected Insect Cells

The following method describes recombinant expression of PRO in Baculovirus-infected insect cells.

The sequence coding for PRO is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO can then be purified, for example, by $Ni^{2+}$-chelate affinity chromatography as follows. Extracts are prepared from recombinant virus-infected Sf9 cells as described by Rupert et al., Nature, 362: 175-179 (1993). Briefly, Sf9 cells are washed, resuspended in sonication buffer (25 mL Hepes, pH 7.9; 12.5 mM $MgCl_2$; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A $Ni^{2+}$-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline $A_{280}$ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching $A_{280}$ baseline again, the column is developed with a 0 to 500 mM Imidazole gradient in the secondary wash buffer. One mL fractions are collected and analyzed by SDS-PAGE and silver staining or Western blot with $Ni^{2+}$-NTA-conjugated to alkaline phosphatase (Qiagen). Fractions containing the eluted $His_{10}$-tagged PRO are pooled and dialyzed against loading buffer.

Alternatively, purification of the IgG tagged (or Fc tagged) PRO can be performed using known chromatography techniques, including for instance, Protein A or protein G column chromatography.

Many of the PRO polypeptides disclosed herein were successfully expressed as described above.

EXAMPLE 13: Preparation of Antibodies that Bind PRO

This example illustrates preparation of monoclonal antibodies which can specifically bind PRO.

Techniques for producing the monoclonal antibodies are known in the art and are described. for instance, in Coding, supra. Immunogens that may be employed include purified PRO, fusion proteins containing PRO, and cells expressing recombinant PRO on the cell surface. Selection of the immunogen can be made by the skilled artisan without undue experimentation.

Mice, such as Balb/c, are immunized with the PRO immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.

After a suitable antibody titer has been detected, the animals "positive" for antibodies can be injected with a final intravenous injection of PRO. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO. Determination of "positive" hybridoma cells secreting the desired monoclonal antibodies against PRO is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

EXAMPLE 14: Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE™ (Pharmacia LKB Biotechnology). The antibody is coupled to the resin, the resin is blocked, and the derivative resin is washed according to the manufacturer's instructions.

Such an immunoaffinity column is utilized in the purification of PRO polypeptide by preparing a fraction from cells containing PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of PRO polypeptide (e.g. , high ionic strength buffers in the presence of detergent). Then, the column is eluted under conditions that disrupt antibody/PRO polypeptide binding (e.g., a low pH buffer such as approximately pH 2-3, or a high concentration of a chaotrope such as urea or thiocyanate ion), and PRO polypeptide is collected.

EXAMPLE 15: Drug Screening

This invention is particularly useful for screening compounds by using PRO polypeptides or binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with an PRO polypeptide or fragment thereof and assaying (I) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, free PRO polypeptide or fragment is separated from that present in bound form, and the amount of free or uncomplexed label is a measure of the ability of the particular agent to bind to PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO polypeptide specifically compete with a test compound for binding to PRO polypeptide or fragments thereof. In this manner, the antibodies can be used to detect the presence of any peptide which shares one or more antigenic determinants with PRO polypeptide.

EXAMPLE 16: Rational Drug Design

The goal of rational drug design is to produce structural analogs of biologically active polypeptide of interest (*i.e.*, a PRO polypeptide) or of small molecules with which they interact, *e.g.*, agonists, antagonists, or inhibitors. Any of these examples can be used to fashion drugs which are more active or stable forms of the PRO polypeptide or which enhance or interfere with the function of the PRO polypeptide *in vivo* (*c.f.*, Hodgson, Bio/Technology, 9: 19-21 (1991)).

In one approach, the three-dimensional structure of the PRO polypeptide, or of an PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by Athauda *et al*., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

Deposit of Material

The following materials have been deposited with the American Type Culture Collection, 10801 University Blvd., Manassas, VA 20110-2209, USA (ATCC).

Table 9

| Material | UNQ | PRO | ATCC # | ATCC Deposit Date |
|---|---|---|---|---|
| DNA16451-1388 | 153 | 179 | 209776 | 4/14/98 |
| DNA19355-1150 | 149 | 175 | 209466 | 11/18/97 |
| DNA27865-1091 | 156 | 182 | 209296 | 9/23/97 |
| DNA33085-1110 | 321 | 366 | 209087 | 5/30/97 |
| DNA34387-1138 | 214 | 240 | 209260 | 9/16/97 |
| DNA35880-1160 | 223 | 256 | 209379 | 9/16/97 |
| DNA39984-1221 | 269 | 306 | 209435 | 11/7/97 |
| DNA47365-1206 | 319 | 364 | 209436 | 11/7/97 |
| DNA47470-1130 | 313 | 356 | 209422 | 10/28/97 |
| DNA57694-1341 | 467 | 826 | 203017 | 6/23/98 |
| DNA59214-1449 | 525 | 1068 | 203046 | 7/1/98 |
| DNA66675-1587 | 698 | 1343 | 203282 | 9/22/98 |
| DNA67004-1614 | 712 | 1375 | 203115 | 8/11/98 |

These deposits was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC 122 and the Commissioner's rules pursuant thereto (including 37 CFR 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

The following numbered paragraphs (paras.) contain statements of broad combinations of the inventive technical features disclosed herein:-

1. A composition comprising a PRO179, PRO175, PRO182, PRO366, PRO240, PRO256, PRO306, PRO364, PRO356, PRO826, PRO1068, PRO1343 or PRO1375 polypeptide, agonist or fragment thereof and a carrier or excipient, useful for:

   (a) increasing infiltration of inflammatory cells into a tissue of a mammal in need thereof,
   (b) stimulating or enhancing an immune response in a mammal in need thereof, or
   (c) increasing the proliferation of T-lymphocytes in a mammal in need thereof in response to an antigen.

2. Use of a PRO179, PRO175, PRO182, PRO366, PRO240, PRO256, PRO306, PRO364, PRO356, PRO826, PRO1068, PRO1343 or PRO1375 polypeptide, agonist or a fragment thereof to prepare a composition useful for:

   (a) increasing infiltration of inflammatory cells into a tissue of a mammal in need thereof,
   (b) stimulating or enhancing an immune response in a mammal in need thereof, or
   (c) increasing the proliferation of T-lymphocytes in a mammal in need thereof in response to an antigen.

3. A composition comprising a PRO179, PRO175, PRO182, PRO366, PRO240, PRO256, PRO306, PRO364, PRO356, PRO826, PRO1068, PRO1343 or PRO1375 polypeptide, antagonist or a fragment thereof and a carrier or excipient, useful for:

   (a) decreasing infiltration of inflammatory cells into a tissue of a mammal in need thereof,
   (b) inhibiting or reducing an immune response in a mammal in need thereof, or
   (c) decreasing the proliferation of T-lymphocytes in a mammal in need thereof in response to an antigen.

4. Use of a PRO179, PRO175, PRO182, PRO366, PRO240, PRO256, PRO306, PRO364, PRO356, PRO826, PRO1068, PRO1343 or PRO1375 polypeptide, antagonist or a fragment thereof to prepare a composition useful for:

   (a) decreasing infiltration of inflammatory cells into a tissue of a mammal in need thereof,
   (b) inhibiting or reducing an immune response in a mammal in need thereof, or
   (c) decreasing the proliferation of T-lymphocytes in a mammal in need thereof in response to an antigen.

5. A method of treating an immune related disorder, such as a T cell mediated disorder, in a mammal in need thereof, comprising administering to the mammal an effective amount of a PRO179, PRO175, PRO182, PRO366, PRO240, PRO256, PRO306, PRO364, PRO356, PRO826, PRO1068, PRO1343 or PRO1375 polypeptide, an agonist antibody thereof, an antagonist antibody thereto, or a fragment thereof.

6. The method of Para. 5, wherein the disorder is selected from systemic lupus erythematosis, rheumatoid arthritis, osteoarthritis, juvenile chronic arthritis, spondyloarthropathies, systemic sclerosis (scleroderma), idiopathic inflammatory myopathies (dermatomyositis, polymyositis), Sjogren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

7. The composition or use of any of the preceding paras., wherein the agonist or antagonist is a monoclonal antibody.

8. The composition or use of any of the preceding paras., wherein the agonist or antagonist is an antibody fragment or a single-chain antibody.

9. The composition or use of Paras. 7 or 8, wherein the antibody has non-human complementarity determining region (CDR) residues and human framework region (FR) residues.

10. A method for determining the presence of a PRO179, PRO175, PRO182, PRO366, PRO240, PRO256, PRO306, PRO364, PRO356, PRO826, PRO1068, PRO1343 or PRO1375 polypeptide, comprising exposing a cell suspected of containing the polypeptide to an anti-PRO179, PRO175, PRO182, PRO366, PRO240, PRO256, PRO306, PRO364, PRO356, PRO826, PRO1068, PRO1343 or PRO1375 antibody and determining binding of the antibody to the cell.

11. A method of diagnosing an immune related disease in a mammal, comprising detecting the level of expression of a gene encoding a PRO179, PRO175, PRO182, PRO366, PRO240, PRO256, PRO306, PRO364, PRO356, PRO826, PRO1068, PRO1343 or PRO1375 polypeptide (a) in a test sample of tissue cells obtained from the mammal, and (b) in a control sample of known normal tissue cells of the same cell type, wherein a higher expression level in the test sample as compared to the control sample indicates the presence of immune related disease in the mammal from which the test tissue cells were obtained.

12. A method of diagnosing an immune related disease in a mammal, comprising (a) contacting an anti-PRO179, PRO175, PRO182, PRO366, PRO240, PRO256, PRO306, PRO364, PRO356, PRO826, PRO1068, PRO1343 or PRO1375 antibody with a test sample of tissue cells obtained from the mammal, and (b) detecting the formation of a complex between the antibody and the polypeptide in the test sample, wherein formation of said complex is indicative of the presence of said immune related disease in said mammal.

13. An immune related disease diagnostic kit comprising an anti-PRO179, PRO175, PRO182, PRO366, PRO240, PRO256, PRO306, PRO364, PRO356, PRO826, PRO1068, PRO1343 or PRO1375 antibody or fragment thereof and a carrier in suitable packaging.

14. The kit of Para. 13 further comprising instructions for using the antibody to detect a PRO179, PRO175, PRO182, PRO366, PRO240, PRO256, PRO306, PRO364, PRO356, PRO826, PRO1068, PRO1343 or PRO1375 polypeptide.

15. An article of manufacture, comprising:

a container;

a label on the container; and
a composition comprising an active agent contained within the container; wherein the composition is effective for inhibiting or reducing an immune response in a mammal, the label on the container indicates that the composition can be used for treating an immune related disease, and the active agent in the composition is an agent inhibiting the expression and/or activity of a PRO179, PRO175, PRO182, PRO366, PRO240, PRO256, PRO306, PRO364, PRO356, PRO826, PRO1068, PRO1343 or PRO1375 polypeptide.

16. The article of manufacture of Para. 15 wherein said active agent is an anti-PRO179, PRO175, PRO182, PRO366, PRO240, PRO256, PRO306, PRO364, PRO356, PRO826, PRO1068, PRO1343 or PRO1375 antibody.

17. A method for identifying a compound capable of inhibiting the expression or activity of a PRO179, PRO175, PRO182, PRO366, PRO240, PRO256, PRO306, PRO364, PRO356, PRO826, PRO1068, PRO1343 or PRO1375 polypeptide, comprising contacting a candidate compound with the polypeptide under conditions and for a time sufficient to allow these two components to interact.

18. The method of Para. 17, wherein the candidate compound or the PRO179, PRO175, PRO182, PRO366, PRO240, PRO256, PRO306, PRO364, PRO356, PRO826, PRO1068, PRO1343 or PRO1375 polypeptide is immobilized on a solid support.

19. The method of Para. 18, wherein the non-immobilized component carries a detectable label.

20. Isolated nucleic acid having at least 80% nucleic acid sequence identity to a nucleotide sequence that encodes an amino acid sequence selected from the group consisting of the amino acid sequence shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24) and Figure 26 (SEQ ID NO:26).

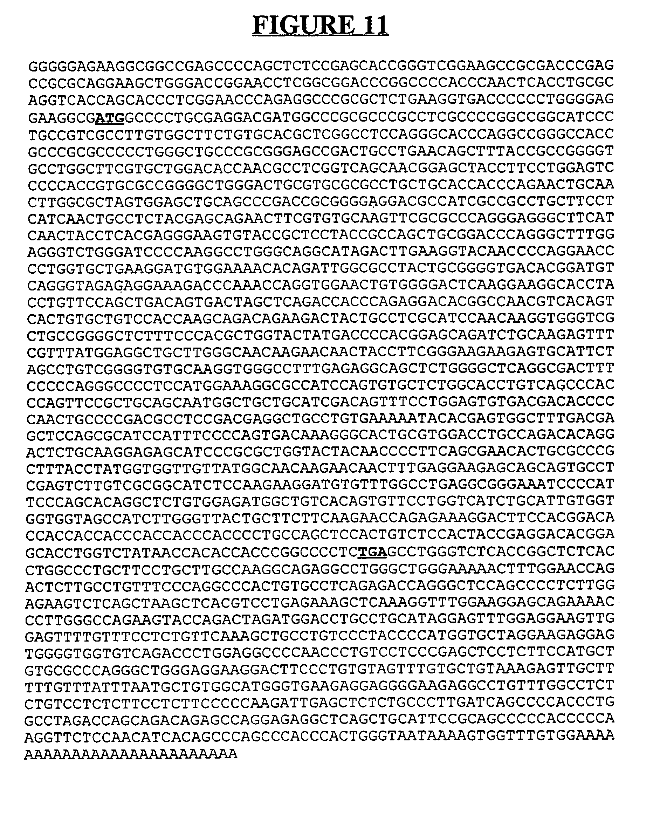

21 Isolated nucleic acid having at least 80% nucleic acid sequence identity to a nucleotide sequence selected from the group consisting of the nucleotide sequence shown in Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23) and Figure 25 (SEQ ID NO:25).

22. Isolated nucleic acid having at least 80% nucleic acid sequence identity to a nucleotide sequence selected from the group consisting of the full-length coding sequence of the nucleotide sequence shown in Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23) and Figure 25 (SEQ ID NO:25).

23. Isolated nucleic acid having at least 80% nucleic acid sequence identity to the full-length coding sequence of the DNA deposited under any of the ATCC accession numbers shown in Table 9.

24. A vector comprising the nucleic acid of any one of Paras. 20 to 23.

25. The vector of Para. 24 operably linked to control sequences recognized by a host cell transformed with the vector.

26. A host cell comprising the vector of Para. 24.

27. The host cell of Para. 26, wherein said cell is a CHO cell.

28. The host cell of Para. 26, wherein said cell is an *E. coli*.

29. The host cell of Para. 26, wherein said cell is a yeast cell.

30. A process for producing a PRO polypeptides comprising culturing the host cell of Para. 26 under conditions suitable for expression of said PRO polypeptide and recovering said PRO polypeptide from the cell culture.

31. An isolated polypeptide having at least 80% amino acid sequence identity to an amino acid sequence selected from the group consisting of the amino acid sequence shown in Figure 2 (SEQ ID NO:2), Figure 4 (SEQ ID NO: 4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24) and Figure 26 (SEQ ID NO:26).

32. An isolated polypeptide scoring at least 80% positives when compared to an amino acid sequence selected from the group consisting of the amino acid sequence shown in Figure 2 (SEQ ID NO:2), Figure 4 (SEQ ID NO: 4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24) and Figure 26 (SEQ ID NO:26).

33. An isolated polypeptide having at least 80% amino acid sequence identity to an amino acid sequence encoded by the full-length coding sequence of the DNA deposited under any of the ATCC accession numbers shown in Table 9.

34. A chimeric molecule comprising a polypeptide according to any one of Paras. 31 to 33 fused to a heterologous amino acid sequence.

35. The chimeric molecule of Para. 34, wherein said heterologous amino acid sequence is an epitope tag sequence.

36. The chimeric molecule of Para. 34, wherein said heterologous amino acid sequence is a Fc region of an immunoglobulin.

37. An antibody which specifically binds to a polypeptide according to any one of Paras. 31 to 33.

38. The antibody of Para. 37, wherein said antibody is a monoclonal antibody, a humanized antibody or a single-chain antibody.

39. Isolated nucleic acid having at least 80% nucleic acid sequence identity to:

(a) a nucleotide sequence encoding the polypeptide shown in Figure 2 (SEQ ID NO:2), Figure 4 (SEQ ID NO: 4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO: 20), Figure 22 (SEQ ID NO:22), Figure 24 (SEQ ID NO:24) or Figure 26 (SEQ ID NO:26), lacking its associated signal peptide;
(b) a nucleotide sequence encoding an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO: 10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24) or Figure 26 (SEQ ID NO:26), with its associated signal peptide; or
(c) a nucleotide sequence encoding an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO: 10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24) or Figure 26 (SEQ ID NO:26), lacking its associated signal peptide.

40. An isolated polypeptide having at least 80% amino acid sequence identity to:

(a) the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24) or Figure 26 (SEQ ID NO:26), lacking its associated signal peptide;
(b) an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO: 20), Figure 22 (SEQ ID NO:22), Figure 24 (SEQ ID NO:24) or Figure 26 (SEQ ID NO:26), with its associated signal peptide; or

(c) an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO: 20), Figure 22 (SEQ ID NO:22), Figure 24 (SEQ ID NO:24) or Figure 26 (SEQ ID NO:26), lacking its associated signal peptide.

41. A method of stimulating the proliferation of T lymphocytes, said method comprising contacting T lymphocytes with a PRO256, PRO306, PRO364, PRO826, PRO1068, PRO1343 or PRO1375 polypeptide in an amount sufficient to stimulate the proliferation of said T lymphocytes.

42. The method according to Para. 41, wherein said PRO256 polypeptide has at least about 85% amino acid sequence identity with the amino acid sequence shown in Figure 12 (SEQ ID NO:12).

43. The method according to Para. 41, wherein said PRO306 polypeptide has at least about 85% amino acid sequence identity with the amino acid sequence shown in Figure 14 (SEQ ID NO:14).

44. The method according to Para. 41, wherein said PRO364 polypeptide has at least about 85% amino acid sequence identity with the amino acid sequence shown in Figure 16 (SEQ ID NO:16).

45. The method according to Para. 41, wherein said PRO826 polypeptide has at least about 85% amino acid sequence identity with the amino acid sequence shown in Figure 20 (SEQ ID NO:20).

46. The method according to Para. 41, wherein said PRO1068 polypeptide has at least about 85% amino acid sequence identity with the amino acid sequence shown in Figure 22 (SEQ ID NO:22).

47. The method according to Para. 41, wherein said PRO1343 polypeptide has at least about 85% amino acid sequence identity with the amino acid sequence shown in Figure 24 (SEQ ID NO:24).

48. The method according to Para. 41, wherein said PRO1375 polypeptide has at least about 85% amino acid sequence identity with the amino acid sequence shown in Figure 26 (SEQ ID NO:26).

49. A method of stimulating an immune response in a mammal, said method comprising administering to said mammal a PRO175, PRO179, PRO240, PRO366 or PRO1375 polypeptide in an amount sufficient to stimulate said immune response.

50. The method according to Para. 49, wherein said PRO175 polypeptide has at least about 85% amino acid sequence identity with the amino acid sequence shown in Figure 4 (SEQ ID NO:4).

51. The method according to Para. 49, wherein said PRO179 polypeptide has at least about 85% amino acid sequence identity with the amino acid sequence shown in Figure 2 (SEQ ID NO:2).

52. The method according to Para. 49, wherein said PRO240 polypeptide has at least about 85% amino acid sequence identity with the amino acid sequence shown in Figure 10 (SEQ ID NO:10).

53. The method according to Para. 49, wherein said PRO366 polypeptide has at least about 85% amino acid sequence identity with the amino acid sequence shown in Figure 8 (SEQ ID NO:8).

54. The method according to Para. 49, wherein said PRO1375 polypeptide has at least about 85% amino acid sequence identity with the amino acid sequence shown in Figure 26 (SEQ ID NO:26).

## Claims

**1.** A composition comprising a PRO306 polypeptide, agonist or fragment thereof and a carrier or excipient, useful for:

(a) increasing infiltration of inflammatory cells into a tissue of a mammal in need thereof,
(b) stimulating or enhancing an immune response in a mammal in need thereof, or
(c) increasing the proliferation of T-lymphocytes in a mammal in need thereof in response to an antigen.

**2.** Use of a PRO306 polypeptide, agonist or a fragment thereof to prepare a composition useful for:

(a) increasing infiltration of inflammatory cells into a tissue of a mammal in need thereof,
(b) stimulating or enhancing an immune response in a mammal in need thereof, or
(c) increasing the proliferation of T-lymphocytes in a mammal in need thereof in response to an antigen.

**3.** A composition comprising a PRO306 polypeptide, antagonist or a fragment thereof and a carrier or excipient, useful for:

(a) decreasing infiltration of inflammatory cells into a tissue of a mammal in need thereof,
(b) inhibiting or reducing an immune response in a mammal in need thereof, or
(c) decreasing the proliferation of T-lymphocytes in a mammal in need thereof in response to an antigen.

**4.** Use of a PRO306 or PRO1375 polypeptide, antagonist or a fragment thereof to prepare a composition useful for:

(a) decreasing infiltration of inflammatory cells into a tissue of a mammal in need thereof,
(b) inhibiting or reducing an immune response in a mammal in need thereof, or
(c) decreasing the proliferation of T-lymphocytes in a mammal in need thereof in response to an antigen.

**5.** A method of treating an immune related disorder, such as a T cell mediated disorder, in a mammal in need thereof, comprising administering to the mammal an effective amount of a PRO306 polypeptide, an agonist antibody thereof, an antagonist antibody thereto, or a fragment thereof.

**6.** The method of Claim 5, wherein the disorder is selected from systemic lupus erythematosis, rheumatoid arthritis, osteoarthritis, juvenile chronic arthritis, spondyloarthropathies, systemic sclerosis (scleroderma), idiopathic inflammatory myopathies (dermatomyositis, polymyositis), Sjogren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

**7.** The composition or use of any of the preceding claims, wherein the agonist or antagonist is a monoclonal antibody.

**8.** The composition or use of any of the preceding claims, wherein the agonist or antagonist is an antibody fragment or a single-chain antibody.

**9.** The composition or use of Claims 7 or 8, wherein the antibody has non-human complementarity determining region (CDR) residues and human framework region (FR) residues.

**10.** A method for determining the presence of a PRO306 polypeptide, comprising exposing a cell suspected of containing the polypeptide to an anti-PRO306 antibody and determining binding of the antibody to the cell.

**11.** A method of diagnosing an immune related disease in a mammal, comprising detecting the level of expression of a gene encoding a PRO306 polypeptide (a) in a test sample of tissue cells obtained from the mammal, and (b) in a control sample of known normal tissue cells of the same cell type, wherein a higher expression level in the test sample as compared to the control sample indicates the presence of immune related disease in the mammal from which the test tissue cells were obtained.

**12.** A method of diagnosing an immune related disease in a mammal, comprising (a) contacting an anti-PRO306 antibody with a test sample of tissue cells obtained from the mammal, and (b) detecting the formation of a complex between the antibody and the polypeptide in the test sample, wherein formation of said complex is indicative of the presence of said immune related disease in said mammal.

**13.** An immune related disease diagnostic kit comprising an anti-PRO306 antibody or fragment thereof and a carrier in suitable packaging.

**14.** The kit of Claim 13 further comprising instructions for using the antibody to detect a PRO306 polypeptide.

**15.** An article of manufacture, comprising:

a container;
a label on the container; and
a composition comprising an active agent contained within the container; wherein the composition is effective for inhibiting or reducing an immune response in a mammal, the label on the container indicates that the composition can be used for treating an immune related disease, and the active agent in the composition is an agent inhibiting the expression and/or activity of a PRO306 polypeptide.

**16.** The article of manufacture of Claim 15 wherein said active agent is an anti-PRO306 antibody.

**17.** A method for identifying a compound capable of inhibiting the expression or activity of a PRO306 polypeptide, comprising contacting a candidate compound with the polypeptide under conditions and for a time sufficient to allow these two components to interact.

**18.** The method of Claim 17, wherein the candidate compound or the PRO306 polypeptide is immobilized on a solid support.

**19.** The method of Claim 18, wherein the non-immobilized component carries a detectable label.

**20.** Isolated nucleic acid having at least 80% nucleic acid sequence identity to a nucleotide sequence that encodes the amino acid sequence shown in Figure 14 (SEQ ID NO:14).

**21.** Isolated nucleic acid having at least 80% nucleic acid sequence identity to the nucleotide sequence shown in Figure 13 (SEQ ID NO:13).

**22.** Isolated nucleic acid having at least 80% nucleic acid sequence identity to the full-length coding sequence of the nucleotide sequence shown in Figure 13 (SEQ ID NO:13).

**23.** Isolated nucleic acid having at least 80% nucleic acid sequence identity to the full-length coding sequence of the DNA deposited under ATCC accession number 209435.

**24.** A vector comprising the nucleic acid of any one of Claims 20 to 23.

**25.** The vector of Claim 24 operably linked to control sequences recognized by a host cell transformed with the vector.

**26.** A host cell comprising the vector of Claim 24.

**27.** The host cell of Claim 26, wherein said cell is a CHO cell.

**28.** The host cell of Claim 26, wherein said cell is an *E. coli*.

**29.** The host cell of Claim 26, wherein said cell is a yeast cell.

**30.** A process for producing a PRO polypeptides comprising culturing the host cell of Claim 26 under conditions suitable for expression of said PRO polypeptide and recovering said PRO polypeptide from the cell culture.

**31.** An isolated polypeptide having at least 80% amino acid sequence identity to the amino acid sequence shown in Figure 14 (SEQ ID NO:14).

**32.** An isolated polypeptide scoring at least 80% positives when compared to the amino acid sequence shown in Figure 14 (SEQ ID NO:14).

**33.** An isolated polypeptide having at least 80% amino acid sequence identity to the amino acid sequence encoded by the full-length coding sequence of the DNA deposited under ATCC accession number 209435.

**34.** A chimeric molecule comprising a polypeptide according to any one of Claims 31 to 33 fused to a heterologous amino acid sequence.

**35.** The chimeric molecule of Claim 34, wherein said heterologous amino acid sequence is an epitope tag sequence.

**36.** The chimeric molecule of Claim 34, wherein said heterologous amino acid sequence is a Fc region of an immunoglobulin.

**37.** An antibody which specifically binds to a polypeptide according to any one of Claims 31 to 33.

**38.** The antibody of Claim 37, wherein said antibody is a monoclonal antibody, a humanized antibody or a single-chain antibody.

**39.** Isolated nucleic acid having at least 80% nucleic acid sequence identity to:

(a) a nucleotide sequence encoding the polypeptide shown in Figure 14 (SEQ ID NO:14), lacking its associated signal peptide;
(b) a nucleotide sequence encoding an extracellular domain of the polypeptide shown in Figure 14 (SEQ ID NO:14), with its associated signal peptide; or
(c) a nucleotide sequence encoding an extracellular domain of the polypeptide shown in Figure 14 (SEQ ID NO:14), lacking its associated signal peptide.

**40.** An isolated polypeptide having at least 80% amino acid sequence identity to:

(a) the polypeptide shown in Figure 14 (SEQ ID NO:14), lacking its associated signal peptide;
(b) an extracellular domain of the polypeptide shown in Figure 14 (SEQ ID NO:14), with its associated signal peptide; or
(c) an extracellular domain of the polypeptide shown in Figure 14 (SEQ ID NO:14), lacking its associated signal peptide.

**41.** A method of stimulating the proliferation of T lymphocytes, said method comprising contacting T lymphocytes with a PRO306 polypeptide in an amount sufficient to stimulate the proliferation of said T lymphocytes.

**42.** The method according to Claim 41, wherein said PRO306 polypeptide has at least about 85% amino acid sequence identity with the amino acid sequence shown in Figure 14 (SEQ ID NO:14).

# FIGURE 1

GCGGACGCGTGGGTGAAATTGAAAATCAAGATAAAA**ATG**TTCACAATTAAGCTCCTTCTT
TTTATTGTTCCTCTAGTTATTTCCTCCAGAATTGATCAAGACAATTCATCATTTGATTCT
CTATCTCCAGAGCCAAAATCAAGATTTGCTATGTTAGACGATGTAAAAATTTTAGCCAAT
GGCCTCCTTCAGTTGGGACATGGTCTTAAAGACTTTGTCCATAAGACGAAGGGCCAAATT
AATGACATATTTCAAAAACTCAACATATTTGATCAGTCTTTTTATGATCTATCGCTGCAA
ACCAGTGAAATCAAAGAAGAAGAAAAGGAACTGAGAAGAACTACATATAAACTACAAGTC
AAAAATGAAGAGGTAAAGAATATGTCACTTGAACTCAACTCAAAACTTGAAAGCCTCCTA
GAAGAAAAAATTCTACTTCAACAAAAAGTGAAATATTTAGAAGAGCAACTAACTAACTTA
ATTCAAAATCAACCTGAAACTCCAGAACACCCAGAAGTAACTTCACTTAAAACTTTTGTA
GAAAAACAAGATAATAGCATCAAAGACCTTCTCCAGACCGTGGAAGACCAATATAAACAA
TTAAACCAACAGCATAGTCAAATAAAAGAAATAGAAAATCAGCTCAGAAGGACTAGTATT
CAAGAACCCACAGAAATTTCTCTATCTTCCAAGCCAAGAGCACCAAGAACTACTCCCTTT
CTTCAGTTGAATGAAATAAGAAATGTAAAACATGATGGCATTCCTGCTGAATGTACCACC
ATTTATAACAGAGGTGAACATACAAGTGGCATGTATGCCATCAGACCCAGCAACTCTCAA
GTTTTTCATGTCTACTGTGATGTTATATCAGGTAGTCCATGGACATTAATTCAACATCGA
ATAGATGGATCACAAAACTTCAATGAAACGTGGGAGAACTACAAATATGGTTTTGGGAGG
CTTGATGGAGAATTTTGGTTGGGCCTAGAGAAGATATACTCCATAGTGAAGCAATCTAAT
TATGTTTTACGAATTGAGTTGGAAGACTGGAAAGACAACAAACATTATATTGAATATTCT
TTTTACTTGGGAAATCACGAAACCAACTATACGCTACATCTAGTTGCGATTACTGGCAAT
GTCCCCAATGCAATCCCGGAAAACAAAGATTTGGTGTTTTCTACTTGGGATCACAAAGCA
AAAGGACACTTCAACTGTCCAGAGGGTTATTCAGGAGGCTGGTGGTGGCATGATGAGTGT
GGAGAAAACAACCTAAATGGTAAATATAACAAACCAAGAGCAAAATCTAAGCCAGAGAGG
AGAAGAGGATTATCTTGGAAGTCTCAAAATGGAAGGTTATACTCTATAAAATCAACCAAA
ATGTTGATCCATCCAACAGATTCAGAAAGCTTTGAA**TGA**ACTGAGGCAATTTAAAGGCAT
ATTTAACCATTAACTCATTCCAAGTTAATGTGGTCTAATAATCTGGTATAAATCCTTAAG
AGAAAGCTTGAGAAATAGATTTTTTTTATCTTAAAGTCACTGTCTATTTAAGATTAAACA
TACAATCACATAACCTTAAAGAATACCGTTTACATTTCTCAATCAAAATTCTTATAATAC
TATTTGTTTTAAATTTTGTGATGTGGGAATCAATTTTAGATGGTCACAATCTAGATTATA
ATCAATAGGTGAACTTATTAAATAACTTTTCTAAATAAAAAATTTAGAGACTTTTATTTT
AAAAGGCATCATATGAGCTAATATCACAACTTTCCCAGTTTAAAAAACTAGTACTCTTGT
TAAAACTCTAAACTTGACTAAATACAGAGGACTGGTAATTGTACAGTTCTTAAATGTTGT
AGTATTAATTTCAAAACTAAAAATCGTCAGCACAGAGTATGTGTAAAAATCTGTAATACA
AATTTTTAAACTGATGCTTCATTTTGCTACAAAATAATTTGGAGTAAATGTTTGATATGA
TTTATTTATGAAACCTAATGAAGCAGAATTAAATACTGTATTAAAATAAGTTCGCTGTCTTT

# FIGURE 2

></usr/seqdb2/sst/DNA/Dnaseqs.min/ss.DNA16451

><subunit 1 of 1, 460 aa, 1 stop

><MW: 53637, pI: 6.61, NX(S/T): 4

MFTIKLLLFIVPLVISSRIDQDNSSFDSLSPEPKSRFAMLDDVKILANGLLQLGHGLKDF
VHKTKGQINDIFQKLNIFDQSFYDLSLQTSEIKEEEKELRRTTYKLQVKNEEVKNMSLEL
NSKLESLLEEKILLQQKVKYLEEQLTNLIQNQPETPEHPEVTSLKTFVEKQDNSIKDLLQ
TVEDQYKQLNQQHSQIKEIENQLRRTSIQEPTEISLSSKPRAPRTTPFLQLNEIRNVKHD
GIPAECTTIYNRGEHTSGMYAIRPSNSQVFHVYCDVISGSPWTLIQHRIDGSQNFNETWE
NYKYGFGRLDGEFWLGLEKIYSIVKQSNYVLRIELEDWKDNKHYIEYSFYLGNHETNYTL
HLVAITGNVPNAIPENKDLVFSTWDHKAKGHFNCPEGYSGGWWWHDECGENNLNGKYNKP
RAKSKPERRRGLSWKSQNGRLYSIKSTKMLIHPTDSESFE

**Important features:**

**Signal peptide:**

amino acids 1-16

**Leucine zipper pattern.**

amino acids 120-142, 127-149

**N-glycosylation sites.**

amino acids 23-27, 115-119, 296-300, 357-363

**cAMP- and cGMP-dependent protein kinase phosphorylation sites.**

amino acids 100-104, 204-208

**Tyrosine kinase phosphorylation site.**

amino acids 342-351

**N-myristoylation sites.**

amino acids 279-285, 352-358, 367-373

**Fibrinogen beta and gamma chains C-terminal domain:**

amino acids 271-310, 312-322, 331-369, 393-424

# FIGURE 3

CAGCTCTCATTTCTCCAAAA**ATG**TGTTTGAGCCACTTGGAAAATATGCCTTTAAGCCATT
CAAGAACTCAAGGAGCTCAGAGATCATCCTGGAAGCTGTGGCTCTTTTGCTCAATAGTTA
TGTTGCTATTTCTTTGCTCCTTCAGTTGGCTAATCTTTATTTTTCTCCAATTAGAGACTG
CTAAGGAGCCCTGTATGGCTAAGTTTGGACCATTACCCTCAAAATGGCAAATGGCATCTT
CTGAACCTCCTTGCGTGAATAAGGTGTCTGACTGGAAGCTGGAGATACTTCAGAATGGCT
TATATTTAATTTATGGCCAAGTGGCTCCCAATGCAAACTACAATGATGTAGCTCCTTTTG
AGGTGCGGCTGTATAAAAACAAAGACATGATACAAACTCTAACAAACAAATCTAAAATCC
AAAATGTAGGAGGGACTTATGAATTGCATGTTGGGGACACCATAGACTTGATATTCAACT
CTGAGCATCAGGTTCTAAAAAATAATACATACTGGGGTATCATTTTACTAGCAAATCCCC
AATTCATCTCC**TAG**AGACTTGATTTGATCTCCTCATTCCCTTCAGCACATGTAGAGGTGC
CAGTGGGTGGATTGGAGGGAGAAGATATTCAATTTCTAGAGTTTGTCTGTCTACAAAAAT
CAACACAAACAGAACTCCTCTGCACGTGAATTTTCATCTATCATGCCTATCTGAAAGAGA
CTCAGGGGAAGAGCCAAAGACTTTTGGTTGGATCTGCAGAAATACTTCATTAATCCATGA
TAAAACAAATATGGATGACAGAGGACATGTGCTTTTCAAAGAATCTTTATCTAATTCTTG
AATTCATGAGTGGAAAAATGGAGTTCTATTCCCATGGAAGATTTACCTGGTATGCAAAAA
GGATCTGGGGCAGTAGCCTGGCTTTGTTCTCATATTCTTGGGCTGCTGTAATTCATTCTT
CTCATACTCCCATCTTCTGAGACCCTCCCAATAAAAAGTAGACTGATAGGATGGCCACAG
ATATGCCTACCATACCCTACTTTAGATATGGTGGTGTTAGAAGATAAAGAACAATCTGAG
AACTATTGGAATAGAGGTACAAGTGGCATAAAATGGAATGTACGCTATCTGGAAATTTCT
CTTGGTTTTATCTTCCTCAGGATGCAGGGTGCTTTAAAAAGCCTTATCAAAGGAGTCATT
CCGAACCCTCACGTAGAGCTTTGTGAGACCTTACTGTTGGTGTGTGTGTCTAAACATTGC
TAATTGTAAAGAAAGAGTAACCATTAGTAATCATTAGGTTTAACCCCAGAATGGTATTAT
CATTACTGGATTATGTCATGTAATGATTTAGTATTTTTAGCTAGCTTTCCACAGTTTGCA
AAGTGCTTTCGTAAAACAGTTAGCAATTCTATGAAGTTAATTGGGCAGGCATTTGGGGGA
AAATTTTAGTGATGAGAATGTGATAGCATAGCATAGCCAACTTTCCTCAACTCATAGGAC
AAGTGACTACAAGAGGCAATGGGTAGTCCCCTGCATTGCACTGTCTCAGCTTTAGAATTG
TTATTTCTGCTATCGTGTTATAAGACTCTAAAACTTAGCGAATTCACTTTTCAGGAAGCA
TATTCCCCTTTAGCCCAAGGTGAGCAGAGTGAAGCTACAACAGATCTTTCCTTTACCAGC
ACACTTTTTTTTTTTTTTCCTGCCTGAATCAGGGAGATCCAGGATGCTGTTCAGGCCAAA
TCCCAACCAAATTCCCCTTTTCACTTTGCAGGGCCCATCTTAGTCAAATGTGCTAACTTC
TAAAATAATAAATAGCACTAATTCAAAATTTTTGGAATCTTAAATTAGCTACTTGCNGGT
TGCTTGTTGAAAGGNATATAATGATTACATTGTAAACAAATTTAAAATATTTATGGATAT
TTGTGAAAAGCTGCATTATGTTAAATAATATTACATGTAAAGCT

# FIGURE 4

></usr/seqdb2/sst/DNA/Dnaseqs.full/ss.DNA19355
><subunit 1 of 1, 177 aa, 1 stop
><MW: 20308, pI: 8.08, NX(S/T): 2

MCLSHLENMPLSHSRTQGAQRSSWKLWLFCSIVMLLFLCSFSWLIFIFLQLETAKEPCMA
KFGPLPSKWQMASSEPPCVNKVSDWKLEILQNGLYLIYGQVAPNANYNDVAPFEVRLYKN
KDMIQTLTNKSKIQNVGGTYELHVGDTIDLIFNSEHQVLKNNTYWGIILLANPQFIS

**Signal sequence:**
amino acids 1-44

**N-glycosylation sites.**
amino acids 129-133, 161-165

**N-myristoylation site.**
amino acids 18-24

# FIGURE 5

TATTTACCATATCAGATTCACATTCAGTCCTCAGCAAA**ATG**AAGGGCTCCATTTTCACTC
TGTTTTTATTCTCTGTCCTATTTGCCATCTCAGAAGTGCGGAGCAAGGAGTCTGTGAGAC
TCTGTGGGCTAGAATACATACGGACAGTCATCTATATCTGTGCTAGCTCCAGGTGGAGAA
GGCATCTGGAGGGGATCCCTCAAGCTCAGCAAGCTGAGACAGGAAACTCCTTCCAGCTCC
CACATAAACGTGAGTTTTCTGAGGAAAATCCAGCGCAAAACCTTCCGAAGGTGGATGCCT
CAGGGGAAGACCGTCTTTGGGGTGGACAGATGCCCACTGAAGAGCTTTGGAAGTCAAAGA
AGCATTCAGTGATGTCAAGACAAGATTTACAAACTTTGTGTTGCACTGATGGCTGTTCCA
TGACTGATTTGAGTGCTCTTTGC**TAA**GACAAGAGCAAATACCCAATGGGTGGCAGAGCTT
TATCACATGTTTAATTACAGTGTTTTACTGCCTGGTAGAACACTAATATTGTGTTATTAA
AATGATGGCTTTTGGGTAGGCAAAACTTCTTTTCTAAAAGGTATAGCTGAGCGGTTGAAA
CCACAGTGATCTCTATTTTCTCCCTTTGCCAAGGTTAATGAACTGTTCTTTTCAAATTCT
ACTAATGCTTTGAAATTTCAAATGCTGCGCAAAATTGCAATAAAAATGCTATAAA

# FIGURE 6

></usr/seqdb2/sst/DNA/Dnaseqs.min/ss.DNA27865
><subunit 1 of 1, 135 aa, 1 stop
><MW: 15319, pI: 7.39, NX(S/T): 0
MKGSIFTLFLFSVLFAISEVRSKESVRLCGLEYIRTVIYICASSRWRRHLEGIPQAQQAE
TGNSFQLPHKREFSEENPAQNLPKVDASGEDRLWGGQMPTEELWKSKKHSVMSRQDLQTL
CCTDGCSMTDLSALC

**Signal sequence:**
amino acids 1-18

**cAMP- and cGMP-dependent protein kinase phosphorylation site.**
amino acids 107-111

**N-myristoylation sites.**
amino acids 3-9, 52-58, 96-102, 125-131

**Insulin family signature.**
amino acids 121-136

# FIGURE 7

GCTGTGGGAACCTCTCCACGCGCACGAACTCAGCCAACGATTTCTGATAGATTTTTGGGA
GTTTGACCAGAGATGCAAGGGGTGAAGGAGCGCTTCCTACCGTTAGGGAACTCTGGGGAC
AGAGCGCCCCGGCCGCCTGATGGCCGAGGCAGGGTGCGACCCAGGACCCAGGACGGCGTC
GGGAACCATACC**ATG**GCCCGGATCCCCAAGACCCTAAAGTTCGTCGTCGTCATCGTCGCG
GTCCTGCTGCCAGTCCTAGCTTACTCTGCCACCACTGCCCGGCAGGAGGAAGTTCCCCAG
CAGACAGTGGCCCCACAGCAACAGAGGCACAGCTTCAAGGGGGAGGAGTGTCCAGCAGGA
TCTCATAGATCAGAACATACTGGAGCCTGTAACCCGTGCACAGAGGGTGTGGATTACACC
AACGCTTCCAACAATGAACCTTCTTGCTTCCCATGTACAGTTTGTAAATCAGATCAAAAA
CATAAAAGTTCCTGCACCATGACCAGAGACACAGTGTGTCAGTGTAAAGAAGGCACCTTC
CGGAATGAAAACTCCCCAGAGATGTGCCGGAAGTGTAGCAGGTGCCCTAGTGGGGAAGTC
CAAGTCAGTAATTGTACGTCCTGGGATGATATCCAGTGTGTTGAAGAATTTGGTGCCAAT
GCCACTGTGGAAACCCCAGCTGCTGAAGAGACAATGAACACCAGCCCGGGGACTCCTGCC
CCAGCTGCTGAAGAGACAATGAACACCAGCCCAGGGACTCCTGCCCCAGCTGCTGAAGAG
ACAATGACCACCAGCCCGGGGACTCCTGCCCCAGCTGCTGAAGAGACAATGACCACCAGC
CCGGGGACTCCTGCCCCAGCTGCTGAAGAGACAATGACCACCAGCCCGGGGACTCCTGCC
TCTTCTCATTACCTCTCATGCACCATCGTAGGGATCATAGTTCTAATTGTGCTTCTGATT
GTGTTTGTT**TGA**AAGACTTCACTGTGGAAGAAATTCCTTCCTTACCTGAAAGGTTCAGGT
AGGCGCTGGCTGAGGGCGGGGGGCGCTGGACACTCTCTGCCCTGCCTCCCTCTGCTGTGT
TCCCACAGACAGAAACGCCTGC

# FIGURE 8

></usr/seqdb2/sst/DNA/Dnaseqs.full/ss.DNA33085
><subunit 1 of 1, 259 aa, 1 stop
><MW: 27395, pI: 4.84, NX(S/T): 5
MARIPKTLKFVVVIVAVLLPVLAYSATTARQEEVPQQTVAPQQQRHSFKGEECPAGSHRS
EHTGACNPCTEGVDYTNASNNEPSCFPCTVCKSDQKHKSSCTMTRDTVCQCKEGTFRNEN
SPEMCRKCSRCPSGEVQVSNCTSWDDIQCVEEFGANATVETPAAEETMNTSPGTPAPAAE
ETMNTSPGTPAPAAEETMTTSPGTPAPAAEETMTTSPGTPAPAAEETMTTSPGTPASSHY
LSCTIVGIIVLIVLLIVFV

**Signal sequence.**
amino acids 1-29

**Transmembrane domain.**
amino acids 240-257

**N-glycosylation sites.**
amino acids 77-81, 140-144, 156-160

**cAMP- and cGMP-dependent protein kinase phosphorylation site.**
amino acids 126-130

**N-myristoylation sites.**
amino acids 56-62, 72-78, 114-120, 154-160, 233-239

**TNFR/NGFR family cysteine-rich region proteins.**
amino acids 85-92

# FIGURE 9

GGGAACGGAAA**ATG**GCGCCTCACGGCCCGGGTAGTCTTACGACCCTGGTGCCCTGGGCTG
CCGCCCTGCTCCTCGCTCTGGGCGTGGAAAGGGCTCTGGCGCTACCCGAGATATGCACCC
AATGTCCAGGGAGCGTGCAAAATTTGTCAAAAGTGGCCTTTTATTGTAAAACGACACGAG
AGCTAATGCTGCATGCCCGTTGCTGCCTGAATCAGAAGGGCACCATCTTGGGGCTGGATC
TCCAGAACTGTTCTCTGGAGGACCCTGGTCCAAACTTTCATCAGGCACATACCACTGTCA
TCATAGACCTGCAAGCAAACCCCCTCAAAGGTGACTTGGCCAACACCTTCCGTGGCTTTA
CTCAGCTCCAGACTCTGATACTGCCACAACATGTCAACTGTCCTGGAGGAATTAATGCCT
GGAATACTATCACCTCTTATATAGACAACCAAATCTGTCAAGGGCAAAAGAACCTTTGCA
ATAACACTGGGGACCCAGAAATGTGTCCTGAGAATGGATCTTGTGTACCTGATGGTCCAG
GTCTTTTGCAGTGTGTTTGTGCTGATGGTTTCCATGGATACAAGTGTATGCGCCAGGGCT
CGTTCTCACTGCTTATGTTCTTCGGGATTCTGGGAGCCACCACTCTATCCGTCTCCATTC
TGCTTTGGGCGACCCAGCGCCGAAAAGCCAAGACTTCA**TGA**ACTACATAGGTCTTACCAT
TGACCTAAGATCAATCTGAACTATCTTAGCCCAGTCAGGGAGCTCTGCTTCCTAGAAAGG
CATCTTTCGCCAGTGGATTCGCCTCAAGGTTGAGGCCGCCATTGGAAGATGAAAAATTGC
ACTCCCTTGGTGTAGACAAATACCAGTTCCCATTGGTGTTGTTGCCTATAATAAACACTT
TTTCTTTTTTNAAAAAAAAAAAAAAAAAAAAA

# FIGURE 10

></usr/seqdb2/sst/DNA/Dnaseqs.full/ss.DNA34387
><subunit 1 of 1, 229 aa, 1 stop
><MW: 24689, pI: 7.83, NX(S/T): 4
MAPHGPGSLTTLVPWAAALLLALGVERALALPEICTQCPGSVQNLSKVAFYCKTTRELML
HARCCLNQKGTILGLDLQNCSLEDPGPNFHQAHTTVIIDLQANPLKGDLANTFRGFTQLQ
TLILPQHVNCPGGINAWNTITSYIDNQICQGQKNLCNNTGDPEMCPENGSCVPDGPGLLQ
CVCADGFHGYKCMRQGSFSLLMFFGILGATTLSVSILLWATQRRKAKTS

**Signal sequence:**
amino acids 1-30

**Transmembrane domain:**
amino acids 198-213

**N-glycosylation site.**
amino acids 44-48, 79-83, 157-161, 168-172

**N-myristoylation sites.**
amino acids 7-13, 24-30, 40-46, 70-76, 132-138, 177-183, 205-211

**EGF-like domain cysteine pattern signature.**
amino acids 181-193

# FIGURE 11

GGGGGAGAAGGCGGCCGAGCCCCAGCTCTCCGAGCACCGGGTCGGAAGCCGCGACCCGAG
CCGCGCAGGAAGCTGGGACCGGAACCTCGGCGGACCCGGCCCCACCCAACTCACCTGCGC
AGGTCACCAGCACCCTCGGAACCCAGAGGCCCGCGCTCTGAAGGTGACCCCCCTGGGGAG
GAAGGCG**ATG**GCCCCTGCGAGGACGATGGCCCGCGCCCGCCTCGCCCCGGCCGGCATCCC
TGCCGTCGCCTTGTGGCTTCTGTGCACGCTCGGCCTCCAGGGCACCCAGGCCGGGCCACC
GCCCGCGCCCCCTGGGCTGCCCGCGGGAGCCGACTGCCTGAACAGCTTTACCGCCGGGGT
GCCTGGCTTCGTGCTGGACACCAACGCCTCGGTCAGCAACGGAGCTACCTTCCTGGAGTC
CCCCACCGTGCGCCGGGGCTGGGACTGCGTGCGCGCCTGCTGCACCACCCAGAACTGCAA
CTTGGCGCTAGTGGAGCTGCAGCCCGACCGCGGGGAGGACGCCATCGCCGCCTGCTTCCT
CATCAACTGCCTCTACGAGCAGAACTTCGTGTGCAAGTTCGCGCCCAGGGAGGGCTTCAT
CAACTACCTCACGAGGGAAGTGTACCGCTCCTACCGCCAGCTGCGGACCCAGGGCTTTGG
AGGGTCTGGGATCCCCAAGGCCTGGGCAGGCATAGACTTGAAGGTACAACCCCAGGAACC
CCTGGTGCTGAAGGATGTGGAAAACACAGATTGGCGCCTACTGCGGGGTGACACGGATGT
CAGGGTAGAGAGGAAAGACCCAAACCAGGTGGAACTGTGGGGACTCAAGGAAGGCACCTA
CCTGTTCCAGCTGACAGTGACTAGCTCAGACCACCCAGAGGACACGGCCAACGTCACAGT
CACTGTGCTGTCCACCAAGCAGACAGAAGACTACTGCCTCGCATCCAACAAGGTGGGTCG
CTGCCGGGGCTCTTTCCCACGCTGGTACTATGACCCCACGGAGCAGATCTGCAAGAGTTT
CGTTTATGGAGGCTGCTTGGGCAACAAGAACAACTACCTTCGGGAAGAAGAGTGCATTCT
AGCCTGTCGGGGTGTGCAAGGTGGGCCTTTGAGAGGCAGCTCTGGGGCTCAGGCGACTTT
CCCCCAGGGCCCCTCCATGGAAAGGCGCCATCCAGTGTGCTCTGGCACCTGTCAGCCCAC
CCAGTTCCGCTGCAGCAATGGCTGCTGCATCGACAGTTTCCTGGAGTGTGACGACACCCC
CAACTGCCCCGACGCCTCCGACGAGGCTGCCTGTGAAAAATACACGAGTGGCTTTGACGA
GCTCCAGCGCATCCATTTCCCCAGTGACAAAGGGCACTGCGTGGACCTGCCAGACACAGG
ACTCTGCAAGGAGAGCATCCCGCGCTGGTACTACAACCCCTTCAGCGAACACTGCGCCCG
CTTTACCTATGGTGGTTGTTATGGCAACAAGAACAACTTTGAGGAAGAGCAGCAGTGCCT
CGAGTCTTGTCGCGGCATCTCCAAGAAGGATGTGTTTGGCCTGAGGCGGGAAATCCCCAT
TCCCAGCACAGGCTCTGTGGAGATGGCTGTCACAGTGTTCCTGGTCATCTGCATTGTGGT
GGTGGTAGCCATCTTGGGTTACTGCTTCTTCAAGAACCAGAGAAAGGACTTCCACGGACA
CCACCACCACCCACCACCCACCCCTGCCAGCTCCACTGTCTCCACTACCGAGGACACGGA
GCACCTGGTCTATAACCACACCACCCGGCCCCTC**TGA**GCCTGGGTCTCACCGGCTCTCAC
CTGGCCCTGCTTCCTGCTTGCCAAGGCAGAGGCCTGGGCTGGGAAAAACTTTGGAACCAG
ACTCTTGCCTGTTTCCCAGGCCCACTGTGCCTCAGAGACCAGGGCTCCAGCCCCTCTTGG
AGAAGTCTCAGCTAAGCTCACGTCCTGAGAAAGCTCAAAGGTTTGGAAGGAGCAGAAAAC
CCTTGGGCCAGAAGTACCAGACTAGATGGACCTGCCTGCATAGGAGTTTGGAGGAAGTTG
GAGTTTTGTTTCCTCTGTTCAAAGCTGCCTGTCCCTACCCCATGGTGCTAGGAAGAGGAG
TGGGGTGGTGTCAGACCCTGGAGGCCCCAACCCTGTCCTCCCGAGCTCCTCTTCCATGCT
GTGCGCCCAGGGCTGGGAGGAAGGACTTCCCTGTGTAGTTTGTGCTGTAAAGAGTTGCTT
TTTGTTTATTTAATGCTGTGGCATGGGTGAAGAGGAGGGGAAGAGGCCTGTTTGGCCTCT
CTGTCCTCTCTTCCTCTTCCCCCAAGATTGAGCTCTCTGCCCTTGATCAGCCCCACCCTG
GCCTAGACCAGCAGACAGAGCCAGGAGAGGCTCAGCTGCATTCCGCAGCCCCCACCCCCA
AGGTTCTCCAACATCACAGCCCAGCCCACCCACTGGGTAATAAAAGTGGTTTGTGGAAAA
AAAAAAAAAAAAAAAAAAAAAA

# FIGURE 12

><MW: 58428.04 pI: 6.22

MAPARTMARARLAPAGIPAVALWLLCTLGLQGTQAGPPPAPPGLPAGADCLNSFTAGVPG
FVLDTNASVSNGATFLESPTVRRGWDCVRACCTTQNCNLALVELQPDRGEDAIAACFLIN
CLYEQNFVCKFAPREGFINYLTREVYRSYRQLRTQGFGGSGIPKAWAGIDLKVQPQEPLV
LKDVENTDWRLLRGDTDVRVERKDPNQVELWGLKEGTYLFQLTVTSSDHPEDTANVTVTV
LSTKQTEDYCLASNKVGRCRGSFPRWYYDPTEQICKSFVYGGCLGNKNNYLREEECILAC
RGVQGGPLRGSSGAQATFPQGPSMERRHPVCSGTCQPTQFRCSNGCCIDSFLECDDTPNC
PDASDEAACEKYTSGFDELQRIHFPSDKGHCVDLPDTGLCKESIPRWYYNPFSEHCARFT
YGGCYGNKNNFEEEQQCLESCRGISKKDVFGLRREIPIPSTGSVEMAVTVFLVICIVVVV
AILGYCFFKNQRKDFHGHHHHPPPTPASSTVSTTEDTEHLVYNHTTRPL

**Signal sequence:**
amino acids 1-35

**Transmembrane domain:**
amino acids 466-483

**N-glycosylation site.**
amino acids 66-70, 235-239, 523-527

**N-myristoylation site.**
amino acids 29-35, 43-49, 161-167, 212-218, 281-287, 282-288, 285-291, 310-316, 313-319, 422-428, 423-429, 426-432

**Cell attachment sequence.**
amino acids 193-196

**Pancreatic trypsin inhibitor (Kunitz) family proteins.**
amino acids 257-300, 398-441

**LDL-receptor class A (LDLRA) domain proteins.**
amino acids 347-366

# FIGURE 13

GTGCTGGGCTTTTTCAGACAAGTGCATCTCCTAACCAGGTCACATTTCAGCCGCGACCCA
CTCTCCGCCAGTCACCGGAGGCAGACCGCGGGAGGAGAGCTGAGGACAGCCGCGTGCGCT
TCGCCAGCAGCGGGGTGGGAGGAAGGACATTAAAATACTGCAGAAGTCAAGACCCCCCCA
GGTCGAACCCAGACCACG<u>**ATG**</u>CGCGCCCCGGGCTGCGGGCGGCTGGTGCTGCCGCTGCTG
CTCCTGGCCGCGGCAGCCCTGGCCGAAGGCGACGCCAAGGGGCTCAAGGAGGGCGAGACC
CCCGGCAATTTCATGGAGGACGAGCAATGGCTGTCGTCCATCTCGCAGTACAGCGGCAAG
ATCAAGCACTGGAACCGCTTCCGAGACGAAGTGGAGGATGACTATATCAAGAGCTGGGAG
GACAATCAGCAAGGAGATGAAGCCCTGGATACCACCAAGGACCCCTGCCAGAAGGTGAAG
TGCAGCCGCCACAAGGTGTGCATTGCCCAGGGCTACCAGCGGGCCATGTGCATCAGTCGC
AAGAAGCTGGAGCACAGGATCAAGCAGCCGACCGTGAAACTCCATGGAAACAAAGACTCC
ATCTGCAAGCCCTGCCACATGGCCCAGCTTGCCTCTGTCTGCGGCTCAGATGGCCACACT
TACAGCTCTGTGTGTAAGCTGGAGCAACAGGCGTGCCTGAGCAGCAAGCAGCTGGCGGTG
CGATGCGAGGGCCCCTGCCCCTGCCCCACGGAGCAGGCTGCCACCTCCACCGCCGATGGC
AAACCAGAGACTTGCACCGGTCAGGACCTGGCTGACCTGGGAGATCGGCTGCGGGACTGG
TTCCAGCTCCTTCATGAGAACTCCAAGCAGAATGGCTCAGCCAGCAGTGTAGCCGGCCCG
GCCAGCGGGCTGGACAAGAGCCTGGGGGCCAGCTGCAAGGACTCCATTGGCTGGATGTTC
TCCAAGCTGGACACCAGTGCTGACCTCTTCCTGGACCAGACGGAGCTGGCCGCCATCAAC
CTGGACAAGTACGAGGTCTGCATCCGTCCCTTCTTCAACTCCTGTGACACCTACAAGGAT
GGCCGGGTCTCTACTGCTGAGTGGTGCTTCTGCTTCTGGAGGGAGAAGCCCCCCTGCCTG
GCAGAGCTGGAGCGCATCCAGATCCAGGAGGCCGCCAAGAAGAAGCCAGGCATCTTCATC
CCGAGCTGCGACGAGGATGGCTACTACCGGAAGATGCAGTGTGACCAGAGCAGCGGTGAC
TGCTGGCGTGTGGACCAGCTGGGCCTGGAGCTGACTGGCACGCGCACGCATGGGAGCCCC
GACTGCGATGACATCGTGGGCTTCTCGGGGGACTTTGGAAGCGGTGTCGGCTGGGAGGAT
GAGGAGGAGAAGGAGACGGAGGAAGCAGGCGAGGAGGCCGAGGAGGAGGAGGGCGAGGCA
GGCGAGGCTGACGACGGGGGCTACATCTGG<u>**TAG**</u>ACGCCCTCAGGAGCCGGCTGCCGGGGG
GGACTCAACAGCAGAGCTCTGAGCAGCAGCAGGCAACTTCGAGAACGGATCCAGAAATGC
AGTCAGAAGGACCCTGCTCCACCTGGGGGGACTGGGAGTGTGAGTGTGCATGGCATGTGT
GTGGCACAGATGGCTGGGACGGGTGACAGTGTGAGTGCATGTGTGCATGCATGTGTGTAT
GTGTGTGTGTGTGTGGCATGCGCTGACAAATGTGTCCTTGATCCACACTGCTCCTGGCAG
AGTGAGTCACCCAAAGGCCCCTTCGGCCTCCTTGTAGCTGTTTTCTTTCCTTTTGTTGTT
GGTTTTAAAATACATTCACACACAAATACAAAAAAAAAAAAAAAAAAAAAAAAAAAAAAA
AAAAAAAAAAAAAAAAAAAAAAAAAAAAAAAAAAAAAA

# FIGURE 14

></usr/seqdb2/sst/DNA/Dnaseqs.min/ss.DNA39984
><subunit 1 of 1, 424 aa, 1 stop
><MW: 46832, pI: 4.76, NX(S/T): 1

MRAPGCGRLVLPLLLLAAAALAEGDAKGLKEGETPGNFMEDEQWLSSISQYSGKIKHWNR
FRDEVEDDYIKSWEDNQQGDEALDTTKDPCQKVKCSRHKVCIAQGYQRAMCISRKKLEHR
IKQPTVKLHGNKDSICKPCHMAQLASVCGSDGHTYSSVCKLEQQACLSSKQLAVRCEGPC
PCPTEQAATSTADGKPETCTGQDLADLGDRLRDWFQLLHENSKQNGSASSVAGPASGLDK
SLGASCKDSIGWMFSKLDTSADLFLDQTELAAINLDKYEVCIRPFFNSCDTYKDGRVSTA
EWCFCFWREKPPCLAELERIQIQEAAKKKPGIFIPSCDEDGYYRKMQCDQSSGDCWRVDQ
LGLELTGTRTHGSPDCDDIVGFSGDFGSGVGWEDEEEKETEEAGEEAEEEEGEAGEADDG
GYIW

**Signal sequence.**
amino acids 1-22
**N-glycosylation site.**
amino acids 225-229
**Glycosaminoglycan attachment site.**
amino acids 388-392
**Tyrosine kinase phosphorylation site.**
amino acids 62-70
**N-myristoylation sites.**
amino acids 28-34, 130-136, 201-207, 226-232, 237-243, 362-368, 372-378, 387-393
**Thyroglobulin type-1 repeat.**
amino acids 335-348
**Kazal serine protease.**
amino acids 140-162
**Osteonectin domain protein.**
amino acids 283-317
**CTF/NF-I proteins.**
amino acids 324-358

# FIGURE 15

CACGCACTTCACCTGGGTCGGGATTCTCAGGTCATGAACGGTCCCAGCCACCTCCGGGCA
GGGCGGGTGAGGACGGGGACGGGGCGTGTCCAACTGGCTGTGGGCTCTTGAAACCCGAGC
**ATG**GCACAGCACGGGGCGATGGGCGCGTTTCGGGCCCTGTGCGGCCTGGCGCTGCTGTGC
GCGCTCAGCCTGGGTCAGCGCCCCACCGGGGGTCCCGGGTGCGGCCCTGGGCGCCTCCTG
CTTGGGACGGGAACGGACGCGCGCTGCTGCCGGGTTCACACGACGCGCTGCTGCCGCGAT
TACCCGGGCGAGGAGTGCTGTTCCGAGTGGGACTGCATGTGTGTCCAGCCTGAATTCCAC
TGCGGAGACCCTTGCTGCACGACCTGCCGGCACCACCCTTGTCCCCCAGGCCAGGGGGTA
CAGTCCCAGGGGAAATTCAGTTTTGGCTTCCAGTGTATCGACTGTGCCTCGGGGACCTTC
TCCGGGGGCCACGAAGGCCACTGCAAACCTTGGACAGACTGCACCCAGTTCGGGTTTCTC
ACTGTGTTCCCTGGGAACAAGACCCACAACGCTGTGTGCGTCCCAGGGTCCCCGCCGGCA
GAGCCGCTTGGGTGGCTGACCGTCGTCCTCCTGGCCGTGGCCGCCTGCGTCCTCCTCCTG
ACCTCGGCCCAGCTTGGACTGCACATCTGGCAGCTGAGGAGTCAGTGCATGTGGCCCCGA
GAGACCCAGCTGCTGCTGGAGGTGCCGCCGTCGACCGAAGACGCCAGAAGCTGCCAGTTC
CCCGAGGAAGAGCGGGGCGAGCGATCGGCAGAGGAGAAGGGGCGGCTGGGAGACCTGTGG
GTG**TGA**GCCTGGCCGTCCTCCGGGGCCACCGACCGCAGCCAGCCCCTCCCCAGGAGCTCC
CCAGGCCGCAGGGGCTCTGCGTTCTGCTCTGGGCCGGGCCCTGCTCCCCTGGCAGCAGAA
GTGGGTGCAGGAAGGTGGCAGTGACCAGCGCCCTGGACCATGCAGTTC

# FIGURE 16

></usr/seqdb2/sst/DNA/Dnaseqs.min/ss.DNA47365
><subunit 1 of 1, 241 aa, 1 stop
><MW: 26000, pI: 6.34, NX(S/T): 1
MAQHGAMGAFRALCGLALLCALSLGQRPTGGPGCGPGRLLLGTGTDARCCRVHTTRCCRD
YPGEECCSEWDCMCVQPEFHCGDPCCTTCRHHPCPPGQGVQSQGKFSFGFQCIDCASGTF
SGGHEGHCKPWTDCTQFGFLTVFPGNKTHNAVCVPGSPPAEPLGWLTVVLLAVAACVLLL
TSAQLGLHIWQLRSQCMWPRETQLLLEVPPSTEDARSCQFPEEERGERSAEEKGRLGDLWV

**Signal sequence.**
amino acids 1-25

**Transmembrane domain.**
amino acids 163-183

**N-glycosylation site.**
amino acids 146-150

**N-myristoylation sites.**
amino acids 5-11, 8-14, 25-31, 30-36, 33-39, 118-124, 122-128, 156-162

**Prokaryotic membrane lipoprotein lipid attachment site.**
amino acids 166-177

**Leucine zipper pattern.**
amino acids 171-193

# FIGURE 17

GAAAGCTATAGGCTACCCATTCAGCTCCCCTGTCAGAGACTCAAGCTTTGAGAAAGGCTA
GCAAAGAGCAAGGAAAGAGAGAAAACAACAAAGTGGCGAGGCCCTCAGAGTGAAAGCGTA
AGGTTCAGTCAGCCTGCTGCAGCTTTGCAGACCTCAGCTGGGCATCTCCAGACTCCCCTG
AAGGAAGAGCCTTCCTCACCCAAACCCACAAAAG**ATG**CTGAAAAAGCCTCTCTCAGCTGT
GACCTGGCTCTGCATTTTCATCGTGGCCTTTGTCAGCCACCCAGCGTGGCTGCAGAAGCT
CTCTAAGCACAAGACACCAGCACAGCCACAGCTCAAAGCGGCCAACTGCTGTGAGGAGGT
GAAGGAGCTCAAGGCCCAAGTTGCCAACCTTAGCAGCCTGCTGAGTGAACTGAACAAGAA
GCAGGAGAGGGACTGGGTCAGCGTGGTCATGCAGGTGATGGAGCTGGAGAGCAACAGCAA
GCGCATGGAGTCGCGGCTCACAGATGCTGAGAGCAAGTACTCCGAGATGAACAACCAAAT
TGACATCATGCAGCTGCAGGCAGCACAGACGGTCACTCAGACCTCCGCAGATGCCATCTA
CGACTGCTCTTCCCTCTACCAGAAGAACTACCGCATCTCTGGAGTGTATAAGCTTCCTCC
TGATGACTTCCTGGGCAGCCCTGAACTGGAGGTGTTCTGTGACATGGAGACTTCAGGCGG
AGGCTGGACCATCATCCAGAGACGAAAAAGTGGCCTTGTCTCCTTCTACCGGGACTGGAA
GCAGTACAAGCAGGGCTTTGGCAGCATCCGTGGGGACTTCTGGCTGGGGAACGAACACAT
CCACCGGCTCTCCAGACAGCCAACCCGGCTGCGTGTAGAGATGGAGGACTGGGAGGGCAA
CCTGCGCTACGCTGAGTATAGCCACTTTGTTTTGGGCAATGAACTCAACAGCTATCGCCT
CTTCCTGGGGAACTACACTGGCAATGTGGGGAACGACGCCCTCCAGTATCATAACAACAC
AGCCTTCAGCACCAAGGACAAGGACAATGACAACTGCTTGGACAAGTGTGCACAGCTCCG
CAAAGGTGGCTACTGGTACAACTGCTGCACAGACTCCAACCTCAATGGAGTGTACTACCG
CCTGGGTGAGCACAATAAGCACCTGGATGGCATCACCTGGTATGGCTGGCATGGATCTAC
CTACTCCCTCAAACGGGTGGAGATGAAAATCCGCCCAGAAGACTTCAAGCCT**TAA**AAGGA
GGCTGCCGTGGAGCACGGATACAGAAACTGAGACACGTGGAGACTGGATGAGGGCAGATG
AGGACAGGAAGAGAGTGTTAGAAAGGGTAGGACTGAGAAACAGCCTATAATCTCCAAAGA
AAGAATAAGTCTCCAAGGAGCACAAAAAAATCATATGTACCAAGGATGTTACAGTAAACA
GGATGAACTATTTAAACCCACTGGGTCCTGCCACATCCTTCTCAAGGTGGTAGACTGAGT
GGGGTCTCTCTGCCCAAGATCCCTGACATAGCAGTAGCTTGTCTTTTCCACATGATTTGT
CTGTGAAAGAAAATAATTTTGAGATCGTTTTATCTATTTTCTCTACGGCTTAGGCTATGT
GAGGGCAAAACACAAATCCCTTTGCTAAAAAGAACCATATTATTTTGATTCTCAAAGGAT
AGGCCTTTGAGTGTTAGAGAAAGGAGTGAAGGAGGCAGGTGGGAAATGGTATTTCTATTT
TTAAATCCAGTGAAATTATCTTGAGTCTACACATTATTTTTAAAACACAAAAATTGTTCG
GCTGGAACTGACCCAGGCTGGACTTGCGGGGAGGAAACTCCAGGGCACTGCATCTGGCGA
TCAGACTCTGAGCACTGCCCCTGCTCGCCTTGGTCATGTACAGCACTGAAAGGAATGAAG
CACCAGCAGGAGGTGGACAGAGTCTCTCATGGATGCCGGCACAAAACTGCCTTAAAATAT
TCATAGTTAATACAGGTATATCTATTTTTATTTACTTTGTAAGAAACAAGCTCAAGGAGC
TTCCTTTTAAATTTTGTCTGTAGGAAATGGTTGAAAACTGAAGGTAGATGGTGTTATAGT
TAATAATAAATGCTGTAAATAAGCATCTCACTTTGTAAAAATAAAATATTGTGGTTTTGT
TTTAAACATTCAACGTTTCTTTTCCTTCTACAATAAACACTTTCAAAATGTG

# FIGURE 18

><subunit 1 of 1, 346 aa, 1 stop
><MW: 40018, pI: 8.19, NX(S/T): 3

MLKKPLSAVTWLCIFIVAFVSHPAWLQKLSKHKTPAQPQLKAANCCEEVKELKAQVANLS
SLLSELNKKQERDWVSVVMQVMELESNSKRMESRLTDAESKYSEMNNQIDIMQLQAAQTV
TQTSADAIYDCSSLYQKNYRISGVYKLPPDDFLGSPELEVFCDMETSGGGWTIIQRRKSG
LVSFYRDWKQYKQGFGSIRGDFWLGNEHIHRLSRQPTRLRVEMEDWEGNLRYAEYSHFVL
GNELNSYRLFLGNYTGNVGNDALQYHNNTAFSTKDKDNDNCLDKCAQLRKGGYWYNCCTD
SNLNGVYYRLGEHNKHLDGITWYGWHGSTYSLKRVEMKIRPEDFKP

**Signal sequence.**
amino acids 1-26

**N-glycosylation sites.**
amino acids 58-62, 253-257, 267-271

**Glycosaminoglycan attachment site.**
amino acids 167-171

**cAMP- and cGMP-dependent protein kinase phosphorylation site.**
amino acids 176-180

**N-myristoylation sites.**
amino acids 168-174, 196-202, 241-247, 252-258, 256-262, 327-333

**Cell attachment sequence.**
amino acids 199-202

# FIGURE 19

AAACTTGACGCC**ATG**AAGATCCCGGTCCTTCCTGCCGTGGTGCTCCTCTCCCTCCTGGTG
CTCCACTCTGCCCAGGGAGCCACCCTGGGTGGTCCTGAGGAAGAAAGCACCATTGAGAAT
TATGCGTCACGACCCGAGGCCTTTAACACCCCGTTCCTGAACATCGACAAATTGCGATCT
GCGTTTAAGGCTGATGAGTTCCTGAACTGGCACGCCCTCTTTGAGTCTATCAAAAGGAAA
CTTCCTTTCCTCAACTGGGATGCCTTTCCTAAGCTGAAAGGACTGAGGAGCGCAACTCCT
GATGCCCAG**TGA**CCATGACCTCCACTGGAAGAGGGGGCTAGCGTGAGCGCTGATTCTCAA
CCTACCATAACTCTTTCCTGCCTCAGGAACTCCAATAAAACATTTTCCATCCAAA

# FIGURE 20

></usr/seqdb2/sst/DNA/Dnaseqs.min/ss.DNA57694
><subunit 1 of 1, 99 aa, 1 stop
><MW: 11050, pI: 7.47, NX(S/T): 0
MKIPVLPAVVLLSLLVLHSAQGATLGGPEEESTIENYASRPEAFNTPFLNIDKLRSAFKA
DEFLNWHALFESIKRKLPFLNWDAFPKLKGLRSATPDAQ

**Signal peptide:**
amino acids 1-22

**N-myristoylation sites.**
amino acids 22-28, 90-96

**Homologous region to perioxdase.**
amino acids 16-48

# FIGURE 21

CAACAGAAGCCAAGAAGGAAGCCGTCTATCTTGTGGCGATC**ATG**TATAAGCTGGCCTCCT
GCTGTTTGCTTTTCACAGGATTCTTAAATCCTCTCTTATCTCTTCCTCTCCTTGACTCCA
GGGAAATATCCTTTCAACTCTCAGCACCTCATGAAGACGCGCGCTTAACTCCGGAGGAGC
TAGAAAGAGCTTCCCTTCTACAGATATTGCCAGAGATGCTGGGTGCAGAAAGAGGGGATA
TTCTCAGGAAAGCAGACTCAAGTACCAACATTTTTAACCCAAGAGGAAATTTGAGAAAGT
TTCAGGATTTCTCTGGACAAGATCCTAACATTTTACTGAGTCATCTTTTGGCCAGAATCT
GGAAACCATACAAGAAACGTGAGACTCCTGATTGCTTCTGGAAATACTGTGTC**TGA**AGTG
AAATAAGCATCTGTTAGTCAGCTCAGAAACACCCATCTTAGAATATGAAAAATAACACAA
TGCTTGATTTGAAAACAGTGTGGAGAAAAACTAGGCAAACTACACCCTGTTCATTGTTAC
CTGGAAAATAAATCCTCTATGTTTTGCACAAAAAAAAAAAAAAAA

# FIGURE 22

</usr/seqdb2/sst/DNA/Dnaseqs.min/ss.DNA59214

<subunit 1 of 1, 124 aa, 1 stop

<MW: 14284, pI: 8.14, NX(S/T): 0

MYKLASCCLLFTGFLNPLLSLPLLDSREISFQLSAPHEDARLTPEELERASLLQILPEML
GAERGDILRKADSSTNIFNPRGNLRKFQDFSGQDPNILLSHLLARIWKPYKKRETPDCFW
KYCV

**Important features:**

**Signal peptide:**

amino acids 1-20

**Urotensin II signature.**

amino acids 118-124

**Cell attachment sequence.**

amino acids 64-67

**cAMP- and cGMP-dependent protein kinase phosphorylation site.**

amino acids 112-116

**N-myristoylation sites.**

amino acids 61-67, 92-98

# FIGURE 23

GGGAGAGAGGATAAATAGCAGCGTGGCTTCCCTGGCTCCTCTCTGCATCCTTCCCGACCT
TCCCAGCAAT**ATG**CATCTTGCACGTCTGGTCGGCTCCTGCTCCCTCCTTCTGCTACTGGG
GGCCCTGTCTGGATGGGCGGCCAGCGATGACCCCATTGAGAAGGTCATTGAAGGGATCAA
CCGAGGGCTGAGCAATGCAGAGAGAGAGGTGGGCAAGGCCCTGGATGGCATCAACAGTGG
AATCACGCATGCCGGAAGGGAAGTGGAGAAGGTTTTCAACGGACTTAGCAACATGGGGAG
CCACACCGGCAAGGAGTTGGACAAAGGCGTCCAGGGGCTCAACCACGGCATGGACAAGGT
TGCCCATGAGATCAACCATGGTATTGGACAAGCAGGAAAGGAAGCAGAGAAGCTTGGCCA
TGGGGTCAACAACGCTGCTGGACAGGCCGGGAAGGAAGCAGACAAAGCGGTCCAAGGGTT
CCACACTGGGGTCCACCAGGCTGGGAAGGAAGCAGAGAAACTTGGCCAAGGGGTCAACCA
TGCTGCTGACCAGGCTGGAAAGGAAGTGGAGAAGCTTGGCCAAGGTGCCCACCATGCTGC
TGGCCAGGCCGGGAAGGAGCTGCAGAATGCTCATAATGGGGTCAACCAAGCCAGCAAGGA
GGCCAACCAGCTGCTGAATGGCAACCATCAAAGCGGATCTTCCAGCCATCAAGGAGGGGC
CACAACCACGCCGTTAGCCTCTGGGGCCTCAGTCAACACGCCTTTCATCAACCTTCCCGC
CCTGTGGAGGAGCGTCGCCAACATCATGCCC**TAA**ACTGGCATCCGGCCTTGCTGGGAGAA
TAATGTCGCCGTTGTCACATCAGCTGACATGACCTGGAGGGGTTGGGGGTGGGGGACAGG
TTTCTGAAATCCCTGAAGGGGGTTGTACTGGGATTTGTGAATAAACTTGATACACCA

# FIGURE 24

></usr/seqdb2/sst/DNA/Dnaseqs.min/ss.DNA66675
><subunit 1 of 1, 247 aa, 1 stop
><MW: 25335, pI: 7.00, NX(S/T): 0

MHLARLVGSCSLLLLLGALSGWAASDDPIEKVIEGINRGLSNAEREVGKALDGINSGITH
AGREVEKVFNGLSNMGSHTGKELDKGVQGLNHGMDKVAHEINHGIGQAGKEAEKLGHGVN
NAAGQAGKEADKAVQGFHTGVHQAGKEAEKLGQGVNHAADQAGKEVEKLGQGAHHAAGQA
GKELQNAHNGVNQASKEANQLLNGNHQSGSSSHQGGATTTPLASGASVNTPFINLPALWR
SVANIMP

**Important features of the protein:**

**Signal peptide:**

amino acids 1-25

**N-myristoylation sites.**

amino acids 17-23, 35-41, 39-45, 53-59, 57-63, 76-82, 89-95, 104-110, 118-124, 140-146, 152-158, 154-160, 172-178, 190-196, 204-210, 215-221, 225-231

**Homologous region to circumsporozoite (CS) repeats:**

amino acids 35-225

# FIGURE 25

CAGCCGGGTCCCAAGCCTGTGCCTGAGCCTGAGCCTGAGCCTGAGCCCGAGCCGGGAGCC
GGTCGCGGGGGCTCCGGGCTGTGGGACCGCTGGGCCCCCAGCG**ATG**GCGACCCTGTGGGG
AGGCCTTCTTCGGCTTGGCTCCTTGCTCAGCCTGTCGTGCCTGGCGCTTTCCGTGCTGCT
GCTGGCGCAGCTGTCAGACGCCGCCAAGAATTTCGAGGATGTCAGATGTAAATGTATCTG
CCCTCCCTATAAAGAAAATTCTGGGCATATTTATAATAAGAACATATCTCAGAAAGATTG
TGATTGCCTTCATGTTGTGGAGCCCATGCCTGTGCGGGGGCCTGATGTAGAAGCATACTG
TCTACGCTGTGAATGCAAATATGAAGAAAGAAGCTCTGTCACAATCAAGGTTACCATTAT
AATTTATCTCTCCATTTTGGGCCTTCTACTTCTGTACATGGTATATCTTACTCTGGTTGA
GCCCATACTGAAGAGGCGCCTCTTTGGACATGCACAGTTGATACAGAGTGATGATGATAT
TGGGGATCACCAGCCTTTTGCAAATGCACACGATGTGCTAGCCCGCTCCCGCAGTCGAGC
CAACGTGCTGAACAAGGTAGAATATGCACAGCAGCGCTGGAAGCTTCAAGTCCAAGAGCA
GCGAAAGTCTGTCTTTGACCGGCATGTTGTCCTCAGC**TAA**TTGGGAATTGAATTCAAGGT
GACTAGAAAGAAACAGGCAGACAACTGGAAAGAACTGACTGGGTTTTGCTGGGTTTCATT
TTAATACCTTGTTGATTTCACCAACTGTTGCTGGAAGATTCAAAACTGGAAGCAAAAACT
TGCTTGATTTTTTTTTCTTGTTAACGTAATAATAGAGACATTTTTAAAAGCACACAGCTC
AAAGTCAGCCAATAAGTCTTTTCCTATTTGTGACTTTTACTAATAAAAATAAATCTGCCT
GTAAATTATCTTGAAGTCCTTTACCTGGAACAAGCACTCTCTTTTTCACCACATAGTTTT
AACTTGACTTTCAAGATAATTTTCAGGGTTTTTGTTGTTGTTGTTTTTTGTTTGTTTGTT
TTGGTGGGAGAGGGGAGGGATGCCTGGGAAGTGGTTAACAACTTTTTTCAAGTCACTTTA
CTAAACAAACTTTTGTAAATAGACCTTACCTTCTATTTTCGAGTTTCATTTATATTTTGC
AGTGTAGCCAGCCTCATCAAAGAGCTGACTTACTCATTTGACTTTTGCACTGACTGTATT
ATCTGGGTATCTGCTGTGTCTGCACTTCATGGTAAACGGGATCTAAAATGCCTGGTGGCT
TTTCACAAAAAGCAGATTTTCTTCATGTACTGTGATGTCTGATGCAATGCATCCTAGAAC
AAACTGGCCATTTGCTAGTTTACTCTAAAGACTAAACATAGTCTTGGTGTGTGTGGTCTT
ACTCATCTTCTAGTACCTTTAAGGACAAATCCTAAGGACTTGGACACTTGCAATAAAGAA
ATTTTATTTTAAACCCAAGCCTCCCTGGATTGATAATATATACACATTTGTCAGCATTTC
CGGTCGTGGTGAGAGGCAGCTGTTTGAGCTCCAATATGTGCAGCTTTGAACTAGGGCTGG
GGTTGTGGGTGCCTCTTCTGAAAGGTCTAACCATTATTGGATAACTGGCTTTTTTCTTCC
TATGTCCTCTTTGGAATGTAACAATAAAAATAATTTTTGAAACATCAA

# FIGURE 26

></usr/seqdb2/sst/DNA/Dnaseqs.min/ss.DNA67004
><subunit 1 of 1, 198 aa, 1 stop
><MW: 22531, pI: 8.47, NX(S/T): 1
MATLWGGLLRLGSLLSLSCLALSVLLLAQLSDAAKNFEDVRCKCICPPYKENSGHIYNKN
ISQKDCDCLHVVEPMPVRGPDVEAYCLRCECKYEERSSVTIKVTIIIYLSILGLLLLYMV
YLTLVEPILKRRLFGHAQLIQSDDDIGDHQPFANAHDVLARSRSRANVLNKVEYAQQRWK
LQVQEQRKSVFDRHVVLS

**Transmembrane domain:**
amino acids 11-28 (type II), 103-125

**N-glycosylation site.**
amino acids 60-64

**Tyrosine kinase phosphorylation site.**
amino acids 78-86

**N-myristoylation site.**
amino acids 12-18

Sequence Listing

<110> Genentech, Inc. et al.

<120> Compositions and Methods for the Treatment of Immune Related Diseases

<130> CMD/FP6249890

<150> EP 00919420.0
<151> 2000-03-15

<150> PCT/US00/06884
<151> 2000-03-15

<150> US 60/144,758
<151> 1999-07-20

<160> 64

<210> 1
<211> 2042
<212> DNA
<213> Homo sapiens

<400> 1
gcggacgcgt gggtgaaatt gaaaatcaag ataaaaatgt tcacaattaa 50

gctccttctt tttattgttc ctctagttat ttcctccaga attgatcaag 100

acaattcatc atttgattct ctatctccag agccaaaatc aagatttgct 150

atgttagacg atgtaaaaat tttagccaat ggcctccttc agttgggaca 200

tggtcttaaa gactttgtcc ataagacgaa gggccaaatt aatgacatat 250

ttcaaaaact caacatattt gatcagtctt tttatgatct atcgctgcaa 300

accagtgaaa tcaaagaaga agaaaaggaa ctgagaagaa ctacatataa 350

actacaagtc aaaaatgaag aggtaaagaa tatgtcactt gaactcaact 400

caaaacttga aagcctccta gaagaaaaaa ttctacttca acaaaaagtg 450

aaatatttag aagagcaact aactaactta attcaaaatc aacctgaaac 500

tccagaacac ccagaagtaa cttcacttaa aacttttgta gaaaaacaag 550

ataatagcat caaagacctt ctccagaccg tggaagacca atataaacaa 600

ttaaaccaac agcatagtca aataaaagaa atagaaaatc agctcagaag 650

gactagtatt caagaaccca cagaaatttc tctatcttcc aagccaagag 700

caccaagaac tactcccttt cttcagttga atgaaataag aaatgtaaaa 750

catgatggca ttcctgctga atgtaccacc atttataaca gaggtgaaca 800

tacaagtggc atgtatgcca tcagacccag caactctcaa gtttttcatg 850

tctactgtga tgttatatca ggtagtccat ggacattaat tcaacatcga 900

atagatggat cacaaaactt caatgaaacg tgggagaact acaaatatgg 950

ttttgggagg cttgatggag aattttggtt gggcctagag aagatatact 1000

ccatagtgaa gcaatctaat tatgttttac gaattgagtt ggaagactgg 1050

aaagacaaca aacattatat tgaatattct ttttacttgg gaaatcacga 1100

aaccaactat acgctacatc tagttgcgat tactggcaat gtccccaatg 1150

caatcccgga aaacaaagat ttggtgtttt ctacttggga tcacaaagca 1200

aaaggacact tcaactgtcc agagggttat tcaggaggct ggtggtggca 1250

tgatgagtgt ggagaaaaca acctaaatgg taaatataac aaaccaagag 1300

caaaatctaa gccagagagg agaagaggat tatcttggaa gtctcaaaat 1350

ggaaggttat actctataaa atcaaccaaa atgttgatcc atccaacaga 1400

ttcagaaagc tttgaatgaa ctgaggcaat ttaaaggcat atttaaccat 1450

taactcattc caagttaatg tggtctaata atctggtata aatccttaag 1500

agaaagcttg agaaatagat ttttttatc ttaaagtcac tgtctattta 1550

agattaaaca tacaatcaca taaccttaaa gaataccgtt tacatttctc 1600

aatcaaaatt cttataatac tatttgtttt aaattttgtg atgtgggaat 1650

caattttaga tggtcacaat ctagattata atcaataggt gaacttatta 1700

aataactttt ctaaataaaa aatttagaga cttttatttt aaaaggcatc 1750

atatgagcta atatcacaac tttcccagtt taaaaaacta gtactcttgt 1800

taaaactcta aacttgacta aatacagagg actggtaatt gtacagttct 1850

taaatgttgt agtattaatt tcaaaactaa aaatcgtcag cacagagtat 1900

gtgtaaaaat ctgtaataca aatttttaaa ctgatgcttc attttgctac 1950

aaaataattt ggagtaaatg tttgatatga tttatttatg aaacctaatg 2000

aagcagaatt aaatactgta ttaaaataag ttcgctgtct tt 2042

<210> 2
<211> 460
<212> PRT
<213> Homo sapiens

<400> 2

```
Met Phe Thr Ile Lys Leu Leu Leu Phe Ile Val Pro Leu Val Ile
1               5                   10                  15

Ser Ser Arg Ile Asp Gln Asp Asn Ser Ser Phe Asp Ser Leu Ser
                20                  25                  30

Pro Glu Pro Lys Ser Arg Phe Ala Met Leu Asp Asp Val Lys Ile
                35                  40                  45
```

Leu Ala Asn Gly Leu Leu Gln Leu Gly His Gly Leu Lys Asp Phe
50 55 60

Val His Lys Thr Lys Gly Gln Ile Asn Asp Ile Phe Gln Lys Leu
65 70 75

Asn Ile Phe Asp Gln Ser Phe Tyr Asp Leu Ser Leu Gln Thr Ser
80 85 90

Glu Ile Lys Glu Glu Glu Lys Glu Leu Arg Arg Thr Thr Tyr Lys
95 100 105

Leu Gln Val Lys Asn Glu Glu Val Lys Asn Met Ser Leu Glu Leu
110 115 120

Asn Ser Lys Leu Glu Ser Leu Leu Glu Glu Lys Ile Leu Leu Gln
125 130 135

Gln Lys Val Lys Tyr Leu Glu Glu Gln Leu Thr Asn Leu Ile Gln
140 145 150

Asn Gln Pro Glu Thr Pro Glu His Pro Glu Val Thr Ser Leu Lys
155 160 165

Thr Phe Val Glu Lys Gln Asp Asn Ser Ile Lys Asp Leu Leu Gln
170 175 180

Thr Val Glu Asp Gln Tyr Lys Gln Leu Asn Gln Gln His Ser Gln
185 190 195

Ile Lys Glu Ile Glu Asn Gln Leu Arg Arg Thr Ser Ile Gln Glu
200 205 210

Pro Thr Glu Ile Ser Leu Ser Ser Lys Pro Arg Ala Pro Arg Thr
215 220 225

Thr Pro Phe Leu Gln Leu Asn Glu Ile Arg Asn Val Lys His Asp
230 235 240

Gly Ile Pro Ala Glu Cys Thr Thr Ile Tyr Asn Arg Gly Glu His
245 250 255

Thr Ser Gly Met Tyr Ala Ile Arg Pro Ser Asn Ser Gln Val Phe
260 265 270

His Val Tyr Cys Asp Val Ile Ser Gly Ser Pro Trp Thr Leu Ile
275 280 285

Gln His Arg Ile Asp Gly Ser Gln Asn Phe Asn Glu Thr Trp Glu
290 295 300

Asn Tyr Lys Tyr Gly Phe Gly Arg Leu Asp Gly Glu Phe Trp Leu
305 310 315

Gly Leu Glu Lys Ile Tyr Ser Ile Val Lys Gln Ser Asn Tyr Val
320 325 330

Leu Arg Ile Glu Leu Glu Asp Trp Lys Asp Asn Lys His Tyr Ile
335 340 345

```
Glu Tyr Ser Phe Tyr Leu Gly Asn His Glu Thr Asn Tyr Thr Leu
                350                 355                 360

His Leu Val Ala Ile Thr Gly Asn Val Pro Asn Ala Ile Pro Glu
                365                 370                 375

Asn Lys Asp Leu Val Phe Ser Thr Trp Asp His Lys Ala Lys Gly
                380                 385                 390

His Phe Asn Cys Pro Glu Gly Tyr Ser Gly Gly Trp Trp Trp His
                395                 400                 405

Asp Glu Cys Gly Glu Asn Asn Leu Asn Gly Lys Tyr Asn Lys Pro
                410                 415                 420

Arg Ala Lys Ser Lys Pro Glu Arg Arg Arg Gly Leu Ser Trp Lys
                425                 430                 435

Ser Gln Asn Gly Arg Leu Tyr Ser Ile Lys Ser Thr Lys Met Leu
                440                 445                 450

Ile His Pro Thr Asp Ser Glu Ser Phe Glu
                455                 460
```

```
<210> 3
<211> 1964
<212> DNA
<213> Homo sapiens

<220>
<221> unsure
<222> 1857, 1875
<223> unknown base

<400> 3
cagctctcat ttctccaaaa atgtgtttga gccacttgga aaatatgcct 50

ttaagccatt caagaactca aggagctcag agatcatcct ggaagctgtg 100

gctcttttgc tcaatagtta tgttgctatt tctttgctcc ttcagttggc 150

taatctttat ttttctccaa ttagagactg ctaaggagcc ctgtatggct 200

aagtttggac cattaccctc aaaatggcaa atggcatctt ctgaacctcc 250

ttgcgtgaat aaggtgtctg actggaagct ggagatactt cagaatggct 300

tatatttaat ttatggccaa gtggctccca atgcaaacta caatgatgta 350

gctccttttg aggtgcggct gtataaaaac aaagacatga tacaaactct 400

aacaaacaaa tctaaaatcc aaaatgtagg agggacttat gaattgcatg 450

ttggggacac catagacttg atattcaact ctgagcatca ggttctaaaa 500

aataatacat actggggtat cattttacta gcaaatcccc aattcatctc 550

ctagagactt gatttgatct cctcattccc ttcagcacat gtagaggtgc 600

cagtgggtgg attggaggga gaagatattc aatttctaga gtttgtctgt 650
```

ctacaaaaat caacacaaac agaactcctc tgcacgtgaa ttttcatcta 700

tcatgcctat ctgaaagaga ctcaggggaa gagccaaaga cttttggttg 750

gatctgcaga aatacttcat taatccatga taaaacaaat atggatgaca 800

gaggacatgt gcttttcaaa gaatctttat ctaattcttg aattcatgag 850

tggaaaaatg gagttctatt cccatggaag atttacctgg tatgcaaaaa 900

ggatctgggg cagtagcctg gctttgttct catattcttg ggctgctgta 950

attcattctt ctcatactcc catcttctga gaccctccca ataaaaagta 1000

gactgatagg atggccacag atatgcctac cataccctac tttagatatg 1050

gtggtgttag aagataaaga acaatctgag aactattgga atagaggtac 1100

aagtggcata aaatggaatg tacgctatct ggaaatttct cttggtttta 1150

tcttcctcag gatgcagggt gctttaaaaa gccttatcaa aggagtcatt 1200

ccgaaccctc acgtagagct ttgtgagacc ttactgttgg tgtgtgtgtc 1250

taaacattgc taattgtaaa gaaagagtaa ccattagtaa tcattaggtt 1300

taaccccaga atggtattat cattactgga ttatgtcatg taatgattta 1350

gtatttttag ctagctttcc acagtttgca aagtgctttc gtaaaacagt 1400

tagcaattct atgaagttaa ttgggcaggc atttggggga aaattttagt 1450

gatgagaatg tgatagcata gcatagccaa ctttcctcaa ctcataggac 1500

aagtgactac aagaggcaat gggtagtccc ctgcattgca ctgtctcagc 1550

tttagaattg ttatttctgc tatcgtgtta taagactcta aaacttagcg 1600

aattcacttt tcaggaagca tattcccctt tagcccaagg tgagcagagt 1650

gaagctacaa cagatctttc ctttaccagc acactttttt ttttttttcc 1700

tgcctgaatc agggagatcc aggatgctgt tcaggccaaa tcccaaccaa 1750

attccccttt tcactttgca gggcccatct tagtcaaatg tgctaacttc 1800

taaaataata aatagcacta attcaaaatt tttggaatct taaattagct 1850

acttgcnggt tgcttgttga aaggnatata atgattacat tgtaaacaaa 1900

tttaaaatat ttatggatat ttgtgaaaag ctgcattatg ttaaataata 1950

ttacatgtaa agct 1964

<210> 4
<211> 177
<212> PRT
<213> Homo sapiens

<400> 4
Met Cys Leu Ser His Leu Glu Asn Met Pro Leu Ser His Ser Arg
1               5                   10                  15

Thr Gln Gly Ala Gln Arg Ser Ser Trp Lys Leu Trp Leu Phe Cys
20 25 30

Ser Ile Val Met Leu Leu Phe Leu Cys Ser Phe Ser Trp Leu Ile
35 40 45

Phe Ile Phe Leu Gln Leu Glu Thr Ala Lys Glu Pro Cys Met Ala
50 55 60

Lys Phe Gly Pro Leu Pro Ser Lys Trp Gln Met Ala Ser Ser Glu
65 70 75

Pro Pro Cys Val Asn Lys Val Ser Asp Trp Lys Leu Glu Ile Leu
80 85 90

Gln Asn Gly Leu Tyr Leu Ile Tyr Gly Gln Val Ala Pro Asn Ala
95 100 105

Asn Tyr Asn Asp Val Ala Pro Phe Glu Val Arg Leu Tyr Lys Asn
110 115 120

Lys Asp Met Ile Gln Thr Leu Thr Asn Lys Ser Lys Ile Gln Asn
125 130 135

Val Gly Gly Thr Tyr Glu Leu His Val Gly Asp Thr Ile Asp Leu
140 145 150

Ile Phe Asn Ser Glu His Gln Val Leu Lys Asn Asn Thr Tyr Trp
155 160 165

Gly Ile Ile Leu Leu Ala Asn Pro Gln Phe Ile Ser
170 175

<210> 5
<211> 715
<212> DNA
<213> Homo sapiens

<400> 5
tatttaccat atcagattca cattcagtcc tcagcaaaat gaagggctcc 50

attttcactc tgtttttatt ctctgtccta tttgccatct cagaagtgcg 100

gagcaaggag tctgtgagac tctgtgggct agaatacata cggacagtca 150

tctatatctg tgctagctcc aggtggagaa ggcatctgga ggggatccct 200

caagctcagc aagctgagac aggaaactcc ttccagctcc cacataaacg 250

tgagttttct gaggaaaatc cagcgcaaaa ccttccgaag gtggatgcct 300

caggggaaga ccgtctttgg ggtggacaga tgcccactga agagctttgg 350

aagtcaaaga agcattcagt gatgtcaaga caagatttac aaactttgtg 400

ttgcactgat ggctgttcca tgactgattt gagtgctctt tgctaagaca 450

agagcaaata cccaatgggt ggcagagctt tatcacatgt ttaattacag 500

tgttttactg cctggtagaa cactaatatt gtgttattaa aatgatggct 550

tttgggtagg caaaacttct tttctaaaag gtatagctga gcggttgaaa 600

ccacagtgat ctctattttc tccctttgcc aaggttaatg aactgttctt 650

ttcaaattct actaatgctt tgaaatttca aatgctgcgc aaaattgcaa 700

taaaaatgct ataaa 715

<210> 6
<211> 135
<212> PRT
<213> Homo sapiens

<400> 6
Met Lys Gly Ser Ile Phe Thr Leu Phe Leu Phe Ser Val Leu Phe
1 5 10 15

Ala Ile Ser Glu Val Arg Ser Lys Glu Ser Val Arg Leu Cys Gly
20 25 30

Leu Glu Tyr Ile Arg Thr Val Ile Tyr Ile Cys Ala Ser Ser Arg
35 40 45

Trp Arg Arg His Leu Glu Gly Ile Pro Gln Ala Gln Gln Ala Glu
50 55 60

Thr Gly Asn Ser Phe Gln Leu Pro His Lys Arg Glu Phe Ser Glu
65 70 75

Glu Asn Pro Ala Gln Asn Leu Pro Lys Val Asp Ala Ser Gly Glu
80 85 90

Asp Arg Leu Trp Gly Gly Gln Met Pro Thr Glu Glu Leu Trp Lys
95 100 105

Ser Lys Lys His Ser Val Met Ser Arg Gln Asp Leu Gln Thr Leu
110 115 120

Cys Cys Thr Asp Gly Cys Ser Met Thr Asp Leu Ser Ala Leu Cys
125 130 135

<210> 7
<211> 1102
<212> DNA
<213> Homo sapiens

<400> 7
gctgtgggaa cctctccacg cgcacgaact cagccaacga tttctgatag 50

atttttggga gtttgaccag agatgcaagg ggtgaaggag cgcttcctac 100

cgttagggaa ctctggggac agagcgcccc ggccgcctga tggccgaggc 150

agggtgcgac ccaggaccca ggacggcgtc gggaaccata ccatggcccg 200

gatccccaag accctaaagt tcgtcgtcgt catcgtcgcg gtcctgctgc 250

cagtcctagc ttactctgcc accactgccc ggcaggagga agttccccag 300

cagacagtgg ccccacagca acagaggcac agcttcaagg gggaggagtg 350

tccagcagga tctcatagat cagaacatac tggagcctgt aacccgtgca 400

cagagggtgt ggattacacc aacgcttcca acaatgaacc ttcttgcttc 450

ccatgtacag tttgtaaatc agatcaaaaa cataaaagtt cctgcaccat 500

gaccagagac acagtgtgtc agtgtaaaga aggcaccttc cggaatgaaa 550

actccccaga gatgtgccgg aagtgtagca ggtgccctag tggggaagtc 600

caagtcagta attgtacgtc ctgggatgat atccagtgtg ttgaagaatt 650

tggtgccaat gccactgtgg aaaccccagc tgctgaagag acaatgaaca 700

ccagcccggg gactcctgcc ccagctgctg aagagacaat gaacaccagc 750

ccagggactc ctgccccagc tgctgaagag acaatgacca ccagcccggg 800

gactcctgcc ccagctgctg aagagacaat gaccaccagc ccggggactc 850

ctgccccagc tgctgaagag acaatgacca ccagcccggg gactcctgcc 900

tcttctcatt acctctcatg caccatcgta gggatcatag ttctaattgt 950

gcttctgatt gtgtttgttt gaaagacttc actgtggaag aaattccttc 1000

cttacctgaa aggttcaggt aggcgctggc tgagggcggg gggcgctgga 1050

cactctctgc cctgcctccc tctgctgtgt tcccacagac agaaacgcct 1100

gc 1102

<210> 8
<211> 259
<212> PRT
<213> Homo sapiens

<400> 8

```
Met Ala Arg Ile Pro Lys Thr Leu Lys Phe Val Val Val Ile Val
 1               5                  10                  15

Ala Val Leu Leu Pro Val Leu Ala Tyr Ser Ala Thr Thr Ala Arg
                20                  25                  30

Gln Glu Glu Val Pro Gln Gln Thr Val Ala Pro Gln Gln Gln Arg
                35                  40                  45

His Ser Phe Lys Gly Glu Glu Cys Pro Ala Gly Ser His Arg Ser
                50                  55                  60

Glu His Thr Gly Ala Cys Asn Pro Cys Thr Glu Gly Val Asp Tyr
                65                  70                  75

Thr Asn Ala Ser Asn Asn Glu Pro Ser Cys Phe Pro Cys Thr Val
                80                  85                  90

Cys Lys Ser Asp Gln Lys His Lys Ser Ser Cys Thr Met Thr Arg
                95                 100                 105

Asp Thr Val Cys Gln Cys Lys Glu Gly Thr Phe Arg Asn Glu Asn
               110                 115                 120
```

Ser Pro Glu Met Cys Arg Lys Cys Ser Arg Cys Pro Ser Gly Glu
125 130 135

Val Gln Val Ser Asn Cys Thr Ser Trp Asp Asp Ile Gln Cys Val
140 145 150

Glu Glu Phe Gly Ala Asn Ala Thr Val Glu Thr Pro Ala Ala Glu
155 160 165

Glu Thr Met Asn Thr Ser Pro Gly Thr Pro Ala Pro Ala Ala Glu
170 175 180

Glu Thr Met Asn Thr Ser Pro Gly Thr Pro Ala Pro Ala Ala Glu
185 190 195

Glu Thr Met Thr Thr Ser Pro Gly Thr Pro Ala Pro Ala Ala Glu
200 205 210

Glu Thr Met Thr Thr Ser Pro Gly Thr Pro Ala Pro Ala Ala Glu
215 220 225

Glu Thr Met Thr Thr Ser Pro Gly Thr Pro Ala Ser Ser His Tyr
230 235 240

Leu Ser Cys Thr Ile Val Gly Ile Ile Val Leu Ile Val Leu Leu
245 250 255

Ile Val Phe Val

<210> 9
<211> 932
<212> DNA
<213> Homo sapiens

<220>
<221> unsure
<222> 911
<223> unknown base

<400> 9
gggaacggaa aatggcgcct cacggcccgg gtagtcttac gaccctggtg 50

ccctgggctg ccgccctgct cctcgctctg ggcgtggaaa gggctctggc 100

gctacccgag atatgcaccc aatgtccagg gagcgtgcaa aatttgtcaa 150

aagtggcctt ttattgtaaa acgacacgag agctaatgct gcatgcccgt 200

tgctgcctga atcagaaggg caccatcttg ggctggatc tccagaactg 250

ttctctggag gaccctggtc caaactttca tcaggcacat accactgtca 300

tcatagacct gcaagcaaac cccctcaaag gtgacttggc caacaccttc 350

cgtggcttta ctcagctcca gactctgata ctgccacaac atgtcaactg 400

tcctggagga attaatgcct ggaatactat cacctcttat atagacaacc 450

aaatctgtca agggcaaaag aacctttgca ataacactgg ggacccagaa 500

```
atgtgtcctg agaatggatc ttgtgtacct gatggtccag gtcttttgca 550
gtgtgtttgt gctgatggtt tccatggata caagtgtatg cgccagggct 600
cgttctcact gcttatgttc ttcgggattc tgggagccac cactctatcc 650
gtctccattc tgctttgggc gacccagcgc cgaaaagcca agacttcatg 700
aactacatag gtcttaccat tgacctaaga tcaatctgaa ctatcttagc 750
ccagtcaggg agctctgctt cctagaaagg catctttcgc cagtggattc 800
gcctcaaggt tgaggccgcc attggaagat gaaaaattgc actcccttgg 850
tgtagacaaa taccagttcc cattggtgtt gttgcctata ataaacactt 900
tttctttttt naaaaaaaaa aaaaaaaaaa aa 932

<210> 10
<211> 229
<212> PRT
<213> Homo sapiens

<400> 10
Met Ala Pro His Gly Pro Gly Ser Leu Thr Thr Leu Val Pro Trp
 1               5                  10                  15

Ala Ala Ala Leu Leu Leu Ala Leu Gly Val Glu Arg Ala Leu Ala
                20                  25                  30

Leu Pro Glu Ile Cys Thr Gln Cys Pro Gly Ser Val Gln Asn Leu
                35                  40                  45

Ser Lys Val Ala Phe Tyr Cys Lys Thr Thr Arg Glu Leu Met Leu
                50                  55                  60

His Ala Arg Cys Cys Leu Asn Gln Lys Gly Thr Ile Leu Gly Leu
                65                  70                  75

Asp Leu Gln Asn Cys Ser Leu Glu Asp Pro Gly Pro Asn Phe His
                80                  85                  90

Gln Ala His Thr Thr Val Ile Ile Asp Leu Gln Ala Asn Pro Leu
                95                 100                 105

Lys Gly Asp Leu Ala Asn Thr Phe Arg Gly Phe Thr Gln Leu Gln
               110                 115                 120

Thr Leu Ile Leu Pro Gln His Val Asn Cys Pro Gly Gly Ile Asn
               125                 130                 135

Ala Trp Asn Thr Ile Thr Ser Tyr Ile Asp Asn Gln Ile Cys Gln
               140                 145                 150

Gly Gln Lys Asn Leu Cys Asn Asn Thr Gly Asp Pro Glu Met Cys
               155                 160                 165

Pro Glu Asn Gly Ser Cys Val Pro Asp Gly Pro Gly Leu Leu Gln
               170                 175                 180
```

Cys Val Cys Ala Asp Gly Phe His Gly Tyr Lys Cys Met Arg Gln
185 190 195

Gly Ser Phe Ser Leu Leu Met Phe Phe Gly Ile Leu Gly Ala Thr
200 205 210

Thr Leu Ser Val Ser Ile Leu Leu Trp Ala Thr Gln Arg Arg Lys
215 220 225

Ala Lys Thr Ser

<210> 11
<211> 2482
<212> DNA
<213> Homo sapiens

<400> 11
gggggagaag gcggccgagc cccagctctc cgagcaccgg gtcggaagcc 50

gcgacccgag ccgcgcagga agctgggacc ggaacctcgg cggacccggc 100

cccacccaac tcacctgcgc aggtcaccag caccctcgga acccagaggc 150

ccgcgctctg aaggtgaccc ccctggggag gaaggcgatg gcccctgcga 200

ggacgatggc ccgcgcccgc ctcgccccgg ccggcatccc tgccgtcgcc 250

ttgtggcttc tgtgcacgct cggcctccag ggcacccagg ccgggccacc 300

gcccgcgccc cctgggctgc ccgcgggagc cgactgcctg aacagcttta 350

ccgccggggt gcctggcttc gtgctggaca ccaacgcctc ggtcagcaac 400

ggagctacct tcctggagtc cccccaccgtg cgccggggct gggactgcgt 450

gcgcgcctgc tgcaccaccc agaactgcaa cttggcgcta gtggagctgc 500

agcccgaccg cggggaggac gccatcgccg cctgcttcct catcaactgc 550

ctctacgagc agaacttcgt gtgcaagttc gcgcccaggg agggcttcat 600

caactacctc acgagggaag tgtaccgctc ctaccgccag ctgcggaccc 650

agggctttgg agggtctggg atccccaagg cctgggcagg catagacttg 700

aaggtacaac cccaggaacc cctggtgctg aaggatgtgg aaaacacaga 750

ttggcgccta ctgcggggtg acacggatgt cagggtagag aggaaagacc 800

caaaccaggt ggaactgtgg ggactcaagg aaggcaccta cctgttccag 850

ctgacagtga ctagctcaga ccacccagag gacacggcca acgtcacagt 900

cactgtgctg tccaccaagc agacagaaga ctactgcctc gcatccaaca 950

aggtgggtcg ctgccggggc tctttcccac gctggtacta tgaccccacg 1000

gagcagatct gcaagagttt cgtttatgga ggctgcttgg gcaacaagaa 1050

caactacctt cgggaagaag agtgcattct agcctgtcgg ggtgtgcaag 1100

gtgggccttt gagaggcagc tctggggctc aggcgacttt cccccagggc 1150

ccctccatgg aaaggcgcca tccagtgtgc tctggcacct gtcagcccac 1200

ccagttccgc tgcagcaatg gctgctgcat cgacagtttc ctggagtgtg 1250

acgacacccc caactgcccc gacgcctccg acgaggctgc ctgtgaaaaa 1300

tacacgagtg gctttgacga gctccagcgc atccatttcc ccagtgacaa 1350

agggcactgc gtggacctgc cagacacagg actctgcaag gagagcatcc 1400

cgcgctggta ctacaacccc ttcagcgaac actgcgcccg ctttacctat 1450

ggtggttgtt atggcaacaa gaacaacttt gaggaagagc agcagtgcct 1500

cgagtcttgt cgcggcatct ccaagaagga tgtgtttggc ctgaggcggg 1550

aaatccccat tcccagcaca ggctctgtgg agatggctgt cacagtgttc 1600

ctggtcatct gcattgtggt ggtggtagcc atcttgggtt actgcttctt 1650

caagaaccag agaaaggact tccacggaca ccaccaccac ccaccaccca 1700

cccctgccag ctccactgtc tccactaccg aggacacgga gcacctggtc 1750

tataaccaca ccacccggcc cctctgagcc tgggtctcac cggctctcac 1800

ctggccctgc ttcctgcttg ccaaggcaga ggcctgggct gggaaaaact 1850

ttggaaccag actcttgcct gtttcccagg cccactgtgc ctcagagacc 1900

agggctccag cccctcttgg agaagtctca gctaagctca cgtcctgaga 1950

aagctcaaag gtttggaagg agcagaaaac ccttgggcca gaagtaccag 2000

actagatgga cctgcctgca taggagtttg gaggaagttg gagttttgtt 2050

tcctctgttc aaagctgcct gtccctaccc catggtgcta ggaagaggag 2100

tggggtggtg tcagaccctg gaggccccaa ccctgtcctc ccgagctcct 2150

cttccatgct gtgcgcccag ggctgggagg aaggacttcc ctgtgtagtt 2200

tgtgctgtaa agagttgctt tttgtttatt taatgctgtg gcatgggtga 2250

agaggagggg aagaggcctg tttggcctct ctgtcctctc ttcctcttcc 2300

cccaagattg agctctctgc ccttgatcag ccccaccctg gcctagacca 2350

gcagacagag ccaggagagg ctcagctgca ttccgcagcc cccacccccca 2400

aggttctcca acatcacagc ccagcccacc cactgggtaa taaaagtggt 2450

ttgtggaaaa aaaaaaaaaa aaaaaaaaaa aa 2482

<210> 12
<211> 529
<212> PRT
<213> Homo sapiens

<400> 12

```
Met Ala Pro Ala Arg Thr Met Ala Arg Ala Arg Leu Ala Pro Ala
 1               5                  10                  15

Gly Ile Pro Ala Val Ala Leu Trp Leu Leu Cys Thr Leu Gly Leu
                20                  25                  30

Gln Gly Thr Gln Ala Gly Pro Pro Pro Ala Pro Pro Gly Leu Pro
                35                  40                  45

Ala Gly Ala Asp Cys Leu Asn Ser Phe Thr Ala Gly Val Pro Gly
                50                  55                  60

Phe Val Leu Asp Thr Asn Ala Ser Val Ser Asn Gly Ala Thr Phe
                65                  70                  75

Leu Glu Ser Pro Thr Val Arg Arg Gly Trp Asp Cys Val Arg Ala
                80                  85                  90

Cys Cys Thr Thr Gln Asn Cys Asn Leu Ala Leu Val Glu Leu Gln
                95                 100                 105

Pro Asp Arg Gly Glu Asp Ala Ile Ala Ala Cys Phe Leu Ile Asn
               110                 115                 120

Cys Leu Tyr Glu Gln Asn Phe Val Cys Lys Phe Ala Pro Arg Glu
               125                 130                 135

Gly Phe Ile Asn Tyr Leu Thr Arg Glu Val Tyr Arg Ser Tyr Arg
               140                 145                 150

Gln Leu Arg Thr Gln Gly Phe Gly Gly Ser Gly Ile Pro Lys Ala
               155                 160                 165

Trp Ala Gly Ile Asp Leu Lys Val Gln Pro Gln Glu Pro Leu Val
               170                 175                 180

Leu Lys Asp Val Glu Asn Thr Asp Trp Arg Leu Leu Arg Gly Asp
               185                 190                 195

Thr Asp Val Arg Val Glu Arg Lys Asp Pro Asn Gln Val Glu Leu
               200                 205                 210

Trp Gly Leu Lys Glu Gly Thr Tyr Leu Phe Gln Leu Thr Val Thr
               215                 220                 225

Ser Ser Asp His Pro Glu Asp Thr Ala Asn Val Thr Val Thr Val
               230                 235                 240

Leu Ser Thr Lys Gln Thr Glu Asp Tyr Cys Leu Ala Ser Asn Lys
               245                 250                 255

Val Gly Arg Cys Arg Gly Ser Phe Pro Arg Trp Tyr Tyr Asp Pro
               260                 265                 270

Thr Glu Gln Ile Cys Lys Ser Phe Val Tyr Gly Gly Cys Leu Gly
               275                 280                 285

Asn Lys Asn Asn Tyr Leu Arg Glu Glu Glu Cys Ile Leu Ala Cys
               290                 295                 300
```

```
Arg Gly Val Gln Gly Gly Pro Leu Arg Gly Ser Ser Gly Ala Gln
                305                 310                 315

Ala Thr Phe Pro Gln Gly Pro Ser Met Glu Arg Arg His Pro Val
                320                 325                 330

Cys Ser Gly Thr Cys Gln Pro Thr Gln Phe Arg Cys Ser Asn Gly
                335                 340                 345

Cys Cys Ile Asp Ser Phe Leu Glu Cys Asp Asp Thr Pro Asn Cys
                350                 355                 360

Pro Asp Ala Ser Asp Glu Ala Ala Cys Glu Lys Tyr Thr Ser Gly
                365                 370                 375

Phe Asp Glu Leu Gln Arg Ile His Phe Pro Ser Asp Lys Gly His
                380                 385                 390

Cys Val Asp Leu Pro Asp Thr Gly Leu Cys Lys Glu Ser Ile Pro
                395                 400                 405

Arg Trp Tyr Tyr Asn Pro Phe Ser Glu His Cys Ala Arg Phe Thr
                410                 415                 420

Tyr Gly Gly Cys Tyr Gly Asn Lys Asn Asn Phe Glu Glu Glu Gln
                425                 430                 435

Gln Cys Leu Glu Ser Cys Arg Gly Ile Ser Lys Lys Asp Val Phe
                440                 445                 450

Gly Leu Arg Arg Glu Ile Pro Ile Pro Ser Thr Gly Ser Val Glu
                455                 460                 465

Met Ala Val Thr Val Phe Leu Val Ile Cys Ile Val Val Val Val
                470                 475                 480

Ala Ile Leu Gly Tyr Cys Phe Phe Lys Asn Gln Arg Lys Asp Phe
                485                 490                 495

His Gly His His His His Pro Pro Pro Thr Pro Ala Ser Ser Thr
                500                 505                 510

Val Ser Thr Thr Glu Asp Thr Glu His Leu Val Tyr Asn His Thr
                515                 520                 525

Thr Arg Pro Leu
```

<210> 13
<211> 1899
<212> DNA
<213> Homo sapiens

<400> 13

```
gtgctgggct ttttcagaca agtgcatctc ctaaccaggt cacatttcag 50

ccgcgaccca ctctccgcca gtcaccggag gcagaccgcg ggaggagagc 100

tgaggacagc cgcgtgcgct tcgccagcag cggggtggga ggaaggacat 150
```

taaaatactg cagaagtcaa gacccccccca ggtcgaaccc agaccacgat 200
gcgcgccccg ggctgcgggc ggctggtgct gccgctgctg ctcctggccg 250
cggcagccct ggccgaaggc gacgccaagg ggctcaagga gggcgagacc 300
cccggcaatt tcatggagga cgagcaatgg ctgtcgtcca tctcgcagta 350
cagcggcaag atcaagcact ggaaccgctt ccgagacgaa gtggaggatg 400
actatatcaa gagctgggag gacaatcagc aaggagatga agccctggat 450
accaccaagg acccctgcca gaaggtgaag tgcagccgcc acaaggtgtg 500
cattgcccag ggctaccagc gggccatgtg catcagtcgc aagaagctgg 550
agcacaggat caagcagccg accgtgaaac tccatggaaa caaagactcc 600
atctgcaagc cctgccacat ggcccagctt gcctctgtct gcggctcaga 650
tggccacact tacagctctg tgtgtaagct ggagcaacag gcgtgcctga 700
gcagcaagca gctggcggtg cgatgcgagg gcccctgccc ctgccccacg 750
gagcaggctg ccacctccac cgccgatggc aaaccagaga cttgcaccgg 800
tcaggacctg gctgacctgg gagatcggct gcgggactgg ttccagctcc 850
ttcatgagaa ctccaagcag aatggctcag ccagcagtgt agccggcccg 900
gccagcgggc tggacaagag cctgggggcc agctgcaagg actccattgg 950
ctggatgttc tccaagctgg acaccagtgc tgacctcttc ctggaccaga 1000
cggagctggc cgccatcaac ctggacaagt acgaggtctg catccgtccc 1050
ttcttcaact cctgtgacac ctacaaggat ggccgggtct ctactgctga 1100
gtggtgcttc tgcttctgga gggagaagcc cccctgcctg gcagagctgg 1150
agcgcatcca gatccaggag gccgccaaga agaagccagg catcttcatc 1200
ccgagctgcg acgaggatgg ctactaccgg aagatgcagt gtgaccagag 1250
cagcggtgac tgctggcgtg tggaccagct gggcctggag ctgactggca 1300
cgcgcacgca tgggagcccc gactgcgatg acatcgtggg cttctcgggg 1350
gactttggaa gcggtgtcgg ctgggaggat gaggaggaga aggagacgga 1400
ggaagcaggc gaggaggccg aggaggagga gggcgaggca ggcgaggctg 1450
acgacggggg ctacatctgg tagacgccct caggagccgg ctgccggggg 1500
ggactcaaca gcagagctct gagcagcagc aggcaacttc gagaacggat 1550
ccagaaatgc agtcagaagg accctgctcc acctgggggg actgggagtg 1600
tgagtgtgca tggcatgtgt gtggcacaga tggctgggac gggtgacagt 1650
gtgagtgcat gtgtgcatgc atgtgtgtat gtgtgtgtgt gtgtggcatg 1700

```
cgctgacaaa tgtgtccttg atccacactg ctcctggcag agtgagtcac 1750

ccaaaggccc cttcggcctc cttgtagctg ttttctttcc ttttgttgtt 1800

ggttttaaaa tacattcaca cacaaataca aaaaaaaaaa aaaaaaaaaa 1850

aaaaaaaaaa aaaaaaaaaa aaaaaaaaaa aaaaaaaaaa aaaaaaaaa 1899

<210> 14
<211> 424
<212> PRT
<213> Homo sapiens

<400> 14
Met Arg Ala Pro Gly Cys Gly Arg Leu Val Leu Pro Leu Leu Leu
1               5                   10                  15

Leu Ala Ala Ala Ala Leu Ala Glu Gly Asp Ala Lys Gly Leu Lys
                20                  25                  30

Glu Gly Glu Thr Pro Gly Asn Phe Met Glu Asp Glu Gln Trp Leu
                35                  40                  45

Ser Ser Ile Ser Gln Tyr Ser Gly Lys Ile Lys His Trp Asn Arg
                50                  55                  60

Phe Arg Asp Glu Val Glu Asp Asp Tyr Ile Lys Ser Trp Glu Asp
                65                  70                  75

Asn Gln Gln Gly Asp Glu Ala Leu Asp Thr Thr Lys Asp Pro Cys
                80                  85                  90

Gln Lys Val Lys Cys Ser Arg His Lys Val Cys Ile Ala Gln Gly
                95                  100                 105

Tyr Gln Arg Ala Met Cys Ile Ser Arg Lys Lys Leu Glu His Arg
                110                 115                 120

Ile Lys Gln Pro Thr Val Lys Leu His Gly Asn Lys Asp Ser Ile
                125                 130                 135

Cys Lys Pro Cys His Met Ala Gln Leu Ala Ser Val Cys Gly Ser
                140                 145                 150

Asp Gly His Thr Tyr Ser Ser Val Cys Lys Leu Glu Gln Gln Ala
                155                 160                 165

Cys Leu Ser Ser Lys Gln Leu Ala Val Arg Cys Glu Gly Pro Cys
                170                 175                 180

Pro Cys Pro Thr Glu Gln Ala Ala Thr Ser Thr Ala Asp Gly Lys
                185                 190                 195

Pro Glu Thr Cys Thr Gly Gln Asp Leu Ala Asp Leu Gly Asp Arg
                200                 205                 210

Leu Arg Asp Trp Phe Gln Leu Leu His Glu Asn Ser Lys Gln Asn
                215                 220                 225
```

Gly Ser Ala Ser Ser Val Ala Gly Pro Ala Ser Gly Leu Asp Lys
230 235 240

Ser Leu Gly Ala Ser Cys Lys Asp Ser Ile Gly Trp Met Phe Ser
245 250 255

Lys Leu Asp Thr Ser Ala Asp Leu Phe Leu Asp Gln Thr Glu Leu
260 265 270

Ala Ala Ile Asn Leu Asp Lys Tyr Glu Val Cys Ile Arg Pro Phe
275 280 285

Phe Asn Ser Cys Asp Thr Tyr Lys Asp Gly Arg Val Ser Thr Ala
290 295 300

Glu Trp Cys Phe Cys Phe Trp Arg Glu Lys Pro Pro Cys Leu Ala
305 310 315

Glu Leu Glu Arg Ile Gln Ile Gln Glu Ala Ala Lys Lys Lys Pro
320 325 330

Gly Ile Phe Ile Pro Ser Cys Asp Glu Asp Gly Tyr Tyr Arg Lys
335 340 345

Met Gln Cys Asp Gln Ser Ser Gly Asp Cys Trp Arg Val Asp Gln
350 355 360

Leu Gly Leu Glu Leu Thr Gly Thr Arg Thr His Gly Ser Pro Asp
365 370 375

Cys Asp Asp Ile Val Gly Phe Ser Gly Asp Phe Gly Ser Gly Val
380 385 390

Gly Trp Glu Asp Glu Glu Glu Lys Glu Thr Glu Glu Ala Gly Glu
395 400 405

Glu Ala Glu Glu Glu Glu Gly Glu Ala Gly Glu Ala Asp Asp Gly
410 415 420

Gly Tyr Ile Trp

<210> 15
<211> 1008
<212> DNA
<213> Homo sapiens

<400> 15
cacgcacttc acctgggtcg ggattctcag gtcatgaacg gtcccagcca 50

cctccgggca gggcgggtga ggacggggac ggggcgtgtc caactggctg 100

tgggctcttg aaacccgagc atggcacagc acggggcgat gggcgcgttt 150

cgggccctgt gcggcctggc gctgctgtgc gcgctcagcc tgggtcagcg 200

ccccaccggg ggtcccgggt gcggccctgg gcgcctcctg cttgggacgg 250

gaacggacgc gcgctgctgc cgggttcaca cgacgcgctg ctgccgcgat 300

tacccgggcg aggagtgctg ttccgagtgg gactgcatgt gtgtccagcc 350

tgaattccac tgcggagacc cttgctgcac gacctgccgg caccaccctt 400

gtcccccagg ccagggggta cagtcccagg ggaaattcag ttttggcttc 450

cagtgtatcg actgtgcctc ggggaccttc tccgggggcc acgaaggcca 500

ctgcaaacct tggacagact gcacccagtt cgggtttctc actgtgttcc 550

ctgggaacaa gacccacaac gctgtgtgcg tcccagggtc cccgccggca 600

gagccgcttg ggtggctgac cgtcgtcctc ctggccgtgg ccgcctgcgt 650

cctcctcctg acctcggccc agcttggact gcacatctgg cagctgagga 700

gtcagtgcat gtggccccga gagacccagc tgctgctgga ggtgccgccg 750

tcgaccgaag acgccagaag ctgccagttc cccgaggaag agcggggcga 800

gcgatcggca gaggagaagg ggcggctggg agacctgtgg gtgtgagcct 850

ggccgtcctc cggggccacc gaccgcagcc agcccctccc caggagctcc 900

ccaggccgca ggggctctgc gttctgctct gggccgggcc ctgctcccct 950

ggcagcagaa gtgggtgcag gaaggtggca gtgaccagcg ccctggacca 1000

tgcagttc 1008

<210> 16
<211> 241
<212> PRT
<213> Homo sapiens

<400> 16

Met Ala Gln His Gly Ala Met Gly Ala Phe Arg Ala Leu Cys Gly
1 5 10 15

Leu Ala Leu Leu Cys Ala Leu Ser Leu Gly Gln Arg Pro Thr Gly
20 25 30

Gly Pro Gly Cys Gly Pro Gly Arg Leu Leu Leu Gly Thr Gly Thr
35 40 45

Asp Ala Arg Cys Cys Arg Val His Thr Thr Arg Cys Cys Arg Asp
50 55 60

Tyr Pro Gly Glu Glu Cys Cys Ser Glu Trp Asp Cys Met Cys Val
65 70 75

Gln Pro Glu Phe His Cys Gly Asp Pro Cys Cys Thr Thr Cys Arg
80 85 90

His His Pro Cys Pro Pro Gly Gln Gly Val Gln Ser Gln Gly Lys
95 100 105

Phe Ser Phe Gly Phe Gln Cys Ile Asp Cys Ala Ser Gly Thr Phe
110 115 120

Ser Gly Gly His Glu Gly His Cys Lys Pro Trp Thr Asp Cys Thr
125 130 135

Gln Phe Gly Phe Leu Thr Val Phe Pro Gly Asn Lys Thr His Asn
140 145 150

Ala Val Cys Val Pro Gly Ser Pro Pro Ala Glu Pro Leu Gly Trp
155 160 165

Leu Thr Val Val Leu Leu Ala Val Ala Ala Cys Val Leu Leu Leu
170 175 180

Thr Ser Ala Gln Leu Gly Leu His Ile Trp Gln Leu Arg Ser Gln
185 190 195

Cys Met Trp Pro Arg Glu Thr Gln Leu Leu Leu Glu Val Pro Pro
200 205 210

Ser Thr Glu Asp Ala Arg Ser Cys Gln Phe Pro Glu Glu Glu Arg
215 220 225

Gly Glu Arg Ser Ala Glu Glu Lys Gly Arg Leu Gly Asp Leu Trp
230 235 240

Val

<210> 17
<211> 2212
<212> DNA
<213> Homo sapiens

<400> 17
gaaagctata ggctacccat tcagctcccc tgtcagagac tcaagctttg 50

agaaaggcta gcaaagagca aggaaagaga gaaaacaaca aagtggcgag 100

gccctcagag tgaaagcgta aggttcagtc agcctgctgc agctttgcag 150

acctcagctg ggcatctcca gactcccctg aaggaagagc cttcctcacc 200

caaacccaca aaagatgctg aaaaagcctc tctcagctgt gacctggctc 250

tgcattttca tcgtggcctt tgtcagccac ccagcgtggc tgcagaagct 300

ctctaagcac aagacaccag cacagccaca gctcaaagcg gccaactgct 350

gtgaggaggt gaaggagctc aaggcccaag ttgccaacct tagcagcctg 400

ctgagtgaac tgaacaagaa gcaggagagg gactgggtca gcgtggtcat 450

gcaggtgatg gagctggaga gcaacagcaa gcgcatggag tcgcggctca 500

cagatgctga gagcaagtac tccgagatga acaaccaaat tgacatcatg 550

cagctgcagg cagcacagac ggtcactcag acctccgcag atgccatcta 600

cgactgctct tccctctacc agaagaacta ccgcatctct ggagtgtata 650

agcttcctcc tgatgacttc ctgggcagcc ctgaactgga ggtgttctgt 700

gacatggaga cttcaggcgg aggctggacc atcatccaga gacgaaaaag 750

tggccttgtc tccttctacc gggactggaa gcagtacaag cagggctttg 800
gcagcatccg tggggacttc tggctgggga acgaacacat ccaccggctc 850
tccagacagc caacccggct gcgtgtagag atggaggact gggagggcaa 900
cctgcgctac gctgagtata gccactttgt tttgggcaat gaactcaaca 950
gctatcgcct cttcctgggg aactacactg gcaatgtggg gaacgacgcc 1000
ctccagtatc ataacaacac agccttcagc accaaggaca aggacaatga 1050
caactgcttg gacaagtgtg cacagctccg caaaggtggc tactggtaca 1100
actgctgcac agactccaac ctcaatggag tgtactaccg cctgggtgag 1150
cacaataagc acctggatgg catcacctgg tatggctggc atggatctac 1200
ctactccctc aaacgggtgg agatgaaaat ccgcccagaa gacttcaagc 1250
cttaaaagga ggctgccgtg gagcacggat acagaaactg agacacgtgg 1300
agactggatg agggcagatg aggacaggaa gagagtgtta gaaagggtag 1350
gactgagaaa cagcctataa tctccaaaga aagaataagt ctccaaggag 1400
cacaaaaaaa tcatatgtac caaggatgtt acagtaaaca ggatgaacta 1450
tttaaaccca ctgggtcctg ccacatcctt ctcaaggtgg tagactgagt 1500
ggggtctctc tgcccaagat ccctgacata gcagtagctt gtcttttcca 1550
catgatttgt ctgtgaaaga aaataatttt gagatcgttt tatctatttt 1600
ctctacggct taggctatgt gagggcaaaa cacaaatccc tttgctaaaa 1650
agaaccatat tattttgatt ctcaaaggat aggcctttga gtgttagaga 1700
aaggagtgaa ggaggcaggt gggaaatggt atttctattt ttaaatccag 1750
tgaaattatc ttgagtctac acattatttt taaaacacaa aaattgttcg 1800
gctggaactg acccaggctg gacttgcggg gaggaaactc cagggcactg 1850
catctggcga tcagactctg agcactgccc ctgctcgcct tggtcatgta 1900
cagcactgaa aggaatgaag caccagcagg aggtggacag agtctctcat 1950
ggatgccggc acaaaactgc cttaaaatat tcatagttaa tacaggtata 2000
tctattttta tttactttgt aagaaacaag ctcaaggagc ttccttttaa 2050
attttgtctg taggaaatgg ttgaaaactg aaggtagatg gtgttatagt 2100
taataataaa tgctgtaaat aagcatctca ctttgtaaaa ataaaatatt 2150
gtggttttgt tttaaacatt caacgtttct tttccttcta caataaacac 2200
tttcaaaatg tg 2212

<210> 18

<211> 346
<212> PRT
<213> Homo sapiens

<400> 18

```
Met Leu Lys Lys Pro Leu Ser Ala Val Thr Trp Leu Cys Ile Phe
1               5                   10                  15

Ile Val Ala Phe Val Ser His Pro Ala Trp Leu Gln Lys Leu Ser
                20                  25                  30

Lys His Lys Thr Pro Ala Gln Pro Gln Leu Lys Ala Ala Asn Cys
                35                  40                  45

Cys Glu Glu Val Lys Glu Leu Lys Ala Gln Val Ala Asn Leu Ser
                50                  55                  60

Ser Leu Leu Ser Glu Leu Asn Lys Lys Gln Glu Arg Asp Trp Val
                65                  70                  75

Ser Val Val Met Gln Val Met Glu Leu Glu Ser Asn Ser Lys Arg
                80                  85                  90

Met Glu Ser Arg Leu Thr Asp Ala Glu Ser Lys Tyr Ser Glu Met
                95                  100                 105

Asn Asn Gln Ile Asp Ile Met Gln Leu Gln Ala Ala Gln Thr Val
                110                 115                 120

Thr Gln Thr Ser Ala Asp Ala Ile Tyr Asp Cys Ser Ser Leu Tyr
                125                 130                 135

Gln Lys Asn Tyr Arg Ile Ser Gly Val Tyr Lys Leu Pro Pro Asp
                140                 145                 150

Asp Phe Leu Gly Ser Pro Glu Leu Glu Val Phe Cys Asp Met Glu
                155                 160                 165

Thr Ser Gly Gly Gly Trp Thr Ile Ile Gln Arg Arg Lys Ser Gly
                170                 175                 180

Leu Val Ser Phe Tyr Arg Asp Trp Lys Gln Tyr Lys Gln Gly Phe
                185                 190                 195

Gly Ser Ile Arg Gly Asp Phe Trp Leu Gly Asn Glu His Ile His
                200                 205                 210

Arg Leu Ser Arg Gln Pro Thr Arg Leu Arg Val Glu Met Glu Asp
                215                 220                 225

Trp Glu Gly Asn Leu Arg Tyr Ala Glu Tyr Ser His Phe Val Leu
                230                 235                 240

Gly Asn Glu Leu Asn Ser Tyr Arg Leu Phe Leu Gly Asn Tyr Thr
                245                 250                 255

Gly Asn Val Gly Asn Asp Ala Leu Gln Tyr His Asn Asn Thr Ala
                260                 265                 270

Phe Ser Thr Lys Asp Lys Asp Asn Asp Asn Cys Leu Asp Lys Cys
                275                 280                 285
```

```
Ala Gln Leu Arg Lys Gly Gly Tyr Trp Tyr Asn Cys Cys Thr Asp
                290                 295                 300

Ser Asn Leu Asn Gly Val Tyr Tyr Arg Leu Gly Glu His Asn Lys
                305                 310                 315

His Leu Asp Gly Ile Thr Trp Tyr Gly Trp His Gly Ser Thr Tyr
                320                 325                 330

Ser Leu Lys Arg Val Glu Met Lys Ile Arg Pro Glu Asp Phe Lys
                335                 340                 345

Pro
```

<210> 19
<211> 415
<212> DNA
<213> Homo sapiens

<400> 19

```
aaacttgacg ccatgaagat cccggtcctt cctgccgtgg tgctcctctc 50

cctcctggtg ctccactctg cccagggagc caccctgggt ggtcctgagg 100

aagaaagcac cattgagaat tatgcgtcac gacccgaggc ctttaacacc 150

ccgttcctga acatcgacaa attgcgatct gcgtttaagg ctgatgagtt 200

cctgaactgg cacgccctct ttgagtctat caaaaggaaa cttcctttcc 250

tcaactggga tgcctttcct aagctgaaag gactgaggag cgcaactcct 300

gatgcccagt gaccatgacc tccactggaa gagggggcta gcgtgagcgc 350

tgattctcaa cctaccataa ctctttcctg cctcaggaac tccaataaaa 400

cattttccat ccaaa 415
```

<210> 20
<211> 99
<212> PRT
<213> Homo sapiens

<400> 20

```
Met Lys Ile Pro Val Leu Pro Ala Val Val Leu Leu Ser Leu Leu
  1               5                  10                  15

Val Leu His Ser Ala Gln Gly Ala Thr Leu Gly Gly Pro Glu Glu
                 20                  25                  30

Glu Ser Thr Ile Glu Asn Tyr Ala Ser Arg Pro Glu Ala Phe Asn
                 35                  40                  45

Thr Pro Phe Leu Asn Ile Asp Lys Leu Arg Ser Ala Phe Lys Ala
                 50                  55                  60

Asp Glu Phe Leu Asn Trp His Ala Leu Phe Glu Ser Ile Lys Arg
                 65                  70                  75
```

```
Lys Leu Pro Phe Leu Asn Trp Asp Ala Phe Pro Lys Leu Lys Gly
                80                  85                  90

Leu Arg Ser Ala Thr Pro Asp Ala Gln
                95
```

<210> 21
<211> 584
<212> DNA
<213> Homo sapiens

<400> 21

```
caacagaagc caagaaggaa gccgtctatc ttgtggcgat catgtataag 50

ctggcctcct gctgtttgct tttcacagga ttcttaaatc ctctcttatc 100

tcttcctctc cttgactcca gggaaatatc ctttcaactc tcagcacctc 150

atgaagacgc gcgcttaact ccggaggagc tagaaagagc ttcccttcta 200

cagatattgc cagagatgct gggtgcagaa agaggggata ttctcaggaa 250

agcagactca agtaccaaca tttttaaccc aagaggaaat ttgagaaagt 300

ttcaggattt ctctggacaa gatcctaaca ttttactgag tcatcttttg 350

gccagaatct ggaaaccata caagaaacgt gagactcctg attgcttctg 400

gaaatactgt gtctgaagtg aaataagcat ctgttagtca gctcagaaac 450

acccatctta gaatatgaaa aataacacaa tgcttgattt gaaaacagtg 500

tggagaaaaa ctaggcaaac tacaccctgt tcattgttac ctggaaaata 550

aatcctctat gttttgcaca aaaaaaaaaa aaaa 584
```

<210> 22
<211> 124
<212> PRT
<213> Homo sapiens

<400> 22

```
Met Tyr Lys Leu Ala Ser Cys Cys Leu Leu Phe Thr Gly Phe Leu
  1               5                  10                  15

Asn Pro Leu Leu Ser Leu Pro Leu Leu Asp Ser Arg Glu Ile Ser
                20                  25                  30

Phe Gln Leu Ser Ala Pro His Glu Asp Ala Arg Leu Thr Pro Glu
                35                  40                  45

Glu Leu Glu Arg Ala Ser Leu Leu Gln Ile Leu Pro Glu Met Leu
                50                  55                  60

Gly Ala Glu Arg Gly Asp Ile Leu Arg Lys Ala Asp Ser Ser Thr
                65                  70                  75

Asn Ile Phe Asn Pro Arg Gly Asn Leu Arg Lys Phe Gln Asp Phe
                80                  85                  90
```

Ser Gly Gln Asp Pro Asn Ile Leu Leu Ser His Leu Leu Ala Arg
95 100 105

Ile Trp Lys Pro Tyr Lys Lys Arg Glu Thr Pro Asp Cys Phe Trp
110 115 120

Lys Tyr Cys Val

<210> 23
<211> 957
<212> DNA
<213> Homo sapiens

<400> 23
gggagagagg ataaatagca gcgtggcttc cctggctcct ctctgcatcc 50

ttcccgacct tcccagcaat atgcatcttg cacgtctggt cggctcctgc 100

tccctccttc tgctactggg ggccctgtct ggatgggcgg ccagcgatga 150

ccccattgag aaggtcattg aagggatcaa ccgagggctg agcaatgcag 200

agagagaggt gggcaaggcc ctggatggca tcaacagtgg aatcacgcat 250

gccggaaggg aagtggagaa ggttttcaac ggacttagca acatggggag 300

ccacaccggc aaggagttgg acaaaggcgt ccaggggctc aaccacggca 350

tggacaaggt tgcccatgag atcaaccatg gtattggaca agcaggaaag 400

gaagcagaga agcttggcca tggggtcaac aacgctgctg gacaggccgg 450

gaaggaagca gacaaagcgg tccaagggtt ccacactggg gtccaccagg 500

ctgggaagga agcagagaaa cttggccaag ggggtcaacca tgctgctgac 550

caggctggaa aggaagtgga gaagcttggc caaggtgccc accatgctgc 600

tggccaggcc gggaaggagc tgcagaatgc tcataatggg gtcaaccaag 650

ccagcaagga ggccaaccag ctgctgaatg gcaaccatca aagcggatct 700

tccagccatc aaggaggggc cacaaccacg ccgttagcct ctggggcctc 750

agtcaacacg cctttcatca accttcccgc cctgtggagg agcgtcgcca 800

acatcatgcc ctaaactggc atccggcctt gctgggagaa taatgtcgcc 850

gttgtcacat cagctgacat gacctggagg ggttgggggt gggggacagg 900

tttctgaaat ccctgaaggg ggttgtactg ggatttgtga ataaacttga 950

tacacca 957

<210> 24
<211> 247
<212> PRT
<213> Homo sapiens

<400> 24

```
Met His Leu Ala Arg Leu Val Gly Ser Cys Ser Leu Leu Leu Leu
1               5                   10                  15

Leu Gly Ala Leu Ser Gly Trp Ala Ala Ser Asp Asp Pro Ile Glu
                20                  25                  30

Lys Val Ile Glu Gly Ile Asn Arg Gly Leu Ser Asn Ala Glu Arg
                35                  40                  45

Glu Val Gly Lys Ala Leu Asp Gly Ile Asn Ser Gly Ile Thr His
                50                  55                  60

Ala Gly Arg Glu Val Glu Lys Val Phe Asn Gly Leu Ser Asn Met
                65                  70                  75

Gly Ser His Thr Gly Lys Glu Leu Asp Lys Gly Val Gln Gly Leu
                80                  85                  90

Asn His Gly Met Asp Lys Val Ala His Glu Ile Asn His Gly Ile
                95                  100                 105

Gly Gln Ala Gly Lys Glu Ala Glu Lys Leu Gly His Gly Val Asn
                110                 115                 120

Asn Ala Ala Gly Gln Ala Gly Lys Glu Ala Asp Lys Ala Val Gln
                125                 130                 135

Gly Phe His Thr Gly Val His Gln Ala Gly Lys Glu Ala Glu Lys
                140                 145                 150

Leu Gly Gln Gly Val Asn His Ala Ala Asp Gln Ala Gly Lys Glu
                155                 160                 165

Val Glu Lys Leu Gly Gln Gly Ala His His Ala Ala Gly Gln Ala
                170                 175                 180

Gly Lys Glu Leu Gln Asn Ala His Asn Gly Val Asn Gln Ala Ser
                185                 190                 195

Lys Glu Ala Asn Gln Leu Leu Asn Gly Asn His Gln Ser Gly Ser
                200                 205                 210

Ser Ser His Gln Gly Gly Ala Thr Thr Thr Pro Leu Ala Ser Gly
                215                 220                 225

Ala Ser Val Asn Thr Pro Phe Ile Asn Leu Pro Ala Leu Trp Arg
                230                 235                 240

Ser Val Ala Asn Ile Met Pro
                245
```

<210> 25
<211> 1728
<212> DNA
<213> Homo sapiens

<400> 25
cagccgggtc ccaagcctgt gcctgagcct gagcctgagc ctgagcccga 50

gccgggagcc ggtcgcgggg gctccgggct gtgggaccgc tgggccccca 100

gcgatggcga ccctgtgggg aggccttctt cggcttggct ccttgctcag 150

cctgtcgtgc ctggcgcttt ccgtgctgct gctggcgcag ctgtcagacg 200

ccgccaagaa tttcgaggat gtcagatgta aatgtatctg ccctccctat 250

aaagaaaatt ctgggcatat ttataataag aacatatctc agaaagattg 300

tgattgcctt catgttgtgg agcccatgcc tgtgcggggg cctgatgtag 350

aagcatactg tctacgctgt gaatgcaaat atgaagaaag aagctctgtc 400

acaatcaagg ttaccattat aatttatctc tccattttgg gccttctact 450

tctgtacatg gtatatctta ctctggttga gcccatactg aagaggcgcc 500

tctttggaca tgcacagttg atacagagtg atgatgatat tggggatcac 550

cagccttttg caaatgcaca cgatgtgcta gcccgctccc gcagtcgagc 600

caacgtgctg aacaaggtag aatatgcaca gcagcgctgg aagcttcaag 650

tccaagagca gcgaaagtct gtctttgacc ggcatgttgt cctcagctaa 700

ttgggaattg aattcaaggt gactagaaag aaacaggcag acaactggaa 750

agaactgact gggttttgct gggtttcatt ttaatacctt gttgatttca 800

ccaactgttg ctggaagatt caaaactgga agcaaaaact tgcttgattt 850

ttttttcttg ttaacgtaat aatagagaca tttttaaaag cacacagctc 900

aaagtcagcc aataagtctt ttcctatttg tgacttttac taataaaaat 950

aaatctgcct gtaaattatc ttgaagtcct ttacctggaa caagcactct 1000

ctttttcacc acatagtttt aacttgactt tcaagataat tttcagggtt 1050

tttgttgttg ttgtttttttg tttgtttgtt ttggtgggag aggggaggga 1100

tgcctgggaa gtggttaaca acttttttca agtcacttta ctaaacaaac 1150

ttttgtaaat agaccttacc ttctattttc gagtttcatt tatattttgc 1200

agtgtagcca gcctcatcaa agagctgact tactcatttg acttttgcac 1250

tgactgtatt atctgggtat ctgctgtgtc tgcacttcat ggtaaacggg 1300

atctaaaatg cctggtggct tttcacaaaa agcagatttt cttcatgtac 1350

tgtgatgtct gatgcaatgc atcctagaac aaactggcca tttgctagtt 1400

tactctaaag actaaacata gtcttggtgt gtgtggtctt actcatcttc 1450

tagtaccttt aaggacaaat cctaaggact tggacacttg caataaagaa 1500

attttatttt aaacccaagc ctccctggat tgataatata tacacatttg 1550

tcagcatttc cggtcgtggt gagaggcagc tgtttgagct ccaatatgtg 1600

cagctttgaa ctagggctgg ggttgtgggt gcctcttctg aaaggtctaa 1650

ccattattgg ataactggct tttttcttcc tatgtcctct ttggaatgta 1700

acaataaaaa taatttttga aacatcaa 1728

<210> 26
<211> 198
<212> PRT
<213> Homo sapiens

<400> 26
Met Ala Thr Leu Trp Gly Gly Leu Leu Arg Leu Gly Ser Leu Leu
1               5                   10                  15

Ser Leu Ser Cys Leu Ala Leu Ser Val Leu Leu Leu Ala Gln Leu
                20                  25                  30

Ser Asp Ala Ala Lys Asn Phe Glu Asp Val Arg Cys Lys Cys Ile
                35                  40                  45

Cys Pro Pro Tyr Lys Glu Asn Ser Gly His Ile Tyr Asn Lys Asn
                50                  55                  60

Ile Ser Gln Lys Asp Cys Asp Cys Leu His Val Val Glu Pro Met
                65                  70                  75

Pro Val Arg Gly Pro Asp Val Glu Ala Tyr Cys Leu Arg Cys Glu
                80                  85                  90

Cys Lys Tyr Glu Glu Arg Ser Ser Val Thr Ile Lys Val Thr Ile
                95                  100                 105

Ile Ile Tyr Leu Ser Ile Leu Gly Leu Leu Leu Leu Tyr Met Val
                110                 115                 120

Tyr Leu Thr Leu Val Glu Pro Ile Leu Lys Arg Arg Leu Phe Gly
                125                 130                 135

His Ala Gln Leu Ile Gln Ser Asp Asp Asp Ile Gly Asp His Gln
                140                 145                 150

Pro Phe Ala Asn Ala His Asp Val Leu Ala Arg Ser Arg Ser Arg
                155                 160                 165

Ala Asn Val Leu Asn Lys Val Glu Tyr Ala Gln Gln Arg Trp Lys
                170                 175                 180

Leu Gln Val Gln Glu Gln Arg Lys Ser Val Phe Asp Arg His Val
                185                 190                 195

Val Leu Ser

<210> 27
<211> 43
<212> DNA
<213> Artificial sequence

<220>
<223> amylase vector PCR oligonucleotide

<400> 27
tgtaaaacga cggccagtta aatagacctg caattattaa tct 43

<210> 28
<211> 41
<212> DNA
<213> Artificial sequence

<220>
<223> amylase vector PCR oligonucleotide

<400> 28
caggaaacag ctatgaccac ctgcacacct gcaaatccat t 41

<210> 29
<211> 25
<212> DNA
<213> Artificial sequence

<220>
<223> cloning oligonucleotide

<400> 29
cacattcagt cctcagcaaa atgaa 25

<210> 30
<211> 40
<212> DNA
<213> Artificial sequence

<220>
<223> cloning oligonucleotide

<400> 30
gagaataaaa acagagtgaa aatggagccc ttcattttgc 40

<210> 31
<211> 23
<212> DNA
<213> Artificial sequence

<220>
<223> cloning oligonucleotide

<400> 31
ctcagcttgc tgagcttgag gga 23

<210> 32
<211> 24
<212> DNA
<213> Artificial sequence

<220>
<223> cloning oligonucleotide

<400> 32
tcagctccag actctgatac tgcc 24

<210> 33
<211> 24
<212> DNA

<213> Artificial sequence

<220>
<223> cloning oligonucleotide

<400> 33
tgcctttcta ggaggcagag ctcc 24

<210> 34
<211> 50
<212> DNA
<213> Artificial sequence

<220>
<223> hybridization probe

<400> 34
ggacccagaa atgtgtcctg agaatggatc ttgtgtacct gatggtccag 50

<210> 35
<211> 21
<212> DNA
<213> Artificial sequence

<220>
<223> PCR cloning oligonucleotide

<400> 35
tgtccaccaa gcagacagaa g 21

<210> 36
<211> 21
<212> DNA
<213> Artificial sequence

<220>
<223> PCR cloning oligonucleotide

<400> 36
actggatggc gcctttccat g 21

<210> 37
<211> 50
<212> DNA
<213> Artificial sequence

<220>
<223> hybridization probe

<400> 37
ctgacagtga ctagctcaga ccacccagag gacacggcca acgtcacagt 50

<210> 38
<211> 45
<212> DNA
<213> Artificial sequence

<220>
<223> hybridization probe

<400> 38

gggctctttc ccacgctggt actatgaccc cacggagcag atctg 45

<210> 39
<211> 24
<212> DNA
<213> Artificial sequence

<220>
<223> PCR cloning oligonucleotide

<400> 39
caggtcgaac ccagaccacg atgc 24

<210> 40
<211> 18
<212> DNA
<213> Artificial sequence

<220>
<223> PCR cloning oligonucleotide

<400> 40
gccacatggc ccagcttg 18

<210> 41
<211> 18
<212> DNA
<213> Artificial sequence

<220>
<223> PCR cloning oligonucleotide

<400> 41
gagacggagg aagcaggc 18

<210> 42
<211> 19
<212> DNA
<213> Artificial sequence

<220>
<223> PCR cloning oligonucleotide

<400> 42
ggccacactt acagctctg 19

<210> 43
<211> 24
<212> DNA
<213> Artificial sequence

<220>
<223> PCR cloning oligonucleotide

<400> 43
agccggcttc tgagggcgtc tacc 24

<210> 44
<211> 45
<212> DNA
<213> Artificial sequence

<220>
<223> hybridization probe

<400> 44
tggtgctgcc gctgctgctc ctggccgcgg cagccctggc cgaag 45

<210> 45
<211> 19
<212> DNA
<213> Artificial sequence

<220>
<223> PCR cloning oligonucleotide

<400> 45
cacagcacgg ggcgatggg 19

<210> 46
<211> 18
<212> DNA
<213> Artificial sequence

<220>
<223> PCR cloning oligonucleotide

<400> 46
gctctgcgtt ctgctctg 18

<210> 47
<211> 28
<212> DNA
<213> Artificial sequence

<220>
<223> PCR cloning oligonucleotide

<400> 47
ggcacagcac ggggcgatgg gcgcgttt 28

<210> 48
<211> 24
<212> DNA
<213> Artificial sequence

<220>
<223> PCR cloning oligonucleotide

<400> 48
ctggtcactg ccaccttcct gcac 24

<210> 49
<211> 17
<212> DNA
<213> Artificial sequence

<220>
<223> PCR cloning oligonucleotide

<400> 49
cgctgaccca ggctgag 17

<210> 50
<211> 27
<212> DNA
<213> Artificial sequence

<220>
<223> PCR cloning oligonucleotide

<400> 50
gaaggtcccc gaggcacagt cgataca 27

<210> 51
<211> 40
<212> DNA
<213> Artificial sequence

<220>
<223> hybridization probe

<400> 51
gaggagtgct gttccgagtg ggactgcatg tgtgtccagc 40

<210> 52
<211> 40
<212> DNA
<213> Artificial sequence

<220>
<223> hybridization probe

<400> 52
agcctgggtc agcgccccac cgggggtccc gggtgcggcc 40

<210> 53
<211> 31
<212> DNA
<213> Artificial sequence

<220>
<223> PCR cloning oligonucleotide

<400> 53
ttcagcacca aggacaagga caatgacaac t 31

<210> 54
<211> 32
<212> DNA
<213> Artificial sequence

<220>
<223> PCR cloning oligonucleotide

<400> 54
tgtgcacact tgtccaagca gttgtcattg tc 32

<210> 55
<211> 23
<212> DNA
<213> Artificial sequence

<220>
<223> hybridization probe

<400> 55
gtagtacact ccattgaggt tgg 23

<210> 56
<211> 23
<212> DNA
<213> Artificial sequence

<220>
<223> PCR cloning oligonucleotide

<400> 56
caatatgcat cttgcacgtc tgg 23

<210> 57
<211> 24
<212> DNA
<213> Artificial sequence

<220>
<223> PCR cloning oligonucleotide

<400> 57
aagcttctct gcttcctttc ctgc 24

<210> 58
<211> 43
<212> DNA
<213> Artificial sequence

<220>
<223> hybridization probe

<400> 58
tgaccccatt gagaaggtca ttgaagggat caaccgaggg ctg 43

<210> 59
<211> 40
<212> DNA
<213> Artificial sequence

<220>
<223> in situ oligonucleotide

<400> 59
tctaatacga ctcactatag ctcaggggaa gagccaaaga 40

<210> 60
<211> 40
<212> DNA
<213> Artificial sequence

<220>
<223> in situ oligonucleotide

<400> 60
tgaattaacc ctcactaaag cagtgcaatg caggggacta 40

<210> 61
<211> 48
<212> DNA
<213> Artificial sequence

<220>
<223> in situ oligonucleotide

<400> 61
ggattctaat acgactcact atagggcaac ccgagcatgg cacagcac 48

<210> 62
<211> 47
<212> DNA
<213> Artificial sequence

<220>
<223> in situ oligonucleotide

<400> 62
ctatgaaatt aaccctcact aaagggatct cccagccgcc ccttctc 47

<210> 63
<211> 48
<212> DNA
<213> Artificial sequence

<220>
<223> in situ oligonucleotide

<400> 63
ggattctaat acgactcact atagggcccg agatatgcac ccaatgtc 48

<210> 64
<211> 47
<212> DNA
<213> Artificial sequence

<220>
<223> in situ oligonucleotide

<400> 64
ctatgaaatt aaccctcact aaagggatcc cagaatcccg aagaaca 47